(19) Europäisches Patentamt
European Patent Office
Office européen des brevets

(11) EP 4 622 618 B1

(12) **EUROPEAN PATENT SPECIFICATION**

(45) Date of publication and mention of the grant of the patent:
**22.04.2026 Bulletin 2026/17**

(21) Application number: **24733475.8**

(22) Date of filing: **29.05.2024**

(51) International Patent Classification (IPC):
***A61K 8/02*** *(2006.01)* ***A61K 8/46*** *(2006.01)*
***A61Q 19/10*** *(2006.01)*

(52) Cooperative Patent Classification (CPC):
**A61K 8/0295; A61K 8/463; A61K 8/466; A61Q 19/10;** A61K 2800/48

(86) International application number:
**PCT/US2024/031339**

(87) International publication number:
**WO 2024/253903 (12.12.2024 Gazette 2024/50)**

(54) **PERSONAL CARE COMPOSITIONS FOR CLEANSING THE SKIN**

KÖRPERPFLEGEZUSAMMENSETZUNGEN ZUR REINIGUNG DER HAUT

COMPOSITIONS DE SOINS PERSONNELS POUR NETTOYAGE DE LA PEAU

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **06.06.2023 US 202363506440 P**

(43) Date of publication of application:
**01.10.2025 Bulletin 2025/40**

(73) Proprietor: **The Procter & Gamble Company**
**Cincinnati, OH 45202 (US)**

(72) Inventors:
- **AKINTELURE, Olubolaji**
  **Cincinnati, Ohio 45202 (US)**
- **WEI, Karl, Shiqing**
  **Cincinnati, Ohio 45202 (US)**
- **JI, Wei**
  **Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**EP-A2- 2 011 545**
**WO-A2-2022/266175**
**CA-A1- 2 643 784**
**US-A1- 2006 079 418**
**US-A1- 2013 029 895**
**US-A1- 2018 185 255**
**US-A1- 2022 081 648**

- **DATABASE GNPD [online] MINTEL; 18 July 2018 (2018-07-18), ANONYMOUS: "Hand Soap", XP055953965, retrieved from https://www.gnpd.com/sinatra/recordpage/5833719/ Database accession no. 5833719**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 622 618 B1

## Description

FIELD OF THE INVENTION

The present application generally relates to personal care compositions for cleansing the skin, its methods and its uses. The personal care composition includes at least a cleansing phase and a benefit phase, wherein the cleansing phase comprises an aqueous structured surfactant phase. The cleansing phase comprises a surfactant A that comprises a C13 alkyl sulfate anionic surfactant; or a surfactant B that comprises a mixture of C12 and C13 alkyl sulfate anionic surfactants, a zwitterionic surfactant that includes an hydroxysultaine, and a structuring system. The structuring system includes from about 3% to about 10% by weight of the personal care composition, of an electrolyte. The benefit phase comprises a benefit agent.

BACKGROUND OF THE INVENTION

Cleansing is an activity that has been done for hundreds of years. Early cleansers were based on either soap chemistry or simple mechanical action in order to remove dirt from the skin, as well as endogenous soils such as sweat, sebum, and body odors. Skin cleansing and methods therefore have involved the utilization of soaps, body washes, and other personal care compositions. Personal care compositions can be structured to suspend and stabilize dispersions of benefit agents while maintaining physical integrity of the personal care compositions, and there are many ways to provide such structure. The ability to provide structure can be an important property for such personal care compositions, but it is also important for personal care compositions to have the ability to rapidly become micellar upon dilution for cleansing the skin and depositing benefit agents. Having too much structure in a personal care composition can result in inferior performance while not having enough structure in a personal care composition can cause instability. Further, achieving a balance between these two properties can be a challenging task. Personal care compositions comprising sodium trideceth-2 sulfate and a structuring system based on specific associative polymers were explored.

Ethoxylated surfactants such as Sodium Laureth Sulfate (SLES or SLE3S) or Sodium Trideceth-n Sulfate (STnS) are used widely across the cosmetic industry in personal care products. These surfactants traditionally have been used to achieve a consumer desirable product profile which includes dispensed viscosity/product texture, lather, cleaning, and deposition of hair/scalp actives. Ethoxylation provides enhanced solubility, reduced crystallization in liquids, enhanced polymer interaction for coacervate formation and subsequent benefit delivery to the skin and scalp, increased mildness to the skin, and improved quality of lather.

Alkoxylated fatty alcohols are used are in many industries. For example, they can be used as non-ionic surfactants in detergents and cleansers. They can also be an intermediate in the production of other surfactants through processes like sulfation. Current sulfation processes of alkoxylated fatty alcohols can result in the formation of unwanted impurities such as dioxane components which can remain as part of the alkoxylated fatty alcohol sulfate as it is sold or used. Such unwanted impurities can be removed by additional treatment processes, e.g. a relatively and costly vacuum stripping process.

Also, 1,4-dioxane is a persistent impurity that forms from ethoxylate surfactants and may result in trace residual amounts being present, particularly during sulfation of ethoxylated fatty alcohols, and in those same ethoxylated surfactants during aging in the supply chain and, more slowly, in finished products that contain them.

US2006079418 A1 discloses a stable multi-phase personal care composition comprising a cleansing phase comprising an anionic surfactant, which may be for example ammonium tridecyl sulphate or monomethyl branched surfactants. Monomethyl branched surfactants include those prepared using the Safol 23 alcohol mixture, and the composition may also comprise zwitterionic surfactants such as betaines, and electrolytes.

As such, there is a desire to make personal care compositions that contain relatively very low amount or no ethoxylated surfactants, to mitigate any undesired impurity profile and any need of any additional treatment processes.

There is a need to develop a formulation approach for personal care compositions that utilize relatively very low or non-ethoxylated surfactants, without having negative consumer noticeable trade-offs, especially in terms of providing the desired balance between structure in the personal care compositions and performance.

There is a need to provide a personal care composition for cleansing the skin with an improved structure, an improved stability, with an optimum rheology profile, any attributes sought by the consumer such as lather performance, mildness, combatting any drying effects of the surfactants, deposition of benefit agents, enhanced fragrance experience and/or any improved skin-feel attributes such as skin feeling less oily and/or sticky.

There is also another need to provide a personal care composition for cleansing the skin that is substantially free or free of betaine for further providing mildness to the user’s skin.

SUMMARY OF THE INVENTION

A personal care composition is provided and comprises: at least a cleansing phase and a benefit phase, wherein the personal care composition is free of ethoxylated anionic sulfate surfactant, wherein the cleansing phase comprises an aqueous structured surfactant phase. The cleansing phase comprises: i) from about 1% to about 20% by weight of the personal care composition, of a surfactant A, wherein the surfactant A comprises a C13 alkyl sulfate anionic surfactant, wherein the C13 alkyl sulfate anionic surfactant consists of: a) less than about 40% by weight of the C13 alkyl sulfate anionic surfactant of a linear C13 alkyl sulfate, and b) more than about 60% by weight of the C13 alkyl sulfate anionic surfactant of a 2-branched C13 alkyl sulfate anionic surfactant, wherein the 2-branched C13 alkyl sulfate anionic surfactant comprises: about 25% or less by weight of the 2-branched C13 alkyl sulfate anionic surfactant of 2-pentyl octyl sulfate anionic surfactant, and more than about 25% by weight the 2-branched C13 alkyl sulfate anionic surfactant of 2-methyl dodecyl sulfate anionic surfactant; and c) less than about 5% by weight of the C13 alkyl sulfate anionic surfactant of other branched C13 alkyl sulfate anionic surfactant, wherein a, b and c add up to about 100% by weight of the C13 alkyl sulfate anionic surfactant.

Alternatively, the cleansing phase comprises: i) from about 1% to about 20% by weight of the personal care composition, of a surfactant B, wherein the surfactant B comprises a mixture of C12 and C13 alkyl sulfate anionic surfactants, optionally wherein the surfactant B consists essentially of a mixture of C12 and C13 alkyl sulfate anionic surfactants. The mixture of the C12 and C13 alkyl sulfate anionic surfactants comprises a mixture of surfactant isomers of Formula I and surfactant isomers of Formula II:

$$CH_3\text{-}(CH_2)_p\text{-}CH_2\text{-}OSO_3^-\ X^+ \qquad (I)$$

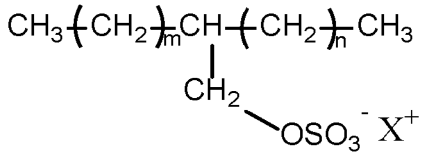

(II)

wherein $10 \leq p \leq 11$;

wherein $8 \leq m + n \leq 9$ and $0 \leq m, n \leq 9$;

wherein X is a counter cation selected from the group consisting of lithium, sodium, potassium and ammonium, preferably sodium;

The surfactant B comprises: from about 37% to about 50%, preferably from about 38% to about 49%, more preferably from about 40% to about 49% of the C12 alkyl sulfate anionic surfactant by weight of the surfactant B. The C12 alkyl sulfate anionic surfactant comprises a 2-alkyl branched C12 alkyl sulfate. The surfactant B comprises from about 50% to about 63%, preferably from about 51% to about 62%, more preferably from about 51% to about 60% of the C13 alkyl sulfate anionic surfactant by weight of the surfactant B. The C13 alkyl sulfate anionic surfactant comprises a 2-alkyl branched C13 alkyl sulfate. Also, the surfactant B comprises less or equal than about 5% of other alkyl sulfate anionic surfactants by weight of the surfactant B, preferably less or equal than about 4% of other alkyl sulfate anionic surfactants by weight of the surfactant B, wherein other alkyl sulfate anionic surfactants are not C12 or C13 alkyl sulfate anionic surfactant.

The cleansing phase also comprises: ii) from about 0.1% to about 20% by weight of the personal care composition, of a zwitterionic surfactant, wherein the zwitterionic surfactant comprises an hydroxysultaine; iii) a structuring system comprising: iiia) a non-ionic emulsifier; iiib) from about 0.01% to about 5% by weight of the personal care composition, of a rheology modifier; iiic) from about 3% to about 10% by weight of the personal care composition, of an electrolyte; wherein the benefit phase comprises: from about 0.1% to about 50%, by weight of said personal care composition, of a benefit agent.

A method of providing a stable personal care composition comprises the step of forming a personal care composition as set out hereinbefore with a surfactant A, wherein the surfactant A comprises a C13 alkyl sulfate anionic surfactant, wherein the C13 alkyl sulfate anionic surfactant consists of: a) less than about 40% by weight of the C13 alkyl sulfate anionic surfactant of a linear C13 alkyl sulfate, and b) more than about 60% by weight of the C13 alkyl sulfate anionic surfactant of a 2-branched C13 alkyl sulfate anionic surfactant, wherein the 2-branched C13 alkyl sulfate anionic surfactant comprises: about 25% or less by weight of the 2-branched C13 alkyl sulfate anionic surfactant of 2-pentyl octyl sulfate anionic surfactant, and more than about 25% by weight the 2-branched C13 alkyl sulfate anionic surfactant of 2-methyl dodecyl sulfate anionic surfactant; and c) less than about 5% by weight of the C13 alkyl sulfate anionic surfactant

of other branched C13 alkyl sulfate anionic surfactant, wherein a, b and c add up to about 100% by weight of the C13 alkyl sulfate anionic surfactant; alternatively with a surfactant B, wherein the surfactant B comprises a mixture of C12 and C13 alkyl sulfate anionic surfactants, optionally wherein the surfactant B consists essentially of a mixture of C12 and C13 alkyl sulfate anionic surfactants, wherein the mixture of the C12 and C13 alkyl sulfate anionic surfactants comprises a mixture of surfactant isomers of Formula I and surfactant isomers of Formula II:

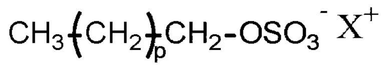

(I)

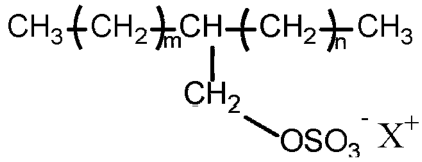

(II)

wherein 10 ≤ p ≤ 11;

wherein 8 ≤ m + n ≤ 9 and 0 ≤ m, n ≤ 9; and

wherein X is a counter cation selected from the group consisting of lithium, sodium, potassium and ammonium, preferably sodium; wherein the surfactant B comprises: from about 37% to about 50%, preferably from about 38% to about 49%, more preferably from about 40% to about 49% of the C12 alkyl sulfate anionic surfactant by weight of the surfactant B, wherein the C12 alkyl sulfate anionic surfactant comprises a 2-alkyl branched C12 alkyl sulfate; from about 50% to about 63%, preferably from about 51% to about 62%, more preferably from about 51% to about 60% of the C13 alkyl sulfate anionic surfactant by weight of the surfactant B, wherein the C13 alkyl sulfate anionic surfactant comprises a 2-alkyl branched C13 alkyl sulfate; and less or equal than about 5% of other alkyl sulfate anionic surfactants by weight of the surfactant B, preferably less or equal than about 4% of other alkyl sulfate anionic surfactants by weight of the surfactant B,

wherein other alkyl sulfate anionic surfactants are not C12 or C13 alkyl sulfate anionic surfactant.

BRIEF DESCRIPTION OF THE DRAWINGS

While the specification concludes with claims particularly pointing out and distinctly claiming the present invention, it is believed that the same will be better understood from the following description read in conjunction with the accompanying drawings in which:
FIG. 1 is an illustration for determining a third-phase volume.

DETAILED DESCRIPTION OF THE INVENTION

**Definitions of terms**

In this document, the following definitions apply unless specifically stated otherwise.

All percentages are by weight (w/w) of the composition, unless otherwise specified. "% wt." means percentage by weight. References to 'parts' e.g. a mixture of 1 part X and 3 parts Y, is a ratio by weight. All ratios or percentages are weight ratios or weight percentages unless specifically stated otherwise.

An "active composition" is the composition absent water, and an "active ingredient" is the ingredient absent its water.

"QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about".

All measurements are understood to be made at 25°C and at ambient conditions, where "ambient conditions" means at 1 atmosphere (atm) of pressure and at 65% relative humidity, unless otherwise stated. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International.

Herein "min" means "minute" or "minutes". Herein "mol" means mole. Herein "g" following a number of means "gram" or "grams". "Ex." means "example". All amounts as they pertain to listed ingredients are based on the active level and do not include carriers or by-products that may be included in commercially available materials.

Herein, "comprising" means that other steps and other ingredients can be in addition. "Comprising" encompasses the terms "consisting of" and "consisting essentially of". The compositions, methods, uses, kits, and processes described herein can comprise, consist of, and consist essentially of the elements and limitations described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

As used herein, the articles including "a" and "an" when used in a claim, are understood to mean "one or more" of what is claimed or described.

The terms "include," "includes," and "including," as used herein are meant to be non-limiting.

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100% (or 100%) of the total amount of the ingredient(s) in the composition.

The term "free of' as used herein means that the composition comprises 0% of an ingredient by total weight of the composition, thus no detectable amount of the stated ingredient.

The term "substantially free of" as used herein means less than about 1%, less than about 0.8%, less than about 0.5%, less than about 0.3%, or less than an immaterial amount of by total weight of the composition.

The term "anhydrous" as used herein, unless otherwise specified, refers to those compositions, phases or materials containing less than about 10%, more preferably less than about 5%, even more preferably less than about 3%, even more preferably zero percent of water, by weight of the composition.

The term "cleansing composition" or "personal care composition for cleansing the skin" as used herein refers to cosmetic compositions intended for topical application to the skin for cleansing.

The term "C12 alkyl sulfate anionic surfactant" refers to a sulfated anionic surfactant including an alkyl group having a total number of 12 carbon atom numbers.

The term "C13 alkyl sulfate anionic surfactant" refers to a sulfated anionic surfactant including an alkyl group having a total number of 13 carbon atom numbers.

The term "2-branched C12 alkyl sulfate anionic surfactant" as used herein refers to a C12 alkyl sulfate anionic surfactant having an alkyl chain positioned at carbon position 2.

The term "2-branched C13 alkyl sulfate anionic surfactant" as used herein refers to a C13 alkyl sulfate anionic surfactant having an alkyl chain positioned at carbon position 2.

The term "Other branched C12 alkyl sulfate anionic surfactant" as used herein refers to any other branched C12 alkyl sulfate anionic surfactants that are not a 2-branched C12 alkyl sulfate anionic surfactant, like 3-branched or 4-branched or 5-branched, etc., C12 alkyl sulfate anionic surfactant.

The term "Other branched C13 alkyl sulfate anionic surfactant" as used herein refers to any other branched C13 alkyl sulfate anionic surfactants that are not a 2-branched C13 alkyl sulfate anionic surfactant, like 3-branched or 4-branched or 5-branched, etc., C13 alkyl sulfate anionic surfactant.

The term "Other alkyl sulfate anionic surfactant" as used herein refers to any other alkyl sulfate anionic surfactants that are not C12 or C13 alkyl sulfate anionic surfactant, like C11, C14, C15, etc., C16 alkyl sulfate anionic surfactant.

The term "mixtures" as used herein is meant to include a simple combination of materials and any compounds that may result from their combination.

The term "package" as used herein includes any suitable container for a personal care composition exhibiting a viscosity from about 1.5 Pa.s (1,500 centipoise (cP)) to about 1 000 Pa.s (1,000,000 cP) as measured by the Viscosity Method as disclosed herein, including but not limited to bottle, tottle, tube, jar, non-aerosol pump and mixtures thereof.

The term "personal care composition" as used herein, refers to compositions intended for topical application to the skin, hair, or scalp. The compositions described herein are rinse-off formulations, in which the product is applied topically to the skin, hair, or scalp and then is subsequently rinsed within minutes from the skin or hair or scalp with water, or otherwise wiped off using a substrate with deposition of a portion of the composition. The compositions also may be used as shaving aids. The personal care composition is typically extrudable or dispensible from a package. The personal care compositions typically exhibit a viscosity of from about 1.5 Pa.s (1,500 centipoise (cP)) to about 1 000 Pa.s (1,000,000 cP) as measured by the T-bar Viscosity Method as disclosed herein. The personal care compositions can be in the form of liquid, semiliquid, cream, lotion or gel compositions intended for topical application to skin. Examples of personal care compositions can include but are not limited to shampoo, conditioning shampoo, body wash, moisturizing body wash, shower gels, skin cleansers, cleansing milks, hair and body wash, in shower body moisturizer, pet shampoo, shaving preparations and cleansing compositions used in conjunction with a disposable cleansing cloth.

The term "rinse-off" as used herein means the intended product usage includes application to skin or hair or scalp

followed by rinsing and/or wiping the product from the skin within a few seconds to minutes of the application step. The product is generally applied and rinsed in the same usage event, for example, a shower.

"Room temperature" refers to a temperature of 25°C.

The term "structured," as used herein means having a rheology that confers stability on the multiphase composition. The degree of structure is determined by characteristics determined by one or more of the following methods: The Young's Modulus Method, or the Zero Shear Viscosity Method or by the Ultracentrifugation Method, all in the Test Methods below. Accordingly, a cleansing phase of the personal care composition is considered "structured," if the surfactant cleansing phase has one or more of the following properties described below according to the Young's Modulus Method, or the Zero Shear Viscosity Method or by the Ultracentrifugation Method. A surfactant phase is considered to be structured, if the phase has one or more of the following characteristics:

A. a Zero Shear Viscosity of at least about 100 Pascal-seconds (Pa.s), alternatively at least about 200 Pa.s, alternatively at least about 500 Pa.s, alternatively at least about 1,000 Pa.s, alternatively at least about 1,500 Pa.s, alternatively at least about 2,500 Pa.s, alternatively at least about 5,000 Pa.s; or

B. a Structured Domain Volume Ratio as measured by the Ultracentrifugation Method described hereafter, of greater than about 40%, or at least about 50%, or at least about 55%, or at least about 60 or at least about 65%, or at least about 70%, or at least about 75%, or at least about 80%, or at least about 85%, or at least about 90%.

C. a Young's Modulus of greater than about 2 Pascal (Pa), more preferably greater than about 10 Pa, even more preferably greater than about 20 Pa, still more preferably greater than about 30 Pa, 40 Pa, 50 Pa, 75 Pa, 100 Pa, most preferably greater than 150 Pa.

The term "SLS" as used herein, means sodium lauryl sulfate.

The term "lather" as used herein, means the aerated foam which results from providing energy to aqueous surfactant mixtures, especially dilute mixtures. Lather is increased in micellar compositions compared to structured, e.g., lamellar compositions, so that a phase change during dilution to micelles typically increases lather.

The term "visually distinct" as used herein, refers to a region of the personal care composition having one average composition, as distinct from another region having a different average composition, wherein the regions are visible to the unaided naked eye. This would not preclude the distinct regions from comprising two similar phases where one phase could comprise pigments, dyes, particles, and various optional ingredients, hence a region of a different average composition. A phase generally occupies a space or spaces having dimensions larger than the colloidal or sub-colloidal components it comprises. A phase can also be constituted or reconstituted, collected, or separated into a bulk phase in order to observe its properties, e.g., by centrifugation, filtration or the like.

The personal care products, methods and uses of the products, the structures and the respective compositions as described in the Summary or as described hereinbelow are for fulfilling the technical effects or goals as set out herein. These objects and other advantages as may be apparent to those skilled in the art can be achieved through the personal cleansing products, methods and uses of the products, the structures and the respective compositions as described herein.

**BENEFITS**

Chemical impurities are sometimes found in raw materials or products utilizing raw materials. For example, 1,4-dioxane is an undesirable byproduct of detergent making. As an industrial processing solvent or chemical intermediate, 1,4-dioxane has previously been reported to be used in the production of products that may have commercial or consumer applications such as paints, adhesives, detergents, and pesticides. As such 1,4-dioxane may be present as a impurity in consumer cosmetics/toiletries, household detergents, pharmaceuticals, foods, agricultural and veterinary products and ethylene glycol-based antifreeze coolants. It is formed as a reaction byproduct during the manufacturing of ethoxylated surfactants.

Also, 1,4-dioxane is a by-product of the manufacturing process of ethoxylated surfactants produced with ethylene oxide. In other words, 1,4-dioxane is an unwanted chemical formed during the surfactant manufacturing process used to create ingredients such as ethoxylated surfactants and polyethylene glycols.

1,4-dioxane can be formed from an ethoxylated product during an unintended side reaction, known as "intramolecular chain transfer" or "backbiting". Two moles of ethylene oxide are eliminated from the ethoxylated product to form 1,4-dioxane. Backbiting can happen once for every 2 moles of ethylene oxide on the molecule.

Hence, 1,4-dioxane is a persistent impurity that forms from ethoxylate products, particularly during sulfation of ethoxylated fatty alcohols to make ethoxylated surfactants, and in those same ethoxylated surfactants during aging in the supply chain and, more slowly, in finished products that contain them.

The amount of 1,4-dioxane by-product within alkoxylated especially ethoxylated alkyl sulfates can be reduced. Based on recent advances in technology, manufacturers can remove most of the 1,4-dioxane in consumer products

through a vacuum stripping process or by improved methods of removing impurities, like 1,4-dioxane from already ethoxylated surfactants.

However, there is a desire to make personal care compositions that contain relatively very low amount or no ethoxylated surfactants, without having negative consumer noticeable trade-offs.

Initially, a comparative non-ethoxylated surfactant, namely non-ethoxylated sodium tridecyl sulfate supplied as tradename Rhodapon® TDS from Solvay or made from sulfation of the corresponding tridecyl alcohol supplied as tradename Exxal® 13 by ExxonMobil has been considered.

However, the personal care compositions did not appear to meet the structure and/or stability properties in terms of rheology or did not provide any significant structured attributes to drive any cleansing and any cosmetic attributes sought by the consumer onto skin. The personal care compositions comprising non-ethoxylated sodium tridecyl sulfate did not form lamellar phase.

To provide a personal care composition for cleansing the skin with an improved structure, an improved stability, with an optimum rheology profile, and any attributes sought by the consumer such as lather performance, mildness, etc., a specific surfactant structure needs to be found and optimized. Previously, the surfactant structure included the branched alkyl hydrocarbon chains, a hydrophilic ethoxylate spacer, and an anionic sulfate head group in combination with other ingredient components to produce personal care compositions that organize into a lamellar phase that is desirable for dispensing rheology and aesthetic.

The sodium trideceth-n sulfate is a highly branched alkyl sulfate, and able to accomplish all these structure-related needs for personal care compositions, but in the absence of ethoxylation, inventors have found that it was unable to produce the abundance of multilamellar vesicles necessary to lead to a stable lamellar phase, and does not have the resulting rheological properties for useful product use aesthetics.

Surprisingly, inventors have discovered that a surfactant A comprising a C13 alkyl sulfate anionic surfactant which is a non-ethoxylated C13 alkyl sulfate anionic surfactant.

The C13 alkyl sulfate anionic surfactant consists of:

(a) less than about 40% by weight of the C13 alkyl sulfate anionic surfactant of a linear C13 alkyl sulfate, and
(b) more than about 60% by weight of the C13 alkyl sulfate anionic surfactant of a 2-branched C13 alkyl sulfate anionic surfactant, wherein the 2-branched C13 alkyl sulfate anionic surfactant comprises: about 25% or less by weight of the 2-branched C13 alkyl sulfate anionic surfactant of 2-pentyl octyl sulfate anionic surfactant, and more than about 25% by weight of the 2-branched C13 alkyl sulfate anionic surfactant of 2-methyl dodecyl sulfate anionic surfactant, and
(c) less than about 5% by weight of the C13 alkyl sulfate anionic surfactant of other branched C13 alkyl sulfate anionic surfactant,

wherein (a), (b) and (c) add up to about 100% by weight of the C13 alkyl sulfate anionic surfactant.

Surprisingly, inventors have found by changing the nature and the distribution of the hydrocarbon branching of a 2-branched alkyl sulfate anionic surfactant for surfactant A, personal care compositions comprising the C13 alkyl sulfate anionic surfactant as described herein are structured, stable, with an optimum rheology profile. Also, further attributes sought by the consumer such as enhanced fragrance experience have been found.

Alternatively, surprisingly, inventors have discovered that a surfactant B comprising a mixture of C12 and C13 alkyl sulfate anionic surfactants, which is a non-ethoxylated surfactant B comprising a mixture of both non-ethoxylated C12 and C13 alkyl sulfate anionic surfactants.

The mixture of the C12 and C13 alkyl sulfate anionic surfactants comprises a mixture of surfactant isomers of Formula I and surfactant isomers of Formula II:

$$CH_3-(CH_2)_p-CH_2-OSO_3^- \; X^+ \qquad (I)$$

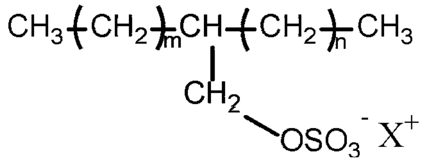

(II)

wherein $10 \leq p \leq 11$;
wherein $8 \leq m + n \leq 9$ and $0 \leq m, n \leq 9$; and
wherein X is a counter cation selected from the group consisting of lithium, sodium, potassium and ammonium, preferably sodium.

The surfactant B comprises from about 37% to about 50%, preferably from about 38% to about 49%, more preferably from about 40% to about 49% of the C12 alkyl sulfate anionic surfactant by weight of the surfactant B. The C12 alkyl sulfate anionic surfactant comprises a 2-alkyl branched C12 alkyl sulfate. The surfactant B comprises from about 50% to about 63%, preferably from about 51% to about 62%, more preferably from about 51% to about 60% of the C13 alkyl sulfate anionic surfactant by weight of the surfactant B. The C13 alkyl sulfate anionic surfactant comprises a 2-alkyl branched C13 alkyl sulfate. Also, the surfactant B comprises less or equal than about 5% of other alkyl sulfate anionic surfactants by weight of the surfactant B, preferably less or equal than about 4% of other alkyl sulfate anionic surfactants by weight of the surfactant B, wherein other alkyl sulfate anionic surfactants are not C12 or C13 alkyl sulfate anionic surfactant.

Surprisingly, inventors also have found by changing the nature and the distribution of the hydrocarbon branching of a 2-branched alkyl sulfate anionic surfactant, personal care compositions comprising the surfactant B as described herein including the specific mixture of C12 and C13 alkyl sulfate anionic surfactants are structured, stable, with an optimum rheology profile.

In addition to the specific surfactants A or B, inventors have provided a personal care composition substantially free or free of betaine. For this, the cleansing phase of the personal care composition comprises from about 0.1% to about 20% by weight of the personal care composition, of a zwitterionic surfactant, wherein the zwitterionic surfactant comprises an hydroxysultaine and a structuring system comprising from about 3% to about 10% by weight of the personal care composition, of an electrolyte.

Also, further attributes sought by the consumer have been found such as lather performance, mildness, combatting any drying effects of the surfactants, deposition of benefit agents, enhanced fragrance experience or malodor control; and/or any improved skin-feel attributes such as skin feeling less oily and/or sticky.

## PERSONAL CARE COMPOSITION

A personal care composition is provided and comprises at least a cleansing phase and a benefit phase.

## CLEANSING PHASE

The cleansing phase comprises an aqueous structured surfactant phase.

The cleansing phase comprises an aqueous structured surfactant phase. The cleansing phase includes a surfactant A, wherein the surfactant A comprises C13 alkyl sulfate anionic surfactant and a zwitterionic surfactant (as a cosurfactant).

The personal care composition comprises from about 1% to about 20%, preferably from about 2% to about 18%, more preferably from about 3% to about 15%, most preferably from about 4% to about 13% by weight of the composition, of a surfactant A.

The personal care composition comprises from about 1% to about 20%, preferably from about 2% to about 18%, more preferably from about 3% to about 15%, most preferably from about 4% to about 13% by weight of the composition, of a C13 alkyl sulfate anionic surfactant.

Alternatively, the cleansing phase includes a surfactant B, wherein the surfactant B comprises a mixture of C12 and C13 alkyl sulfate anionic surfactants; and a zwitterionic surfactant (as a cosurfactant).

In that alternative aspect, the personal care composition comprises from about 1% to about 20%, preferably from about 2% to about 18%, more preferably from about 3% to about 15%, even more preferably from about 4% to about 13%, most preferably from about 5% to 8% by weight of the composition, of a surfactant B.

The surfactant A or surfactant B can help to provide a cleaning benefit, lather properties, and rheology properties to the compositions.

### SURFACTANT A

The surfactant A comprises C13 alkyl sulfate anionic surfactant. The C13 alkyl sulfate anionic surfactant as described herein can be used for personal care compositions selected from the group consisting of personal bar soap, hand soap, shower gels, a shower or bath cream, a foaming body wash, and mixtures thereof.

The C13 alkyl sulfate anionic surfactant consists of: a) less than about 40% by weight of the C13 alkyl sulfate anionic surfactant of a linear C13 alkyl sulfate, b) more than about 60% by weight of the C13 alkyl sulfate anionic surfactant of a 2-branched C13 alkyl sulfate anionic surfactant; and c) less than about 5% by weight of the C13 alkyl sulfate anionic surfactant of other branched C13 alkyl sulfate anionic surfactant, wherein (a), (b) and (c) add up to 100% by weight of the C13 alkyl sulfate anionic surfactant.

The 2-branched C13 alkyl sulfate anionic surfactant comprises: about 25% or less by weight of the 2-branched C13 alkyl sulfate anionic surfactant of 2-pentyl octyl sulfate anionic surfactant, and more than about 25% by weight of the 2-branched C13 alkyl sulfate anionic surfactant of 2-methyl dodecyl sulfate anionic surfactant.

Alternatively, the 2-branched C13 alkyl sulfate anionic surfactant comprises 2-branched alkyl chains: about 25% or less by weight of the 2-branched alkyl chains of 2-pentyl octyl, and more than about 25% by weight of the 2-branched alkyl chains of 2-methyl dodecyl.

By C13 alkyl sulfate anionic surfactant, it is meant that the alkyl sulfate anionic surfactant comprises an alkyl chain which consists of 13 carbon atoms. Thus, for blends of alkyl sulfate anionic surfactant having an average chain length of 13 carbon atoms, only those alkyl sulfate anionic surfactants which comprise a C13 alkyl chain fall under the definition of C13 alkyl sulfate anionic surfactant.

For blends of alkyl sulfate anionic surfactant comprising a mixture of different chain lengths including a C13 alkyl subfraction, independent of the average alkyl chain length, solely this C13 alkyl subfraction falls under the definition of C13 alkyl sulfate anionic surfactant.

With regards to the specific degree and type of C2-branching, the C13 alkyl sulfate anionic surfactant may consist of: a) less than about 30%, preferably from about 5.0% to about 25% by weight of the C13 alkyl sulfate anionic surfactant of the linear C13 alkyl sulfate; b) more than about 70%, preferably from about 75% to about 95% by weight of the C13 alkyl sulfate anionic surfactant of the 2-branched C13 alkyl sulfate anionic surfactant; and c) less than about 3.0%, preferably from about 0.1% to about 2.0% by weight of the C13 alkyl sulfate anionic surfactant of other branched C13 alkyl sulfate anionic surfactant.

The 2-branched C13 alkyl sulfate anionic surfactant may comprise: less than about 20% , preferably from about 5.0% to about 20%, more preferably from about 10% to about 20%, by weight of the 2-branched alkyl chains of 2-pentyl octyl, and more than about 30%, preferably from about 30% to about 50%, more preferably from about 33% to about 50%, by weight of the 2-branched alkyl chains of 2-methyl dodecyl.

Alternatively, the 2-branched C13 alkyl sulfate anionic surfactant may comprise less than about 20%, preferably from about 5.0% to about 20%, more preferably from about 10% to about 20%, by weight of the 2-branched C13 alkyl sulfate anionic surfactant of 2-pentyl-1-octyl sulfate anionic surfactant, and more than about 30%, preferably from about 30% to about 50%, more preferably from about 33% to about 50%, by weight of the 2-branched C13 alkyl sulfate anionic surfactant of 2-methyl-1-dodecyl sulfate anionic surfactant.

Alternatively, the 2-branched C13 alkyl sulfate anionic surfactant may comprise less than about 20%, preferably from about 5.0% to about 20%, more preferably from about 10% to about 20%, by weight of the C13 alkyl sulfate anionic surfactant of 2-pentyl-1-octyl sulfate anionic surfactant, and more than about 28%, preferably from about 28% to about 50%, more preferably from about 29% to about 50%, by weight of the C13 alkyl sulfate anionic surfactant of 2-methyl-1-dodecyl sulfate anionic surfactant.

The remaining fraction within the 2-branched C13 alkyl sulfate anionic surfactant can comprise 2-ethyl-1-undecyl sulfate anionic surfactant (preferably at a level about 25% or less, more preferably about 20% or less, most preferably from about 10% to about 20%, by weight of the 2-branched C13 alkyl sulfate anionic surfactant), 2-propyl-1-decyl sulfate anionic surfactant (preferably at a level about 25% or less, more preferably about 20% or less, most preferably from about 10% to about 18% by weight of the 2-branched C13 alkyl sulfate anionic surfactant) and 2-butyl-1-nonyl sulfate anionic surfactant (preferably at a level about 25% or less, more preferably about 20% or less, most preferably from about 5% to about 18%, by weight of the 2-branched C13 alkyl sulfate anionic surfactant).

Alternatively, the remaining fraction within the 2-branched C13 alkyl sulfate can comprise 2-ethyl-1-undecyl sulfate anionic surfactant (preferably at a level about 25% or less, more preferably about 20% or less, most preferably from about 10% to about 18% by weight of the C13 alkyl sulfate anionic surfactant), 2-propyl-1-decyl sulfate anionic surfactant (preferably at a level about 25% or less, more preferably about 20% or less, most preferably from about 10% to about 15% by weight of the C13 alkyl sulfate anionic surfactant) and 2-butyl-1-nonyl sulfate anionic surfactant (preferably at a level about 25% or less, more preferably about 20% or less, most preferably from about 5% to about 15% by weight of the C13 alkyl sulfate anionic surfactant).

Hence, the distribution of the 2-branched C13 alkyl sulfate with 2-methyl-1-dodecyl sulfate, 2-pentyl-1-octyl sulfate, 2-ethyl-1-undecyl sulfate, 2-propyl-1-decyl sulfate and 2-butyl-1-nonyl sulfate can be provided either by weight of the 2-branched C13 alkyl sulfate anionic surfactant, or by weight of the C13 alkyl sulfate anionic surfactant.

As such, the alkyl chains of the C13 alkyl sulfate anionic surfactant are highly branched, having an increased methyl to pentyl branching ratio compared to other highly branched alcohols such as for example those sold under the Isalchem® trademark which have a much higher pentyl to methyl branching ratio. The average degree of branching is much higher than lower branched alkyl alcohols produced via the OXO process, such as those sold under the Neodol® trademark. Such Neodol® alkyl alcohols have a weight average degree of branching of around 18%.

The personal care composition may be substantially free, or free of alkoxylated anionic sulfate surfactant. Preferably, the personal care composition may be substantially free, or free of ethoxylated anionic sulfate surfactant.

The C13 alkyl sulfate anionic surfactant is free of alkoxylated anionic sulfate surfactant. In other words, the C13 alkyl sulfate anionic surfactant is free of alkoxylation.

Thus the C13 alkyl sulfate anionic surfactant is free of ethoxylated anionic sulfate surfactant. In other words, the

C13 alkyl sulfate anionic surfactant is free of ethoxylation.

The average degree of alkoxylation is the mol average degree of alkoxylation (*i.e.*, mol average alkoxylation degree) of all the C13 alkyl sulfate anionic surfactant. Hence, when calculating the mol average alkoxylation degree, the moles of C13 non-alkoxylated sulfate anionic surfactant are included:

Mol average alkoxylation degree = (x1 * alkoxylation degree of surfactant 1 + x2 * alkoxylation degree of surfactant 2 + ....) / (x1 + x2 + ....)

wherein x1, x2, ... are the number of moles of each alkyl (or alkoxy) sulfate anionic surfactant of the mixture and alkoxylation degree is the number of alkoxy groups in each alkyl sulfate anionic surfactant.

Suitable alkyl sulfate anionic surfactants can be made using the following process.

A two-step process can be used to produce branched aldehyde products from linear alpha olefin feedstocks, from which the C13 alkyl sulfate anionic surfactants as described herein can be derived. The two-step process uses a rhodium organophosphorus catalyst for both a first process step and a second step. The first step is an isomerization reaction step and the second process step is a hydroformylation reaction step. The branched aldehydes can undergo a further hydrogenation step to produce branched alcohols.

The isomerization and hydroformylation reactions disclosed herein can be catalyzed by a rhodium organophosphorus catalyst which can be at least one of: (1) an organometallic complex of rhodium and one type of an organophosphorus ligand; (2) or an organometallic complex of rhodium and more than one type of an organophosphorus ligand.

The organophosphorous ligand can be a phosphine. In a nonlimiting example of a phosphine ligand, the phosphine ligand can be triphenylphosphine. The organophosphorous ligand can also be a phosphite. In a nonlimiting example of a phosphite ligand, the phosphite ligand can be tris (2,4-di-t-butylphenyl) phosphite. A mixture of organophosphorous ligands of different types can also be used, such as a mixture of a phosphine and a phosphite. In a nonlimiting example of a mixture of organophosphorous ligands, the organophosphorous ligands can be a mixture of triphenylphosphine and tris (2,4-di-t-butylphenyl) phosphite. The reaction system can contain an inert high-boiling solvent, for example a polyalphaolefin. The first catalyst can be formed when the molar ratio of phosphorous to rhodium is in a range of 1:1 to 1000:1, or 5:1 to 50:1, or 15:1 to 25:1. The rhodium concentration can be in a range of 1 ppm to 1000 ppm, or 10 ppm to 200 ppm, or 25 ppm to 75 ppm. The Carbon Monoxide (CO) to Hydrogen ($H_2$) molar ratio can be in a range of 10:1 to 1:10, or 2:1 to 1:2, or 1.3:1 to 1:1.3.

During the isomerization reaction, the first step can be a reaction isomerizing a linear alpha olefin in the presence of Carbon Monoxide (CO) and Hydrogen ($H_2$) at a first pressure. The isomerizing can be catalyzed by the rhodium organophosphorus catalyst which can be at least one of: (1) an organometallic complex of rhodium and one type of an organophosphorus ligand; (2) or an organometallic complex of rhodium and more than one type of an organophosphorus ligand. The isomerization reactions can produce an isomerized olefin comprising linear internal olefins of the same or different types.

The isomerization step can be performed at a temperature in a range of 30°C to 500°C, or 50°C to 150°C, or 70°C to 100°C. The isomerization step can be performed at a gauge pressure in a range of 0.1 bar (0.01MPa above atmospheric) to 10 bar (1MPa above atmospheric), or 0.5 bar (0.05MPa above atmospheric) to 5 bar (0.5MPa above atmospheric), or 1 bar (0.1MPa above atmospheric) to 2 bar (0.2MPa above atmospheric).

The isomerizing step can produce a reaction product comprising a 20 wt.% or greater isomerized olefin, or a 40 wt.% or greater isomerized olefin, or a 60 wt.% or greater isomerized olefin, or a 90 wt.% or greater isomerized olefin.

During the hydroformylation reaction step, the isomerized olefin is hydroformylated in the presence of CO and $H_2$ at a second pressure higher than the first pressure to produce a branched aldehyde. The hydroformylation reaction can be catalyzed by the rhodium organophosphorus catalyst which can be at least one of: (1) an organometallic complex of rhodium and one type of an organophosphorus ligand; (2) or an organometallic complex of rhodium and more than one type of an organophosphorus ligand. The resultant branched aldehyde is a 2-alkyl branched aldehyde. The linear alpha olefin is 1-dodecene and the branched aldehyde is a branched C13 aldehyde.

The hydroformylating step can be performed at a temperature in a range of 30°C to 500°C, or 50°C to 150°C, or 70°C to 100°C. The hydroformylating step can be performed at a gauge pressure in a range of 5 bar (0.5MPa above atmospheric) to 400 bar (40MPa above atmospheric), or 10 bar (1.0MPa above atmospheric) to 100 bar (10MPa above atmospheric), or 15 bar (1.5MPa above atmospheric) to 20 bar (2MPa above atmospheric).

The hydroformylating step can produce a reaction product comprising a 25 wt.% or greater branched aldehyde, or a 40 wt.% or greater branched aldehyde, or a 60 wt.% or greater branched aldehyde, or a 90 wt.% or greater branched aldehyde.

The products of the hydroformylation reaction can be distilled. The process can have the step of separating the branched aldehyde products resulting from hydroformylation as an overhead product from the first catalyst stream via a distillation process. The distillation step can be performed at a temperature in a range of 100°C to 200°C, or 125°C to 175°C. The distillation step can be performed under vacuum at a pressure of less than 500 millibar absolute (0.05MPa), or

less than 100 millibar absolute (0.01MPa), or less than 30 millibar absolute (0.003MPa),

The process can also have the steps of: hydrogenating the branched aldehyde product in the presence of a hydrogenation catalyst to produce a branched alcohols product composition. The hydrogenating catalyst can be a base metal catalyst, a supported nickel catalyst, a supported cobalt catalyst, a Raney® (W. R. Grace & Co., 7500 Grace Drive, Columbia, MD 21044) nickel catalyst or a precious metal catalyst. The hydrogenating step can be performed at a temperature in a range of 30°C to 500°C, or 50°C to 200°C, or 100°C to 150°C. The hydrogenating step can be performed at a gauge pressure in a range of 5 bar (0.5MPa above atmospheric) to 400 bar (40MPa above atmospheric), or 10 bar (1MPa above atmospheric) to 100 bar (10MPa above atmospheric), or 30 bar (3MPa above atmospheric) to 50 bar (5MPa above atmospheric).

The hydrogenating step can produce a reaction product comprising 25 wt.% or greater branched alcohols, or 40 wt.% or greater branched alcohols, or 60 wt.% or greater branched alcohols, or 90 wt.% or greater branched alcohols.

The C12 olefin source used in the hydroformylation to make the starting C13 aldehydes and subsequent alcohols of use in the present disclosure can have low levels of impurities that lead to impurities in the starting C13 alcohol and therefore also in the C13 alkyl sulfate. While not intending to be limited by theory, such impurities present in the C12 olefin feed can include vinylidene olefins, branched olefins, paraffins, aromatic components, and low levels of olefins having chain-lengths other than 12 carbons. Branched and vinylidene olefins are typically at or below 5% in C12 alpha olefin sources. Impurities in the resulting C13 alcohols can include low levels of linear and branched alcohols in the range of C10 to C16 alcohols, especially C11 and C15 alcohols, typically less than 2% by weight of the mixture, preferably less than 1%; low levels of branching in positions other than the 2-alkyl position resulting from branched and vinylidene olefins are typically less than about 5% by weight of the alcohol mixture, preferably less than 2%; paraffins and olefins, typically less than 1% by weight of the alcohol mixture, preferably less than about 0.5%; low levels of aldehydes with a carbonyl value typically below 500 mg/kg, preferably less than about 200 mg/kg. These impurities in the alcohol can result in low levels of paraffin, linear and branched alkyl sulfates having total carbon numbers other than C13, and alkyl sulfates with branching in positions other than the 2-alkyl location, wherein these branches can vary in length, but are typically linear alkyl chains having from 1 to 6 carbons. The step of hydroformylation can also yield impurities, such as linear and branched paraffins, residual olefin from incomplete hydroformylation, as well as esters, formates, and heavy-ends (dimers, trimers). Impurities that are not reduced to alcohol in the hydrogenation step may be removed during the final purification of the alcohol by distillation.

Alkyl sulfates are typically prepared by the reaction of fatty alcohols with sulfur trioxide ($SO_3$) or its derivatives or by the reaction of unsaturated compounds with sulfuric acid. Processes using sulfur trioxide in particular have gained prominence for fabricating alkyl sulfate anionic surfactants for use in detergent compositions.

Suitable derivatives of sulfur trioxide include sulfur trioxide complexes such as chlorosulfonic acid, sulfuric acid, or sulfamic acid. Sulfur trioxide is preferred since it tends to result in more pure products. The sulfation reaction typically takes place in a continuous process using a cascade, falling film or tube bundle reactor, with the sulfur trioxide being applied in an equimolar or small excess, usually in a temperature range of 20°C to 60°C, with the reaction temperature being determined at least partially by the solidification point of the fatty alcohol in the reaction. The reaction typically results in the acid form of the C13 alkyl sulfate anionic surfactant which is typically neutralized in a subsequent step, using an alkali such as sodium hydroxide, potassium hydroxide, magnesium hydroxide lithium hydroxide, calcium hydroxide, ammonium hydroxide, monoethanolamine, diethanolamine, triethanolamine, monoisopropanolamine, diamines, polyamines, primary amines, secondary amines, tertiary amines, amine containing surfactants, and mixtures thereof.

Also, it is well known that the process of sulfating fatty alcohols to yield alkyl sulfate anionic surfactants also yields various impurities. The exact nature of these impurities depends on the conditions of sulfation and neutralization. Generally, however, the impurities of the sulfation process include one or more inorganic salts, unreacted fatty alcohol, and olefins ("The Effect of Reaction By-Products on the Viscosities of Sodium Lauryl Sulfate Solutions," Journal of the American Oil Chemists' Society, Vol. 55, No. 12, p. 909-913 (1978), C.F. Putnik and S.E. McGuire). The level of non-alkyl sulfate impurities in the alkyl sulfate anionic surfactant of the present disclosure can be less than 6% by weight, preferably less than 4% by weight, and most preferably less than 2% by weight of the alkyl sulfate anionic surfactant.

For alkyl alkoxy sulfates, the fatty alcohol is first alkoxylated before sulfation. Alkoxylation is a process that reacts lower molecular weight epoxides (oxiranes), such as ethylene oxide, propylene oxide, and butylene oxide with the fatty alcohol. These epoxides are capable of reacting with the fatty alcohol using various base or acid catalysts. In base catalyzed alkoxylation, an alcoholate anion, formed initially by reaction with a catalyst (alkali metal, alkali metal oxide, carbonate, hydroxide, or alkoxide), nucleophilically attacks the epoxide.

Traditional alkaline catalysts for alkoxylation include potassium hydroxide and sodium hydroxide, which give rise to a somewhat broader distribution of alkoxylates. Other catalysts have been developed for alkoxylation that provide a more narrow distribution of alkoxylate oligomers. Suitable examples of narrow range alkoxylation catalysts include many alkaline earth (Mg, Ca, Ba, Sr, etc.) derived catalysts, Lewis acid catalysts, such as Zirconium dodecanoxide sulfate, and certain boron halide catalysts. A specific average degree of alkoxylation may be achieved by selecting the starting quantities of fatty alcohol and ethylene oxide or by blending together varying amounts of alkoxylated surfactants differing

from one another in average degree of alkoxylation.

The C13 alkyl sulfate anionic surfactant may be a sodium C13 alkyl sulfate anionic surfactant, potassium C13 alkyl sulfate anionic surfactant, triethylammonium C13 alkyl sulfate anionic surfactant, or ammonium C13 alkyl sulfate anionic surfactant.

SURFACTANT B

Alternatively, the surfactant B as described herein can be used for a personal care composition, wherein the personal care composition is selected from the group consisting of a personal bar soap, a hand soap, shower gels, a shower or bath cream, a foaming body wash, and mixtures thereof.

The surfactant B comprises a mixture of C12 and C13 alkyl sulfate anionic surfactants. Optionally, the surfactant B may consist essentially of a mixture of C12 and C13 alkyl sulfate anionic surfactants. The mixture of the C12 and C13 alkyl sulfate anionic surfactants comprises a mixture of surfactant isomers of Formula I and surfactant isomers of Formula II:

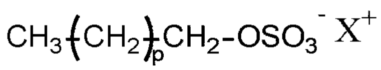
(I)

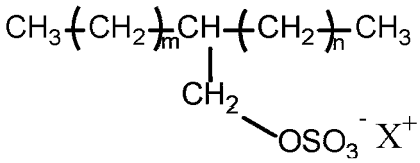
(II)

wherein $10 \leq p \leq 11$;
wherein $8 \leq m + n \leq 9$ and $0 \leq m, n \leq 9$;
wherein X is a counter cation selected from the group consisting of lithium, sodium, potassium and ammonium, preferably sodium.

The surfactant B comprises from about 37% to about 50% of the C12 alkyl sulfate anionic surfactant by weight of the surfactant B, wherein the C12 alkyl sulfate anionic surfactant comprises a 2-alkyl branched C12 alkyl sulfate. The surfactant B comprises from about 50% to about 63% of the C13 alkyl sulfate anionic surfactant by weight of the surfactant B, wherein the C13 alkyl sulfate anionic surfactant comprises a 2-alkyl branched C13 alkyl sulfate. Also, the surfactant B comprises less or equal than about 5% of other alkyl sulfate anionic surfactants by weight of the surfactant B. Other alkyl sulfate anionic surfactants are not C12 or C13 alkyl sulfate anionic surfactant.

2-alkyl branched or "beta" branched primary alkyl sulfates are sulfate surfactants that have branching at the C2 position. C1 position is at the carbon atom covalently attached to the sulfate moiety as shown in Formula (II).

Preferably, the surfactant B may comprise from about 38% to about 49% of the C12 alkyl sulfate anionic surfactant by weight of the surfactant B, wherein the C12 alkyl sulfate anionic surfactant comprises a 2-alkyl branched C12 alkyl sulfate. The surfactant B may preferably comprise from about 51% to about 62% of the C13 alkyl sulfate anionic surfactant by weight of the surfactant B, wherein the C13 alkyl sulfate anionic surfactant comprises a 2-alkyl branched C13 alkyl sulfate. The surfactant B may comprise less or equal than about 4% of other alkyl sulfate anionic surfactants by weight of the surfactant B, wherein other alkyl sulfate anionic surfactants are not C12 or C13 alkyl sulfate anionic surfactant.

More preferably, the surfactant B may comprise from about 40% to about 49% of the C12 alkyl sulfate anionic surfactant by weight of the surfactant B, wherein the C12 alkyl sulfate anionic surfactant comprises a 2-alkyl branched C12 alkyl sulfate. The surfactant B may more preferably comprise from about 51% to about 60% of the C13 alkyl sulfate anionic surfactant by weight of the surfactant B, wherein the C13 alkyl sulfate anionic surfactant comprises a 2-alkyl branched C13 alkyl sulfate. The surfactant B may comprise less or equal than about 4% of other alkyl sulfate anionic surfactants by weight of the surfactant B, wherein other alkyl sulfate anionic surfactants are not C12 or C13 alkyl sulfate anionic surfactant.

Most preferably, the surfactant B may comprise from about 43% to about 46% of the C12 alkyl sulfate anionic surfactant by weight of the surfactant B, wherein the C12 alkyl sulfate anionic surfactant comprises a 2-alkyl branched C12 alkyl sulfate. The surfactant B may more preferably comprise from about 51% to about 57% of the C13 alkyl sulfate anionic surfactant by weight of the surfactant B, wherein the C13 alkyl sulfate anionic surfactant comprises a 2-alkyl branched C13 alkyl sulfate. The surfactant B may comprise less or equal than about 3% of other alkyl sulfate anionic surfactants by weight of the surfactant B, wherein other alkyl sulfate anionic surfactants are not C12 or C13 alkyl sulfate anionic surfactant.

As shown in Table 3 more below, an example of a surfactant B is the sulfated product obtained from commercially available tradename LIAL®123A alcohol supplied from SASOL (See also Table 2).

In that example, the surfactant B comprises about 45.2%, or, depending on the commercial batch from the starting alcohol, from about 38% to about 48% of the C12 alkyl sulfate anionic surfactant by weight of the surfactant B, wherein the C12 alkyl sulfate anionic surfactant comprises a 2-alkyl branched C12 alkyl sulfate (at about 23.6% by weight of the surfactant B). The surfactant B comprises from about 51.9%, or, depending on the commercial batch from the starting alcohol, from about 52% to about 62% of the C13 alkyl sulfate anionic surfactant by weight of the surfactant B, wherein the C13 alkyl sulfate anionic surfactant comprises a 2-alkyl branched C13 alkyl sulfate (at about 25.7% by weight of the surfactant B). The surfactant B comprises about 2.9%, or, depending on the commercial batch, from the starting alcohol, less than about 3% of other alkyl sulfate anionic surfactants by weight of the surfactant B, wherein other alkyl sulfate anionic surfactants are not C12 or C13 alkyl sulfate anionic surfactant.

In another example, a surfactant B may be the sulfated product obtained from commercially available tradename ISALCHELM®123A alcohol supplied from SASOL. In that example as shown in Table 4, the surfactant B comprises about 42.8%, or, depending on the commercial batch from the starting alcohol, from about 37% to about 48% of the C12 alkyl sulfate anionic surfactant by weight of the surfactant B, wherein the C12 alkyl sulfate anionic surfactant comprises a 2-alkyl branched C12 alkyl sulfate. The surfactant B comprises from about 55.1%, or, depending on the commercial batch from the starting alcohol, from about 52% to about 63% of the C13 alkyl sulfate anionic surfactant by weight of the surfactant B, wherein the C13 alkyl sulfate anionic surfactant comprises a 2-alkyl branched C13 alkyl sulfate. The surfactant B comprises about 2.1%, or less than about 3% or, depending on the commercial batch from the starting alcohol, of other alkyl sulfate anionic surfactants by weight of the surfactant B, wherein other alkyl sulfate anionic surfactants are not C12 or C13 alkyl sulfate anionic surfactant.

According to the technical data information from the starting alcohol, the surfactant B comprises from about 37% to about 50%, preferably from about 38% to about 49%, more preferably from about 40% to about 49%, most preferably about 43% to about 46% of the C12 alkyl sulfate anionic surfactant by weight of the surfactant B, wherein the C12 alkyl sulfate anionic surfactant comprises a 2-alkyl branched C12 alkyl sulfate. The surfactant B comprises from about 50% to about 63%, preferably from about 51% to about 62%, more preferably from about 51% to about 60%, most preferably from about 51% to about 57% of the C13 alkyl sulfate anionic surfactant by weight of the surfactant B, wherein the C13 alkyl sulfate anionic surfactant comprises a 2-alkyl branched C13 alkyl sulfate. Also, the surfactant B comprises less or equal than about 5%, preferably less or equal than about 4%, more preferably less or equal than about 3% of other alkyl sulfate anionic surfactants by weight of the surfactant B. Other alkyl sulfate anionic surfactants are not C12 or C13 alkyl sulfate anionic surfactant.

The alcohol starting materials can be analyzed by gas chromatography with flame ionization detection (GC/FID).

The samples are typically assessed by diluting the starting Alcohol Example to about 0.1% (v/v) in a volatile alcohol such as methanol or ethanol. The prepared samples were injected (1 μL Injection Volume) into a Splitless Inlet at 280°C and onto an Agilent DB-1, 30 m x 0.250 mm ID, 0.25 μm film thickness capillary GC column. The $H_2$ carrier gas is in constant pressure mode of 0.68 Bar (10.00 psi). The oven temperature program [50°C (2 min), ramped (10°C/min) to 300°C ( 3 min)] has a total run time of 30 minutes. The flame ionization detection (FID) temperature is 320°C with 30 mL/min $H_2$ flow, 300 mL/min Air flow, and 30 mL/min Makeup ($N_2$) flow.

The sulfation of the alcohol starting materials does not change the alkyl chain distribution in the resulting surfactant B. The surfactants B as sulfates could be separated and analyzed by Ultra High Performance Liquid Chromatography (UHPLC) and detected by both charged aerosol detection (CAD) and high-resolution mass spectrometry (HRMS). However, the results as shown in Table 3 are only indicative since the method is not yet standardized. A plurality of single and pure branched isomers would need to be assessed and quantified by UHPLC-CAD to validate the method.

For Table 3 results, an inverse gradient could be used such that the mobile phase composition at both detectors was constant throughout the separation ensuring that analyte response was not biased differences in organic composition of the mobile phase at the elution time. The CAD response is structure agnostic while electrospray (ESI) MS response may also be influenced by analyte structure. The surfactants B as sulfates possess a hard negative charge that should minimize the variation in ionization efficiency across different branched and chain length species. The ESI MS response is consistent, when the chain length distribution is determined by both UHPLC-CAD and UHPLC-HRMS on Surfactant B Example 1. The HRMS provides a molar response, and the CAD provides a mass response. The HRMS data can be converted to mass response for comparison.

The relative chain length distributions for the starting alcohol and the resulted surfactant B comprising a mixture of C12 and C13 alkyl sulfate anionic surfactants are the same with some typical margin errors.

The surfactant B may have an average degree of branching of greater than about 90%, preferably greater than about 92%, more preferably from about 92% to about 100%. Alternatively, the surfactant B may have an average degree of branching from about 40% to about 60% by weight, preferably from about 45% to about 55% by weight, more preferably from about 46% to about 54% by weight, most preferably from about 47% to about 54%.

For instance, the surfactant B may be the sulfated product obtained from commercially available tradename LIAL®123A alcohol supplied from SASOL has an average degree of branching of about 49.4%. Depending on the commercial batch of the starting alcohol, the surfactant B may have an average degree of branching from about 40% to

about 60% by weight, preferably from about 45% to about 55% by weight, more preferably from about 46% to about 54% by weight, most preferably from about 47% to about 54%.

As another instance, the surfactant B may be the sulfated product obtained from commercially available tradename ISALCHEM®123A alcohol supplied from SASOL has an average degree of branching of about 92.6%. Depending on the commercial batch of the starting alcohol, the surfactant B may have an average degree of branching of greater than about 90%, preferably of greater than about 92%, more preferably from about 92% to about 100%.

The C12 alkyl sulfate anionic surfactant of the surfactant B may consist of: a) less than about 70% of a linear C12 alkyl sulfate by weight of the C12 alkyl sulfate anionic surfactant; b) more than about 30% of a 2-alkyl branched C12 alkyl sulfate by weight of the C12 alkyl sulfate anionic surfactant, wherein; and c) less than about 10% of other branched C12 alkyl sulfates by weight of the C12 alkyl sulfate anionic surfactant; wherein (a), (b) and (c) add up to about 100% by weight of the C12 alkyl sulfate anionic surfactant.

**In** that aspect, the 2-alkyl branched C12 alkyl sulfate may comprise more than about 25% of 2-methyl undecyl sulfate by weight the 2-alkyl branched C12 alkyl sulfate; and about 25% or less of 2-ethyl decyl sulfate by weight of the 2-alkyl-branched C12 alkyl sulfate.

Alternatively, the 2-alkyl branched C12 alkyl sulfate may comprise 2-alkyl branched C12 alkyl chains and: more than about 25% of 2-methyl undecyl by weight the 2-alkyl branched C12 alkyl chains; and about 25% or less of 2-ethyl decyl by weight of the 2-alkyl branched C12 alkyl chains.

By C12 alkyl sulfate anionic surfactant, it is meant that the alkyl sulfate anionic surfactant comprises an alkyl chain which consists of 12 carbon atoms. Thus, for blends of alkyl sulfate anionic surfactant having an average chain length of 12 carbon atoms, only those alkyl sulfate anionic surfactants which comprise a C12 alkyl chain fall under the definition of C12 alkyl sulfate anionic surfactant.

For blends of alkyl sulfate anionic surfactant comprising a mixture of different chain lengths including a C12 alkyl subfraction, independent of the average alkyl chain length, solely this C12 alkyl subfraction falls under the definition of C12 alkyl sulfate anionic surfactant.

With regards to the specific degree and type of C2-branching, the C12 alkyl sulfate anionic surfactant may consist of: a) less than or equal to about 60% of the linear C12 alkyl sulfate by weight of the C12 alkyl sulfate anionic surfactant; b) more than or equal to about 40%, of the 2-alkyl branched C12 alkyl sulfate by weight of the C12 alkyl sulfate anionic surfactant; and c) less than about 10% of other branched C12 alkyl sulfates by weight of the C12 alkyl sulfate anionic surfactant; wherein (a), (b) and (c) add up to about 100% by weight of the C12 alkyl sulfate anionic surfactant.

Preferably, the C12 alkyl sulfate anionic surfactant may consist of: a) from about 45% to about 55% of the linear C12 alkyl sulfate by weight of the C12 alkyl sulfate anionic surfactant; b) from about 45% to about 55% of the 2-alkyl branched C12 alkyl sulfate by weight of the C12 alkyl sulfate anionic surfactant; and c) from about 0% to about 5% of other branched C12 alkyl sulfates by weight of the C12 alkyl sulfate anionic surfactant; wherein (a), (b) and (c) add up to about 100% by weight of the C12 alkyl sulfate anionic surfactant.

More preferably, the C12 alkyl sulfate anionic surfactant may consist of: a) from about 45% to 51% of the linear C12 alkyl sulfate by weight of the C12 alkyl sulfate anionic surfactant; b) from about 49% to about 55% of the 2-alkyl branched C12 alkyl sulfate by weight of the C12 alkyl sulfate anionic surfactant; and c) from about 0% to about 5% of other branched C12 alkyl sulfates by weight of the C12 alkyl sulfate anionic surfactant; wherein (a), (b) and (c) add up to about 100% by weight of the C12 alkyl sulfate anionic surfactant.

The above features for the C12 alkyl sulfate anionic surfactant can be exemplified when the surfactant B is the sulfated product obtained from commercially available tradename LIAL®123A alcohol supplied from SASOL.

Alternatively, for instance when the surfactant B is the sulfated product obtained from commercially available tradename ISALCHEM®123A alcohol supplied from SASOL, the C12 alkyl sulfate anionic surfactant may consist of: a) less than about 10%, preferably less than about 9%, more preferably from about 0% to about 8% of the linear C12 alkyl sulfate by weight of the C12 alkyl sulfate anionic surfactant; b) more than about 90%, preferably more than about 91%, more preferably from about 92% to about 100% of the 2-alkyl branched C12 alkyl sulfate by weight of the C12 alkyl sulfate anionic surfactant; and c) from about 0% to about 5% of other branched C12 alkyl sulfates by weight of the C12 alkyl sulfate anionic surfactant; wherein (a), (b) and (c) add up to about 100% by weight of the C12 alkyl sulfate anionic surfactant.

In that aspect, the 2-alkyl branched C12 alkyl sulfate may comprise more than about 25% of 2-methyl undecyl sulfate by weight the 2-alkyl branched C12 alkyl sulfate; and about 25% or less of 2-ethyl decyl sulfate by weight of the 2-alkyl-branched C12 alkyl sulfate.

In any cases, the 2-alkyl branched C12 alkyl sulfate may comprise: more than about 30% of 2-methyl undecyl sulfate by weight of the 2-alkyl branched C12 alkyl sulfate; less than about 20% of 2-ethyl decyl sulfate by weight of the 2-alkyl branched C12 alkyl sulfate; less than about 20% of 2-propyl nonyl sulfate by weight of the 2-alkyl branched C12 alkyl sulfate; and more than about 25% of 2-butyl octyl sulfate and 2-pentyl heptyl sulfate and other 2-alkyl branched C12 alkyl sulfates by weight of the 2-alkyl branched C12 alkyl sulfate.

Preferably, the 2-alkyl branched C12 alkyl sulfate may comprise: from about 30.5% to about 35% of 2-methyl undecyl sulfate by weight of the 2-alkyl branched C12 alkyl sulfate; from about 15% to about 19.5% of 2-ethyl decyl sulfate

by weight of the 2-alkyl branched C12 alkyl sulfate; from about 15% to about 19.5% of 2-propyl nonyl sulfate by weight of the 2-alkyl branched C12 alkyl sulfate; and from about 26% to about 35% of 2-butyl octyl sulfate and 2-pentyl heptyl sulfate and other 2-alkyl branched C12 alkyl sulfates by weight of the 2-alkyl branched C12 alkyl sulfate.

Alternatively, the 2-alkyl branched C12 alkyl sulfate may comprise: more than about 30%, preferably from about 30.5% to about 35% of 2-methyl undecyl by weight of the 2-alkyl branched C12 alkyl chains; less than about 20%, preferably from about 15% to about 19.5% of 2-ethyl decyl by weight of the 2-alkyl branched C12 alkyl chains; less than about 20%, preferably from about 15% to about 19.5% of 2-propyl nonyl by weight of the 2-alkyl branched C12 alkyl chains; and more than about 25%, preferably from about 26% to about 35% of 2-butyl octyl and 2-pentyl heptyl and other 2-alkyl branched C12 alkyl by weight of the 2-alkyl branched C12 alkyl chains.

The distribution of the 2-alkyl branched C12 alkyl sulfate with 2-methyl-1-undecyl sulfate, 2-ethyl-1-decyl sulfate, 2-propyl-1-nonyl sulfate, 2-butyl-1-octyl sulfate, 2-pentyl-1-heptyl sulfate and other 2-alkyl branched C12 alkyl sulfates can be provided either by weight of the 2-alkyl branched C12 alkyl sulfate, or by weight of the C12 alkyl sulfate anionic surfactant, or by weight of the surfactant B.

The C13 alkyl sulfate anionic surfactant of the surfactant B may consist of: d) less than about 60% of a linear C13 alkyl sulfate by weight of the C13 alkyl sulfate anionic surfactant, and e) more than about 40% of a 2-alkyl branched C13 alkyl sulfate by weight of the C13 alkyl sulfate anionic surfactant; and f) less than about 10% of other branched C13 alkyl sulfates by weight of the C13 alkyl sulfate anionic surfactant; wherein (d), (e) and (f) add up to about 100% by weight of the C13 alkyl sulfate anionic surfactant.

**In** that aspect, the 2-alkyl branched C13 alkyl sulfate may comprise more than about 30% of 2-methyl dodecyl sulfate by weight the 2-alkyl branched C13 alkyl sulfate, and about 25% or less of 2-ethyl undecyl sulfate by weight of the 2-alkyl branched C13 alkyl sulfate.

Alternatively, the 2-alkyl branched C13 alkyl sulfate may comprise 2-alkyl branched C13 alkyl chains, and: more than about 30% of 2-methyl dodecyl by weight the 2-alkyl branched C13 alkyl chains, and about 25% or less of 2-ethyl undecyl by weight of the 2-alkyl branched C13 alkyl chains.

By C13 alkyl sulfate anionic surfactant, it is also meant that the alkyl sulfate anionic surfactant comprises an alkyl chain which consists of 13 carbon atoms. Thus, for blends of alkyl sulfate anionic surfactant having an average chain length of 13 carbon atoms, only those alkyl sulfate anionic surfactants which comprise a C13 alkyl chain fall under the definition of C13 alkyl sulfate anionic surfactant.

For blends of alkyl sulfate anionic surfactant comprising a mixture of different chain lengths including a C13 alkyl subfraction, independent of the average alkyl chain length, solely this C13 alkyl subfraction falls under the definition of C13 alkyl sulfate anionic surfactant.

With regards to the specific degree and type of C2-branching, the C13 alkyl sulfate anionic surfactant may consist of: d) less than about 55% of the linear C13 alkyl sulfate by weight of the C13 alkyl sulfate anionic surfactant; e) more than about 45% of the 2-alkyl branched C13 alkyl sulfate by weight of the C13 alkyl sulfate anionic surfactant; and f) less than about 10% of other branched C13 alkyl sulfates by weight of the C13 alkyl sulfate anionic surfactant; wherein (d), (e) and (f) add up to about 100% by weight of the C13 alkyl sulfate anionic surfactant.

Preferably, the C13 alkyl sulfate anionic surfactant may consist of: d) from about 45% to about 54% of the linear C13 alkyl sulfate by weight of the C13 alkyl sulfate anionic surfactant; e) from about 46% to about 55% of the 2-alkyl branched C13 alkyl sulfate by weight of the C13 alkyl sulfate anionic surfactant; and f) from about 0% to about 5% of other branched C13 alkyl sulfates by weight of the C13 alkyl sulfate anionic surfactant; wherein (d), (e) and (f) add up to about 100% by weight of the C13 alkyl sulfate anionic surfactant.

More preferably, the C13 alkyl sulfate anionic surfactant may consist of: d) from about 48% to about 53% of the linear C13 alkyl sulfate by weight of the C13 alkyl sulfate anionic surfactant; e) from about 47% to about 52% of the 2-alkyl branched C13 alkyl sulfate by weight of the C13 alkyl sulfate anionic surfactant; and f) from about 0% to about 5% of other branched C13 alkyl sulfates by weight of the C13 alkyl sulfate anionic surfactant; wherein (d), (e) and (f) add up to about 100% by weight of the C13 alkyl sulfate anionic surfactant.

The above features for the C13 alkyl sulfate anionic surfactant can be exemplified when the surfactant B is the sulfated product obtained from commercially available tradename LIAL®123A alcohol supplied from SASOL.

Alternatively, for instance when the surfactant B is the sulfated product obtained from commercially available tradename ISALCHEM®123A alcohol supplied from SASOL, the C13 alkyl sulfate anionic surfactant may consist of: a) less than about 10%, preferably less than about 9%, more preferably from about 0% to about 8% of the linear C13 alkyl sulfate by weight of the C13 alkyl sulfate anionic surfactant; b) more than about 90%, preferably more than about 91%, more preferably from about 92% to about 100% of the 2-alkyl branched C13 alkyl sulfate by weight of the C13 alkyl sulfate anionic surfactant; and c) from about 0% to about 5% of other branched C13 alkyl sulfates by weight of the C13 alkyl sulfate anionic surfactant; wherein (a), (b) and (c) add up to about 100% by weight of the C13 alkyl sulfate anionic surfactant.

In that aspect, the 2-alkyl branched C13 alkyl sulfate may comprise more than about 30% of 2-methyl dodecyl sulfate by weight the 2-alkyl branched C13 alkyl sulfate, and about 25% or less of 2-ethyl undecyl sulfate by weight of the 2-alkyl branched C13 alkyl sulfate.

In any cases, the 2-alkyl branched C13 alkyl sulfate may comprise: more than about 30.5% of 2-methyl dodecyl sulfate by weight of the 2-alkyl branched C13 alkyl sulfate; less than about 20% of 2-ethyl undecyl sulfate by weight of the 2-alkyl branched C13 alkyl sulfate; less than about 20% of 2-propyl decyl sulfate by weight of the 2-alkyl branched C13 alkyl sulfate; and more than about 25% of 2-butyl nonyl sulfate and 2-pentyl octyl sulfate and other 2-alkyl branched C13 alkyl sulfates by weight of the 2-alkyl branched C13 alkyl sulfate.

Preferably, the 2-alkyl branched C13 alkyl sulfate may comprise: from about 31% to about 40% of 2-methyl dodecyl sulfate by weight of the 2-alkyl branched C13 alkyl sulfate; from about 10% to about 18% of 2-ethyl undecyl sulfate by weight of the 2-alkyl branched C13 alkyl sulfate; from about 10% to about 18% of 2-propyl decyl sulfate by weight of the 2-alkyl branched C13 alkyl sulfate; and from about 26% to about 40% of 2-butyl nonyl sulfate and 2-pentyl octyl sulfate and other 2-alkyl branched C13 alkyl sulfates by weight of the 2-alkyl branched C13 alkyl sulfate.

Alternatively, the 2-alkyl branched C13 alkyl sulfate may comprise: more than about 30.5%, preferably from about 31% to about 40% of 2-methyl dodecyl by weight of the 2-alkyl branched C13 alkyl chains; less than about 20%, preferably from about 10% to about 18% of 2-ethyl undecyl by weight of the 2-alkyl branched C13 alkyl chains; less than about 20%, preferably from about 10% to about 18% of 2-propyl decyl by weight of the 2-alkyl branched C13 alkyl chains; and more than about 25%, preferably from about 26% to about 40% of 2-butyl nonyl and 2-pentyl octyl sand other 2-alkyl branched C13 alkyl by weight of the 2-alkyl branched C13 alkyl chains.

Also, the distribution of the 2-alkyl branched C13 alkyl sulfate with 2-methyl-1-dodecyl sulfate, 2-ethyl-1-undecyl sulfate, 2-propyl-1-decyl sulfate, 2-butyl-1-nonyl sulfate, 2-pentyl-1-octyl sulfate and other 2-alkyl branched C13 alkyl sulfates can be provided either by weight of the 2-branched C13 alkyl sulfate anionic surfactant, or by weight of the C13 alkyl sulfate anionic surfactant or by weight of the surfactant B.

As such, the alkyl chains of the C12 and C13 alkyl sulfate anionic surfactants are highly branched, and have a completely different alkyl branching distribution compared to other sulfates derived from complex branched alcohols such as for example the alcohol sold under the Exxal® 13 supplied from ExxonMobil.

The personal care composition is free of alkoxylated anionic sulfate surfactant.

The surfactant B is free of alkoxylated anionic sulfate surfactant. In other words, the surfactant B is free of alkoxylation.

Thus the surfactant B is free of ethoxylated anionic sulfate surfactant. In other words, the surfactant B is free of ethoxylation.

The C12 alkyl sulfate anionic surfactant is free of alkoxylated anionic sulfate surfactant. In other words, the C12 alkyl sulfate anionic surfactant is free of alkoxylation.

Thus the C12 alkyl sulfate anionic surfactant is free of ethoxylated anionic sulfate surfactant. In other words, the C12 alkyl sulfate anionic surfactant is free of ethoxylation.

The C13 alkyl sulfate anionic surfactant is free of alkoxylated anionic sulfate surfactant. In other words, the C13 alkyl sulfate anionic surfactant is free of alkoxylation.

Thus the C13 alkyl sulfate anionic surfactant is free of ethoxylated anionic sulfate surfactant. In other words, the C13 alkyl sulfate anionic surfactant is free of ethoxylation.

The average degree of alkoxylation is the mol average degree of alkoxylation (*i.e.*, mol average alkoxylation degree) of all the first or C12 or C13 alkyl sulfate anionic surfactant. Hence, when calculating the mol average alkoxylation degree, the moles of first or C12 or C13 non-alkoxylated sulfate anionic surfactant are included:

Mol average alkoxylation degree = (x1 * alkoxylation degree of surfactant 1 + x2 * alkoxylation degree of surfactant 2 + ....) / (x1 + x2 + ....)

wherein x1, x2, ... are the number of moles of each alkyl (or alkoxy) sulfate anionic surfactant of the mixture and alkoxylation degree is the number of alkoxy groups in each alkyl sulfate anionic surfactant.

Suitable alkyl sulfate anionic surfactants can be made using the following process.

Alkyl sulfates are typically prepared by the reaction of fatty alcohols with sulfur trioxide ($SO_3$) or its derivatives or by the reaction of unsaturated compounds with sulfuric acid. Processes using sulfur trioxide in particular have gained prominence for fabricating alkyl sulfate anionic surfactants for use in detergent compositions.

Suitable derivatives of sulfur trioxide include sulfur trioxide complexes such as chlorosulfonic acid, sulfuric acid, or sulfamic acid. Sulfur trioxide is preferred since it tends to result in more pure products. The sulfation reaction typically takes place in a continuous process using a cascade, falling film or tube bundle reactor, with the sulfur trioxide being applied in an equimolar or small excess, usually in a temperature range of 20°C to 60°C, with the reaction temperature being determined at least partially by the solidification point of the fatty alcohol in the reaction. The reaction typically results in the acid form of the resulting alkyl sulfate anionic surfactant, here the surfactant B which is typically neutralized in a subsequent step, using an alkali such as sodium hydroxide, potassium hydroxide, magnesium hydroxide lithium hydroxide, calcium hydroxide, ammonium hydroxide, monoethanolamine, diethanolamine, triethanolamine, monoisopropanolamine, diamines, polyamines, primary amines, secondary amines, tertiary amines, amine containing surfactants, and mixtures thereof.

Also, as already mentioned for Surfactant A, it is well known that the process of sulfating fatty alcohols to yield alkyl sulfate anionic surfactants also yields various impurities. The exact nature of these impurities depends on the conditions of sulfation and neutralization. Generally, however, the impurities of the sulfation process include one or more inorganic salts, unreacted fatty alcohol, and olefins ("The Effect of Reaction By-Products on the Viscosities of Sodium Lauryl Sulfate Solutions," Journal of the American Oil Chemists' Society, Vol. 55, No. 12, p. 909-913 (1978), C.F. Putnik and S.E. McGuire). The level of non-alkyl sulfate impurities in the alkyl sulfate anionic surfactant described herein can be less than about 6% by weight, preferably less than about 4% by weight, and most preferably less than about 2% by weight of the alkyl sulfate anionic surfactant.

Additional anionic surfactant

The personal care composition may comprise an additional anionic surfactant. The additional anionic surfactant is a non-alkoxylated, such as a non-ethoxylated anionic surfactant. The additional anionic surfactant may be different from the surfactant A and/or surfactant B.

The additional anionic surfactant may be different from the surfactant and/or surfactant B and may be selected from the group consisting of ammonium lauryl sulfate, ammonium C10-15 alkyl sulfate, ammonium C11-15 alkyl sulfate, ammonium decyl sulfate, ammonium undecyl sulfate, triethylamine lauryl sulfate, triethanolamine lauryl sulfate, monoethanolamine lauryl sulfate, diethanolamine lauryl sulfate, lauric monoglyceride sodium sulfate, sodium lauryl sulfate, sodium C10-15 alkyl sulfate, sodium C11-15 alkyl sulfate, sodium decyl sulfate, sodium undecyl sulfate, potassium lauryl sulfate, potassium C10-15 alkyl sulfate, potassium C11-15 alkyl sulfate, potassium decyl sulfate, potassium undecyl sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, monoethanolamine cocoyl sulfate, sodium tridecyl benzene sulfonate, sodium dodecyl benzene sulfonate, sodium cocoyl isethionate and combinations thereof.

Preferably, the additional anionic surfactant may be selected from the group consisting of ammonium lauryl sulfate, ammonium undecyl sulfate, triethylamine lauryl sulfate, triethanolamine lauryl sulfate, monoethanolamine lauryl sulfate, diethanolamine lauryl sulfate, sodium lauryl sulfate, sodium undecyl sulfate, potassium lauryl sulfate, potassium undecyl sulfate, sodium lauroyl sarcosinate, sodium cocoyl isethionate and combinations thereof.

Most preferred, the additional anionic surfactant may be selected from the group consisting of ammonium lauryl sulfate, triethylamine lauryl sulfate, triethanolamine lauryl sulfate, monoethanolamine lauryl sulfate, diethanolamine lauryl sulfate, sodium lauryl sulfate, potassium lauryl sulfate and combinations thereof, even most preferred sodium lauryl sulfate.

The personal care composition may comprise from about 0.5% to about 10%, preferably from about 1% to about 8%, more preferably from about 2% to about 6%, most preferably from about 2.5% to about 5.5% of an additional anionic surfactant by weight of the composition.

ZWITTERIONIC SURFACTANT

The cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises an hydroxysultaine.

The hydroxysultaine may be an alkyl hydoxysultaine:

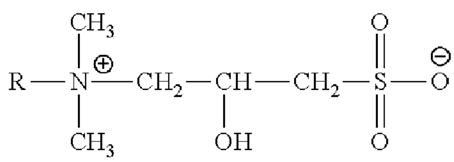

Alkyl hydroxysultaines

where R is alkyl group with $C_{8\ to}\ C_{24}$ carbon chain (saturated or unsaturated) or mixture thereof. Examples include lauryl hydroxysultaine (where R is lauryl; $C_{12}H_{25}$) and coco-hydroxysultaine (where R is coco alkyl).

Alternatively, the hydroxysultaine may be an alkylamidoalkyl hydoxysultaine:

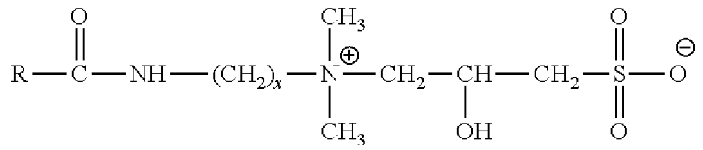

where RCO=$C_6$-$C_{24}$ acyl (saturated or unsaturated) or mixtures thereof. Examples include Cocamidopropyl Hydroxysultaine (RCO=coco acyl, x=3 for the propyl moiety bearing the hydroxy group), Lauramidopropyl Hydroxysultaine (RCO=lauroyl, and x=3), Myristamidopropyl Hydroxysultaine (RCO=myristoyl, and x=3), Oleamidopropyl Hydroxysultaine (RCO=oleoyl, and x=3) and tallowamidopropyl hydroxysultaine (RCO=acyl groups derived from tallow, and x=3).

Alternatively, the R group of the amidoalkyl hydroxysultaine may not be limited to an alkyl group such as erucamidopropyl hydroxysultaine, or methoxycinnamidopropyl hydroxysultaine.

The hydroxysultaine may be selected from the group consisting of lauryl hydroxysultaine, coco-hydroxysultaine, lauramidopropyl hydroxysultaine, cocamidopropyl hydroxysultaine, cocobutyramido hydroxysultaine, capryl/capramidopropyl hydroxysultaine, cetyl/lauryl/myristyl hydroxysultaine, erucamidopropyl hydroxysultaine, methoxycinnamidopropyl hydroxysultaine, myristamidopropyl hydroxysultaine, oleamidopropyl hydroxysultaine, tallowamidopropyl hydroxysultaine and mixtures thereof.

The hydroxysultaine may be preferably selected from the group consisting of coco-hydroxysultaine, lauramidopropyl hydroxysultaine, cocamidopropyl hydroxysultaine, cocobutyramido hydroxysultaine, myristamidopropyl hydroxysultaine, oleamidopropyl hydroxysultaine, tallowamidopropyl hydroxysultaine, and mixtures thereof.

More preferably, the hydroxysultaine may comprise cocamidopropyl hydroxysultaine, cocobutyramido hydroxysultaine, coco-hydroxysultaine and mixtures thereof.

Most preferably, the hydroxysultaine may comprise cocamidopropyl hydroxysultaine.

Hence, the cleansing phase may include from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises cocamidopropyl hydroxysultaine.

When the cleansing phase comprises a surfactant A or surfactant B as recited herein and a betaine, the viscosity profile of the cleansing phase and thus of the resulting personal care composition is improved in terms of an increased lamellar phase volume, an enhanced Young's modulus and an increased zero shear viscosity.

The cleansing phase may further comprise an additional cosurfactant may be, for example, an amphoteric surfactant, a nonionic surfactant, or a combination thereof. Suitable amphoteric or zwitterionic surfactants can include those described in U.S. Patent No. 5,104,646 and U.S. Patent No. 5,106,609.

Additional amphoteric detersive surfactants suitable for use in the cleansing phase can include those surfactants broadly described as derivatives of aliphatic secondary and tertiary amines in which an aliphatic radical can be straight or branched chain and wherein an aliphatic substituent can contain from about 8 to about 18 carbon atoms such that one carbon atom can contain an anionic water solubilizing group, e.g., carboxy, sulfonate, sulfate, phosphate, or phosphonate. Examples of compounds falling within this definition can be N-alkyltaurines such as the one prepared by reacting dodecylamine with sodium isethionate according to the teaching of U.S. Patent No. 2,658,072, N-higher alkyl aspartic acids such as those produced according to the teaching of U.S. Patent No. 2,438,091, and products described in U.S. Patent No. 2,528,3 78. Other examples of amphoteric surfactants can include sodium lauroamphoacetate, sodium cocoamphoacetate, disodium lauroamphoacetate disodium cocodiamphoacetate, and mixtures thereof. Amphoacetates and diamphoacetates can also be used.

Nonionic surfactants suitable for use in the personal care compositions can include those selected from the group consisting of alkyl ethoxylates, alkyl glucosides, polyglucosides (e.g., alkyl polyglucosides, decyl polyglucosides), polyhydroxy fatty acid amides, alkoxylated fatty acid esters, sucrose esters, amine oxides, or mixtures thereof. Some exemplary nonionic surfactants can include cocamide monoethanolamine, decyl glucoside, or a mixture thereof.

The ratio of the weight percent of the surfactant A or surfactant B to the weight percent of the betaine be from about 0.5:1 to about 20:1, preferably from about 0.75:1 to about 9:1, more preferably from about 0.95:1 to about 2:1.

The personal care composition may be substantially free or free of betaine to enhance the mildness properties of the personal care composition.

LAMELLAR STRUCTURE

The cleansing phase of the personal care composition comprises an aqueous structured surfactant phase. The cleansing phase is comprised of a structured domain that comprises the surfactants as set out hereinabove. The structured domain may be preferably an opaque structured domain, which is preferably a lamellar phase. The lamellar phase produces a lamellar gel network. The lamellar phase can provide resistance to shear, adequate yield to suspend particles and droplets and at the same time provides long term stability, since it is thermodynamically stable.

The personal care composition may be a structured lamellar composition. The personal care composition may comprise at least an about 20% lamellar structure or at least an about 40% lamellar structure, preferably at least an about 50% lamellar structure, more preferably at least an about 70% lamellar structure.

Alternatively, the personal care composition may comprise a lamellar phase volume from about 20% to about 100% or about 40% to about 100%, preferably from about 50% to about 100%, more preferably from about 70% to about 100% of a lamellar phase volume according to the Ultracentrifugation Method disclosed herein.

STRUCTURING SYSTEM

The personal care composition includes a structuring system. The structuring system can help to provide structure to the cleansing phase. A structuring system includes an electrolyte. The structuring system further includes a rheology modifier. The structuring system also includes a non-ionic emulsifier.

Electrolyte

The structuring system of the personal care composition comprises an electrolyte. The electrolyte may comprise an anion, wherein the anion is selected from the group consisting of phosphate, chloride, sulfate, citrate, and mixtures thereof; and a cation, wherein the cation is selected from the group consisting of sodium, ammonium, potassium, magnesium, and mixtures thereof.

The electrolyte may be selected from the group consisting of sodium chloride, ammonium chloride, sodium sulfate, ammonium sulfate, and mixtures thereof. Preferably, the electrolyte may comprise sodium chloride.

The personal care composition comprises from about 3% to about 10%, preferably from about 3.5% to about 7.5%, more preferably from about 4% to about 7%, even more preferably from about 4.5% to about 6%, most preferably from about 4.5% to about 5.8%, by weight of said personal care composition, of the electrolyte.

In an aspect, the personal care composition may comprise from about 3% to about 10%, preferably from about 3.5% to about 7.5%, more preferably from about 4% to about 7%, even more preferably from about 4.5% to about 6%, most preferably from about 4.5% to about 5.8%, by weight of said personal care composition, of sodium chloride.

The total level of electrolyte can come from the amount of electrolyte added to the composition and the amount of electrolyte coming from any other ingredient added in the composition such as the hydroxysultaine.

As defined hereinabove, where amount ranges are given, these are to be understood as being the total amount of said ingredient in the composition, or where more than one species fall within the scope of the ingredient definition, the total amount of all ingredients fitting that definition, in the composition. For instance, the total level of electrolyte is provided by the level of added electrolyte and any further level coming from another material like the co-surfactant if applicable.

The level of the electrolyte should not be beyond 10% by weight of the composition. Above 10 wt.% of the electrolyte, corrosion issues may occur on the manufacturing line.

Surprisingly, inventors have found that a lamellar structure is obtained when the cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises an hydroxysultaine and from about 3% to about 10%, preferably from about 3.5% to about 7.5%, more preferably from about 4% to about 7%, even more preferably from about 4.5% to about 6%, most preferably from about 4.5% to about 5.8%, by weight of said personal care composition, of the electrolyte.

Especially but not limited to, a lamellar structure above 20% or 40% or 50% or 70% is obtained when the cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises cocamidopropyl hydroxysultaine and from about 3% to about 10%, preferably from about 3.5% to about 7.5%, more preferably from about 4% to about 7%, even more preferably from about 4.5% to about 6%, most preferably from about 4.5% to about 5.8%, by weight of said personal care composition, of sodium chloride.

Surfactant A

When the cleansing phase comprise the surfactant A without any additional anionic surfactant, inventors have found that a lamellar structure is obtained when the cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises an hydroxysultaine and from about 3% to about 10%, preferably from about 3.5% to about 7.5%, more preferably from about 3.5% to about 7%, even more preferably from about 4% to about 6%, most preferably from about 4.5% to about 5.8%, by weight of said personal care composition, of the electrolyte.

When the cleansing phase comprise the surfactant A without any additional anionic surfactant, a lamellar structure above 20% or 40% or 50% or 70% is obtained when the cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises cocamidopropyl hydroxysultaine and from about 3% to about 10%, preferably from about 3.5% to about 7.5%, more preferably from about 3.5% to about 7%, even more preferably from about 4% to about 6%, most preferably from about 4.5% to about 5.8%, by weight of said personal care composition, of sodium chloride.

When the cleansing phase comprise the surfactant A and an additional anionic surfactant, inventors have found that a lamellar structure is obtained when the cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises an hydroxysultaine and from about 3% to about 10%, preferably from about 3.75% to about 7.5%, more preferably from about 4.5% to about 7%, even more preferably from about 4.75% to about 6%, most preferably from about 4.75% to about 5.8%, by weight of said personal care composition, of the electrolyte.

When the cleansing phase comprise the surfactant A with an additional anionic surfactant, especially sodium lauryl sulfate, a lamellar structure above 20% or 40% or 50% or 70% is obtained when the cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises cocamidopropyl hydroxysultaine and from about 3% to about 10%, preferably from about 3.75% to about 7.5%, more preferably from about 4.5% to about 7%, even more preferably from about 4.75% to about 6%, most preferably from about 4.75% to about 5.8%, by weight of said personal care composition, of sodium chloride.

Surfactant B

When the cleansing phase comprise the surfactant B without any additional anionic surfactant, inventors have found that a lamellar structure is obtained when the cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises an hydroxysultaine and from about 3% to about 10%, preferably from about 3.5% to about 7.5%, more preferably from about 3.5% to about 7%, even more preferably from about 4% to about 6%, most preferably from about 4.5% to about 5.8%, by weight of said personal care composition, of the electrolyte.

When the cleansing phase comprise the surfactant B without any additional anionic surfactant, a lamellar structure above 20% or 40% or 50% or 70% is obtained when the cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises cocamidopropyl hydroxysultaine and from about 3% to about 10%, preferably from about 3.5% to about 7.5%, more preferably from about 3.5% to about 7%, even more preferably from about 4% to about 6%, most preferably from about 4.5% to about 5.8%, by weight of said personal care composition, of sodium chloride.

When the cleansing phase comprise the surfactant B and an additional anionic surfactant, inventors have found that a lamellar structure is obtained when the cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises an hydroxysultaine and from about 3% to about 10%, preferably from about 3.75% to about 7.5%, more preferably from about 4% to about 7%, even more preferably from about 4.5% to about 6%, most preferably from about 4.75% to about 5.8%, by weight of said personal care composition, of the electrolyte.

When the cleansing phase comprise the surfactant B with an additional anionic surfactant, especially sodium lauryl sulfate, a lamellar structure above 20% or 40% or 50% or 70% is obtained when the cleansing phase includes from about 0.1% to about 20%, preferably from about 2% to about 10%, more preferably from about 3% to about 8%, most preferably from about 4% to 7% by weight of the composition, of a zwitterionic surfactant, wherein the zwitterionic surfactant comprises cocamidopropyl hydroxysultaine and from about 3% to about 10%, preferably from about 3.75% to about 7.5%, more preferably from about 4% to about 7%, even more preferably from about 4.5% to about 6%, most preferably from about 4.75% to about 5.8%, by weight of said personal care composition, of sodium chloride.

Rheology modifier

The structuring system of the cleansing phase comprises from about 0.01% to about 5%, preferably from about 0.1% to about 2%, more preferably from about 0.2% to about 1%, most preferably from about 0.5% to about 1% by weight of the personal care composition, of a rheology modifier.

The rheology modifier may be an associative polymer. Associative polymers are polymers constituted by a hydrophilic main chain and hydrophobic side chains. Their behavior in solution is a result of competition between the hydrophobic and hydrophilic properties of their structure. The hydrophobic units tend to form aggregates constituting linkage points between the macromolecular chains. From a rheological viewpoint, associative water-soluble polymers have a very high viscosifying power in water and retain their viscosity well in a saline medium. **In** mixed polymer and surfactant systems, surfactant aggregates can form, which are stabilized by diverse types of interactions: electrostatic interactions, dipolar interactions, or hydrogen bonds. Associative water-soluble polymers can interact more specifically with surfactants due to their hydrophobic portions.

The hydrophilic main chain of these associative polymers can, in particular, result from polymerization of a hydrophilic monomer containing functions onto which hydrophobic chains can subsequently be grafted, for example acid functions. This method of preparing associative polymers is described in particular in the "Water Soluble Polymers", ACS Symposium Series 467, ed. Shalaby W Shalaby et al., Am. Chem. Soc. Washington (1991), pp. 82-200. However, a water-soluble polymer of natural origin, or a natural polymer rendered water-soluble by chemical modification, can also be used. Associative polymers can also be formed by copolymerization of hydrophilic monomers and hydrophobic monomers. These hydrophobic polymers, introduced into the reaction medium in a much smaller quantity than the hydrophilic polymers, generally comprise a fatty hydrocarbon chain. This method of preparation is described in the publication by S. Biggs et. al., J. Phys Chem. (1992, 96. pp 1505-11).

The rheology modifier may be selected from the group consisting of a polyacrylate, a polysaccharide, a modified polyol, an hydrophobically modified polyacrylate, an hydrophobically modified polysaccharide, and mixtures thereof.

The rheology modifier may be preferably selected from the group consisting of a polyacrylate, a polysaccharide such as xanthan gum, a modified polyol, an hydrophobically modified polyacrylate, an hydrophobically modified polysaccharide such as hydroxypropyl starch phosphate, and mixtures thereof.

The rheology modifier may be selected from the group consisting of sodium polyacrylate, acrylates copolymer, Acrylates/Vinyl Isodecanoate Crosspolymer, Acrylates/C10-30 Alkyl Acrylate Crosspolymer, Acrylates/C10-30 alkyl acrylate crosspolymer comprising stearyl side chains with less than about 1% Hydrophobic modification, acrylates/C10-30 alkyl acrylate crosspolymer including octyl side chains with less than about 5% Hydrophobic modification, Ammonium Acryloyldimethyltaurate/Beheneth-25 Methacrylate Crosspolymer, Acrylates/Beheneth-25 Methacrylate Copolymer, Acrylates/Steareth-20 Methacrylate Copolymer, and Acrylates/Steareth-20 Methacrylate Crosspolymer, PEG-150/Decyl Alcohol/SMDI Copolymer, PEG-150/stearyl alcohol/SMDI copolymer, hydroxypropyl starch phosphate, distarch phosphate, sodium carboxymethyl starch, starch, Tapioca starch, xanthan gum, gellan gum, carboxymethyl cellulose, carboxymethyl hydroxyethyl cellulose, hydroxypropyl methyl cellulose, guar gum, hydroxypropyl guar, sodium alginate, and mixtures thereof.

Non-limiting examples of associative polymers being a polyacrylate or an hydrophobically modified polyacrylate include sodium polyacrylate, acrylates copolymer, Acrylates/Vinyl Isodecanoate Crosspolymer (Stabylen 30 from 3V), Acrylates/C10-30 Alkyl Acrylate Crosspolymer (Pemulen TR1 and TR2), Aqupec SER-300 made by Sumitomo Seika of Japan, which is Acrylates/C10-30 alkyl acrylate crosspolymer comprising stearyl side chains with less than about 1% HM, Ammonium Acryloyldimethyltaurate/Beheneth-25 Methacrylate Crosspolymer (Aristoflex HMB from Clariant), Acrylates/Beheneth-25 Methacrylate Copolymer (Aculyn 28 from Rohm and Haas); Acrylates/Steareth-20 Methacrylate Copolymer (Aculyn 22 from Rohm and Haas), and Acrylates/Steareth-20 Methacrylate Crosspolymer (Aculyn 88 from Rohm and Haas).

Acrylate copolymers are defined as polymers of two or more monomers consisting of acrylic acid, methacrylic acid (q.v.) or one of their simple esters. Simple esters of methacrylic acid are made with simple alkyl groups such as methyl, ethyl, propyl and butyl and their respective regioisomers. An example of acrylate copolymers may be Luvimer 100 from BASF which is made of a terpolymer of tert-butyl acrylate, ethyl acrylate and methacrylic acid.

Non-limiting examples of associative polymers being a modified polyol include PEG-150/Decyl Alcohol/SMDI Copolymer (Aculyn 44 from Dow Chemical Company), and PEG-150/stearyl alcohol/SMDI copolymer (Aculyn 46 from Dow Chemical Company).

"SMDI" as used herein means saturated methylene diphenyl diisocyanate. "PEG-150/decyl alcohol/SMDI copolymer" is a copolymer of PEG-150 (q.v.), Decyl Alcohol (q.v.), and Saturated Methylene Diphenyl Diisocyanate (q.v.) (SMDI) monomers. "PEG-150/stearyl alcohol/SMDI copolymer" is a copolymer of PEG-150 (q.v.), Saturated Methylene Diphenyl Diisocyanate (q.v.) (SMDI), and Stearyl Alcohol (q.v.) monomers.

Preferably, the rheology modifier may comprise acrylates/C10-30 alkyl acrylate crosspolymer. Acrylates/C10-30 alkyl acrylate Crosspolymer is a copolymer of C10-30 alkyl acrylate and one or more monomers of acrylic acid, methacrylic acid or one of their simple esters crosslinked with an allyl ether of sucrose or an allyl ether of pentaerythritol.

An exemplary preferred acrylates/C10-30 alkyl acrylate crosspolymer may be Aqupec SER-300 made by Sumitomo Seika of Japan, which is Acrylates/C10-30 alkyl acrylate crosspolymer comprising stearyl side chains with less than about 1% Hydrophobic modification (HM). Other preferred rheology modifiers in that category may comprise stearyl, octyl, decyl and lauryl side chains.

Preferred acrylates/C10-30 alkyl acrylate crosspolymer may be Aqupec SER-150 that is acrylates/C10-30 alkyl acrylates crosspolymer comprising about C18 (stearyl) side chains and about 0.4% HM, and Aqupec HV-701EDR that is acrylates/C10-30 alkyl acrylates crosspolymer which comprises about C8 (octyl) side chains and about 3.5% HM.

The crosslinked rheology modifier may include a percentage hydrophobic modification, which is the mole percentage of monomers expressed as a percentage of the total number of all monomers in the polymer backbone, including both acidic and other non-acidic monomers. The percentage hydrophobic modification of the polymer, hereafter %HM, can be determined by the ratio of monomers added during synthesis, or by analytical techniques such as proton nuclear magnetic resonance (NMR). The alkyl side chain length can be determined similarly.

The structuring system of the cleansing phase comprises from about 0.01% to about 5%, preferably from about 0.01% to about 1%, more preferably from about 0.02% to about 0.1%, most preferably from about 0.03% to about 0.05% by weight of the personal care composition, of acrylates/C10-30 alkyl acrylate crosspolymer.

Non-limiting example of an associative polymer being a polysaccharide or a modified polysaccharide includes starch, Tapioca starch, xanthan gum, gellan gum, carboxymethyl cellulose, carboxymethyl hydroxyethyl cellulose, hydroxypropyl methyl cellulose, guar gum, hydroxypropyl guar, sodium alginate, and mixtures thereof.

The rheology modifier may comprise xanthan gum. Xanthan gum can help to improve the stability of the personal care composition.

The structuring system of the cleansing phase comprises from about 0.01% to about 5%, preferably from about 0.1% to about 2%, more preferably from about 0.2% to about 1%, most preferably from about 0.5% to about 1% by weight of the personal care composition, of xanthan gum.

Alternatively, the rheology modifier may comprise an hydrophobically modified polysaccharide, especially a modified starch. The modified starch may be selected from the group consisting of hydroxypropyl starch phosphate, distarch phosphate, sodium carboxymethyl starch, and mixtures thereof.

In particular, the modified starch may comprise hydroxypropyl starch phosphate. Hydroxypropyl starch phosphate may be provided as Structure® XL from Nouryon, or C*HiForm™A12747 from Cargill. Distarch phosphate may be provided as Agenajel 20.306 from Agrana Stärke. Sodium carboxymethyl starch may be provided as Vivastar® CS Instant Powder from J. Rettenmaier & Söhne.

The structuring system of the cleansing phase comprises from about 0.01% to about 5%, preferably from about 0.1% to about 2%, more preferably from about 0.2% to about 1%, most preferably from about 0.5% to about 1%, even most preferably from about 0.75% to about 0.95% by weight of the personal care composition, of hydroxypropyl starch phosphate.

Starch is a carbohydrate polymer consisting of a large number of glucose units linked together primarily by alpha 1-4 glucosidic bonds. The starch polymers come in two forms: linear (amylose) and branched through alpha 1-6 glucosidic bonds (amylopectin), with each glucose unit possessing a maximum of three hydroxyls that can undergo chemical substitution.

Hydroxypropyl starch phosphate is a modified starch. It is obtained in accordance with good manufacturing practice by esterification of food starch with sodium trimetaphosphate or phosphorus oxychloride combined with etherification by propylene oxide. Hydroxypropylation results in substitution of hydroxyl groups with 2-hydroxypropyl ether. In cases of cross-linking, where phosphorus oxychloride, connects two chains, the structure can be represented by: Starch-O-R-O-Starch, where R = cross-linking group and Starch refers to the linear and/or branched structure.

Hydroxypropyl starch phosphate is a naturally-derived and biodegradable rheology modifier that enables more stable and natural emulsions for the personal care application over other rheology modifiers in particular Acrylate/C10-C30 alkyl acrylate crosspolymer. When the rheology modifier is a modified starch, lather has been improved.

Such rheology modifiers or associative polymers can help to provide significant enhancement of structure to the cleansing phase and thus the personal care composition, especially when the personal care composition comprises reduced levels of surfactant; and provide said structure at relatively low levels of rheology modifiers. Also, lather can be further improved.

Not all crosslinked, associative polymers are effective, and many are deleterious to structure. Associative polymers having hydrophobic side chains with fewer than 7 carbons and having %HM greater than about 25% or about 50% are not preferred.

Non-ionic emulsifier

The personal care composition comprises a non-ionic emulsifier. Preferably the nonionic emulsifier has an HLB from about 3.4 to 13.0, preferably 3.4 to about 8.0.

The personal care composition may comprise a non-ionic emulsifier at concentrations ranging from about 0.1% to about 10%, more preferably from about 0.25% to about 8%, even more preferably from about 0.5% to about 5%, most preferably from about 1.5% to about 2.5%, by weight of the composition.

The balance between the hydrophilic and lipophilic moieties in a surfactant molecule is used as a method of

classification (hydrophile-lipophile balance, HLB). The HLB values for commonly-used surfactants are readily available in the literature (e.g., HLB Index in McCutcheon's Emulsifiers and Detergents, MC Publishing Co., 2004). Another way of obtaining HLB values is to estimate by calculations. The HLB system was originally devised by Griffin (J. Soc. Cosmetic Chem., 1, 311, 1949). Griffin defined the HLB value of a surfactant as the mol% of the hydrophilic groups divided by 5, where a completely hydrophilic molecule (with no nonpolar groups) had an HLB value of 20. Other examples of how to calculate HLB values are described by Davies in Interfacial Phenomena, 2nd Edition, Academic Press, London, 1963 and by Lin in J. Phys. Chem. 76, 2019-2013, 1972.

The non-ionic emulsifiers for use herein may be selected from the group consisting of glyceryl monohydroxystearate, glyceryl caprylate/caprate, isosteareth-2, trideceth-2, trideceth-3, hydroxystearic acid, propylene glycol stearate, PEG-2 stearate, sorbitan monostearate, glyceryl laurate, laureth-2, cocamide monoethanolamine, lauramide monoethanolamine, and mixtures thereof.

The personal care composition may comprise a non-ionic emulsifier at concentrations ranging from about 0.1% to about 10%, more preferably from about 0.25% to about 8%, even more preferably from about 0.5% to about 5%, most preferably from about 1.5% to about 2.5%, by weight of the composition, wherein the non-ionic emulsifier is selected from the group consisting of glyceryl monohydroxystearate, glyceryl caprylate/caprate, isosteareth-2, trideceth-2, trideceth-3, hydroxystearic acid, propylene glycol stearate, PEG-2 stearate, sorbitan monostearate, glyceryl laurate, laureth-2, cocamide monoethanolamine, lauramide monoethanolamine, and mixtures thereof.

Preferably, the non-ionic emulsifiers for use herein may comprise at least one of the following: trideceth-2 and trideceth-3. The personal care composition may comprise trideceth-2 or trideceth-3 at concentrations ranging from about 0.1% to about 10%, more preferably from about 0.25% to about 8%, even more preferably from about 0.5% to about 5%, most preferably from about 1.5% to about 2.5%, by weight of the composition.

Alternatively, the non-ionic emulsifiers for use herein may comprise at least one of the following: glyceryl monohydroxystearate and glyceryl caprylate/caprate. The personal care composition may comprise glyceryl monohydroxystearate or glyceryl caprylate/caprate at concentrations ranging from about 0.1% to about 10%, more preferably from about 0.25% to about 8%, even more preferably from about 0.5% to about 5%, most preferably from about 1.5% to about 2.5%, by weight of the composition.

The non-ionic emulsifier can help to increase the Young's modulus and thus improve the structure and stability of the personal care composition.

Deposition polymer

The personal care composition may additionally comprise a cationic deposition polymer in the cleansing phase as a deposition aid for the benefit agents described herein.

Suitable cationic deposition polymers for use in the compositions may contain cationic nitrogen-containing moieties such as quaternary ammonium moieties. Nonlimiting examples of cationic deposition polymers for use in the personal care composition include cationic cellulose derivatives. Preferred cationic cellulose polymers are the salts of hydroxyethyl cellulose reacted with trimethyl ammonium substituted epoxide, referred to in the industry (CTFA) as Polyquaternium 10 which are available from Amerchol Corp. (Edison, N.J., USA) in their Polymer KG, JR and LR series of polymers with the most preferred being KG-30M. Other suitable cationic deposition polymers include cationic guar gum derivatives, such as guar hydroxypropyltrimonium chloride, specific examples of which include the Jaguar series (preferably Jaguar C-17) commercially available from Rhodia Inc., and N-Hance polymer series commercially available from Aqualon.

The cationic deposition polymers of the personal care composition may have a cationic charge density from about 0.8 meq/g to about 2.0 meq/g, alternatively from about 1.0 meq/g to about 1.5 meq/g.

The personal care composition may comprise from 0.01% to 5%, preferably from 0.1% to 2%, more preferably from 0.2% to 1%, most preferably from 0.3% to 1% by weight of the personal care composition, of a cationic deposition polymer.

In an aspect, the personal care composition may comprise from 0.01% to 5%, preferably from 0.1% to 2%, more preferably from 0.2% to 1%, most preferably from 0.3% to 1% by weight of the personal care composition, of guar hydroxypropyltrimonium chloride.

Water

The cleansing phase of the personal care composition may comprise water. The cleansing phase of the personal care composition may comprise from about 10% to about 90%, alternatively from about 40% to about 85%, alternatively from about 60% to about 80% by weight water.

## BENEFIT PHASE

The personal care composition comprises a benefit phase. The benefit phase in the personal care composition may be hydrophobic or essentially anhydrous and may be substantially free of water. The benefit phase may be substantially free or free of surfactant.

The benefit phase typically comprise a benefit agent. A benefit agent may include water insoluble or hydrophobic benefit agent. The benefit phase may comprise from about 0.1% to about 50%, preferably from about 1% to about 30%, more preferably from about 4.5% to about 10%, by weight of the personal care composition, of a benefit agent.

The hydrophobic skin benefit agent for use in the benefit phase of the composition may have a Vaughan Solubility Parameter (VSP) of from about 5 to about 15, preferably from about 5 to less than 10. These solubility parameters are well known in the formulation arts, and are defined by Vaughan in Cosmetics and Toiletries, Vol. 103, p47-69, Oct. 1988.

The benefit agent may be selected from the group consisting of petrolatum; lanolin; derivatives of lanolin; natural waxes; synthetic waxes; volatile organosiloxanes; derivatives of volatile organosiloxanes; non-volatile organosiloxanes; derivatives of non-volatile organosiloxanes; lanolin oil; lanolin esters; natural triglycerides; synthetic triglycerides; and mixtures thereof.

Preferably, the benefit agent may comprise petrolatum. The benefit phase may comprise from about 0.1% to about 50%, preferably from about 1% to about 30%, more preferably from about 4.5% to about 10%, by weight of the personal care composition, of petrolatum.

Alternatively, non-limiting examples glycerides suitable for use as hydrophobic skin benefit agents herein include castor oil, soybean oil, derivatized soybean oils such as maleated soybean oil, safflower oil, cotton seed oil, corn oil, walnut oil, peanut oil, olive oil, cod liver oil, almond oil, avocado oil, palm oil and sesame oil, vegetable oils, sunflower seed oil, and vegetable oil derivatives; coconut oil and derivatized coconut oil, cottonseed oil and derivatized cottonseed oil, jojoba oil, cocoa butter, shea butter, and mixtures thereof.

Preferably, the benefit agent may comprise a glyceride, wherein the glyceride is selected from the group consisting of castor oil, soybean oil, derivatized soybean oils such as maleated soybean oil, safflower oil, cotton seed oil, corn oil, walnut oil, peanut oil, olive oil, cod liver oil, almond oil, avocado oil, palm oil and sesame oil, vegetable oils, sunflower seed oil, and vegetable oil derivatives; coconut oil and derivatized coconut oil, cottonseed oil and derivatized cottonseed oil, jojoba oil, cocoa butter, shea butter, and mixtures thereof.

The benefit phase may comprise from about 0.1% to about 50%, preferably from about 1% to about 30%, more preferably from about 4.5% to about 10%, by weight of the personal care composition, of a glyceride, wherein the glyceride is selected from the group consisting of castor oil, soybean oil, derivatized soybean oils such as maleated soybean oil, safflower oil, cotton seed oil, corn oil, walnut oil, peanut oil, olive oil, cod liver oil, almond oil, avocado oil, palm oil and sesame oil, vegetable oils, sunflower seed oil, and vegetable oil derivatives; coconut oil and derivatized coconut oil, cottonseed oil and derivatized cottonseed oil, jojoba oil, cocoa butter, shea butter, and mixtures thereof.

Preferably, the benefit phase may comprise from about 0.1% to about 50%, preferably from about 1% to about 30%, more preferably from about 4.5% to about 10%, by weight of the personal care composition, of a glyceride, wherein the glyceride is selected from the group consisting of castor oil, soybean oil, maleated soybean oil, safflower oil, cotton seed oil, almond oil, sunflower seed oil, coconut oil, jojoba oil, cocoa butter, shea butter, and mixtures thereof.

More preferably, the benefit phase may comprise from about 0.1% to about 50%, preferably from about 1% to about 30%, more preferably from about 4.5% to about 10%, by weight of the personal care composition, of a glyceride, wherein the glyceride is selected from the group consisting of castor oil, soybean oil, maleated soybean oil, sunflower seed oil, coconut oil, jojoba oil, cocoa butter, shea butter, and mixtures thereof.

Most preferably, the benefit phase may comprise from about 0.1% to about 50%, preferably from about 1% to about 30%, more preferably from about 4.5% to about 10%, by weight of the personal care composition, of a glyceride, wherein the glyceride comprises soybean oil, maleated soybean oil, and mixtures thereof.

Non-limiting examples of acetoglyceride esters suitable for use as hydrophobic skin benefit agents herein include acetylated monoglycerides.

Non-limiting examples of alkyl esters suitable for use as hydrophobic skin benefit agents herein include isopropyl esters of fatty acids and long chain esters of long chain (i.e. C10-C24) fatty acids, e.g. cetyl ricinoleate, non-limiting examples of which include isopropyl palmitate, isopropyl myristate, cetyl riconoleate and stearyl riconoleate. Other examples are: hexyl laurate, isohexyl laurate, myristyl myristate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and mixtures thereof.

Non-limiting examples of alkenyl esters suitable for use as hydrophobic skin benefit agents herein include oleyl myristate, oleyl stearate, oleyl oleate, and mixtures thereof.

Non-limiting examples of polyglycerin fatty acid esters suitable for use as hydrophobic skin benefit agents herein include decaglyceryl distearate, decaglyceryl diisostearate, decaglyceryl monomyristate, decaglyceryl monolaurate, hexaglyceryl monooleate, glycerol monooleate glycerol monooleate and mixtures thereof.

Non-limiting examples of lanolin and lanolin derivatives suitable for use as hydrophobic skin benefit agents herein include lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol riconoleate, and mixtures thereof.

Non-limiting examples of silicone oils suitable for use as hydrophobic skin benefit agents herein include dimethicone copolyol, dimethylpolysiloxane, diethylpolysiloxane, mixed C1-C30 alkyl polysiloxanes, phenyl dimethicone, dimethiconol, and mixtures thereof. Preferred are non-volatile silicones selected from dimethicone, dimethiconol, mixed C1-C30 alkyl polysiloxane, and mixtures thereof. Nonlimiting examples of silicone oils useful herein are described in U.S. Patent No. 5,011,681 (Ciotti et a .).

Still other suitable hydrophobic skin benefit agents include milk triglycerides (e.g., hydroxylated milk glyceride) and polyol fatty acid polyesters.

Still other suitable hydrophobic skin benefit agents include wax esters, non-limiting examples of which include beeswax and beeswax derivatives, spermaceti, myristyl myristate, stearyl stearate, and mixtures thereof. Also useful are vegetable waxes such as carnauba and candelilla waxes; sterols such as cholesterol, cholesterol fatty acid esters; and phospholipids such as lecithin and derivatives, sphingo lipids, ceramides, glycosphingo lipids, and mixtures thereof. Also suitable benefit agents include glycerol monooleate.

Preferably, the benefit phase may comprise a hydrophobic benefit agent and a lipid bilayer structurant. The lipid bilayer structurant comprises glyceryl monooleate, glyceryl monostearate, glyceryl monolaurate, or a mixture thereof. The benefit agent comprises petrolatum, soybean oil, sucrose polyester, mineral oil, or a mixture thereof.

In that aspect, the benefit phase may comprise from about 0.1% to about 50%, preferably from about 1% to about 30%, more preferably from about 4.5% to about 10%, by weight of the personal care composition, of a hydrophobic benefit agent, wherein the hydrophobic benefit agent is selected from the group consisting of petrolatum, soybean oil, sucrose polyester, mineral oil, or a mixture thereof; and from about 0.01% to about 20%, preferably from about 0.02% to about 10%, more preferably from about 0.02% to about 1%, most preferably from about 0.05% to about 0.5% by weight of the personal care composition, of a lipid bilayer structurant, wherein the lipid bilayer structurant is selected from the group consisting of glyceryl monooleate, glyceryl monostearate, glyceryl monolaurate, or a mixture thereof.

Preferably, the benefit phase may comprise from about 0.1% to about 50%, preferably from about 1% to about 30%, more preferably from about 4.5% to about 10%, by weight of the personal care composition, of a hydrophobic benefit agent, wherein the hydrophobic benefit agent comprises petrolatum or soybean oil; and from about 0.01% to about 20%, preferably from about 0.02% to about 10%, more preferably from about 0.02% to about 1%, most preferably from about 0.05% to about 0.5% by weight of the personal care composition, of a lipid bilayer structurant, wherein the lipid bilayer structurant comprises glyceryl monooleate, or glyceryl monostearate or glyceryl monolaurate.

<u>Physical contact between the cleansing and benefit phases</u>

In the personal care composition, the cleansing phase and the benefit phase may be in physical contact. The phases may be blended or mixed to a significant degree, but still be physically distinct such that the physical distinctiveness is undetectable to the naked eye.

The phases can also be made to occupy separate and distinct physical spaces inside a package in which the phases can be stored. In such an arrangement, the structured cleansing phase and the benefit phase can be stored such that the phases are not in direct contact with one another.

Alternatively, the personal care composition may be a multiphase personal care composition where the phases of the personal care composition are made to occupy separate but distinct physical spaces inside the package in which they are stored, but are in direct contact with one another (i.e., they are not separated by a barrier and they are not emulsified or mixed to any significant degree).

The cleaning phase and the benefit phase can be in physical contact while remaining visibly distinct to give, for example, a striped or marbled or geometric configuration.

<u>Optional Ingredients</u>

While not essential for the purposes of the present disclosure, the non-limiting list of materials, in addition to the previously disclosed, optional materials, illustrated hereinafter are suitable for use in the personal care composition, and may be desirably incorporated in certain embodiments, for example to assist or enhance cleansing performance, for treatment of the skin, or to modify the aesthetics of the personal care composition as is the case with perfumes, colorants, dyes or the like. Optional materials useful in the products herein are categorized or described by their cosmetic and/or therapeutic benefit or their postulated mode of action or function. However, it is to be understood that the active and other materials useful herein can, in some instances, provide more than one cosmetic and/or therapeutic benefit or function or operate via more than one mode of action. Therefore, classifications herein are made for the sake of convenience and are not intended to limit an ingredient to the particularly stated application or applications listed. The precise nature of these

optional materials, and levels of incorporation thereof, will depend on the physical form of the composition and the nature of the cleansing operation for which it is to be used. The optional materials are usually formulated at less than about less than about 6%, less than about 5%, less than about 4%, less than about 3%, less than about 2%, less than about 1%, less than about 0.5%, less than about 0.25%, less than about 0.1%, less than about 0.01%, less than about 0.005% of the personal care composition.

The phases of the personal care compositions, preferably the cleansing phase, optionally can further comprise a liquid crystalline phase inducing structurant, which when present is at concentrations ranging from about 0.3% to about 15%, by weight of the phase. Suitable liquid crystalline phase inducing structurants include trihydroxystearin (available from Rheox, Inc. under the trade name THIXCIN® R). The personal care composition is free of fatty acid due to its negative impact on lather performance.

Other non-limiting optional ingredients that can be used in the personal care composition may comprise an optional benefit component that is selected from the group consisting of thickening agents; preservatives; antimicrobials; fragrances; chelators (e.g. such as those described in U.S. Pat. No. 5,487,884 issued to Bisset, et al.); sequestrants; vitamins (e.g. Retinol); vitamin derivatives (e.g. tocophenyl actetate, niacinamide, panthenol); sunscreens; desquamation actives (e.g. such as those described in U.S. Pat. No. 5,681,852 and 5,652,228 issued to Bisset,); anti-wrinkle/ anti-atrophy actives (e.g. N-acetyl derivatives, thiols, hydroxyl acids, phenol); antioxidants (e.g. ascorbic acid derivatives, tocophenol) skin soothing agents/skin healing agents (e.g. panthenoic acid derivatives, aloe vera, allantoin); skin lightening agents (e.g. kojic acid, arbutin, ascorbic acid derivatives) skin tanning agents (e.g. dihydroxyacetone); anti-acne medicaments; essential oils; sensates; pigments; colorants; pearlescent agents; interference pigments (e.g. such as those disclosed in U.S. Pat. No. 6,395,691 issued to Liang Sheng Tsaur, U.S. Pat. No. 6,645,511 issued to Aronson, et al., U.S. Pat. No. 6,759,376 issued to Zhang, et al, U.S. Pat. No. 6,780,826 issued to Zhang, et al.) particles (e.g. talc, kaolin, mica, smectite clay, cellulose powder, polysiloxane, silicas, carbonates, titanium dioxide, polyethylene beads) hydrophobically modified non-platelet particles (e.g. hydrophobically modified titanium dioxide and other materials described in a commonly owned, patent application published on Aug. 17, 2006 under Publication No. 2006/0182699A, entitled "Personal Care Compositions Containing Hydrophobically Modified Non-platelet particle filed on Feb. 15, 2005 by Taylor, et al.) and mixtures thereof.

The personal care composition may comprise from about 0.1% to about 4%, by weight of the personal care composition, of hydrophobically modified titanium dioxide.

The personal care composition may comprise from about 0.1% to about 10%, by weight of the personal care composition, of citric acid, for adjusting the pH from 5.0 to 6.0. Alternatively, the personal care composition may comprise from about 4.5% to about 6%, by weight of the personal care composition, of citric acid, for adjusting the pH from 5.5 to 5.8.

One or more of the phases of the personal care composition can comprise a variety of additional optional ingredients such as shiny particles, beads, exfoliating beads. Such optional ingredients are most typically those materials approved for use in cosmetics and that are described in reference books such as the CTFA Cosmetic Ingredient Handbook, Second Edition, The Cosmetic, Toiletries, and Fragrance Association, Inc. 1988, 1992.

## METHODS OF USE

The personal care compositions may be preferably applied topically to the desired area of the skin or hair in an amount sufficient to provide effective delivery of the skin cleansing agent, hydrophobic material, and particles to the applied surface. The compositions can be applied directly to the skin or indirectly via the use of a cleansing puff, washcloth, sponge or other implement. The compositions may be preferably diluted with water prior to, during, or after topical application, and then subsequently the skin or hair rinsed or wiped off, preferably rinsed off of the applied surface using water or a water-insoluble substrate in combination with water. The present disclosure is therefore also directed to methods of cleansing the skin through the above-described application of the compositions.

A method of providing a stable personal care composition comprises the step of forming a personal care composition as set out hereinbefore with a surfactant A, wherein the surfactant A comprises a C13 alkyl sulfate anionic surfactant, wherein the C13 alkyl sulfate anionic surfactant consists of: a) less than about 40% by weight of the C13 alkyl sulfate anionic surfactant of a linear C13 alkyl sulfate, and b) more than about 60% by weight of the C13 alkyl sulfate anionic surfactant of a 2-branched C13 alkyl sulfate anionic surfactant, wherein the 2-branched C13 alkyl sulfate anionic surfactant comprises: about 25% or less by weight of the 2-branched C13 alkyl sulfate anionic surfactant of 2-pentyl octyl sulfate anionic surfactant, and more than about 25% by weight the 2-branched C13 alkyl sulfate anionic surfactant of 2-methyl dodecyl sulfate anionic surfactant; and c) less than about 5% by weight of the C13 alkyl sulfate anionic surfactant of other branched C13 alkyl sulfate anionic surfactant, wherein a, b and c add up to about 100% by weight of the C13 alkyl sulfate anionic surfactant; alternatively with a surfactant B, wherein the surfactant B comprises a mixture of C12 and C13 alkyl sulfate anionic surfactants, optionally wherein the surfactant B consists essentially of a mixture of C12 and C13 alkyl sulfate anionic surfactants, wherein the mixture of the C12 and C13 alkyl sulfate anionic surfactants comprises a mixture of surfactant isomers of Formula I and surfactant isomers of Formula II:

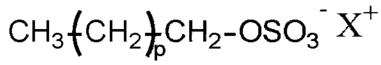

(I)

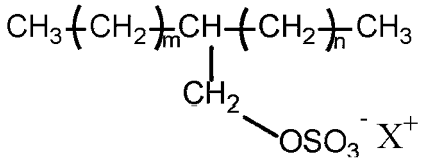

(II)

wherein $10 \leq p \leq 11$;

wherein $8 \leq m + n \leq 9$ and $0 \leq m, n \leq 9$; and

wherein X is a counter cation selected from the group consisting of lithium, sodium, potassium and ammonium, preferably sodium; wherein the surfactant B comprises: from about 37% to about 50%, preferably from about 38% to about 49%, more preferably from about 40% to about 49% of the C12 alkyl sulfate anionic surfactant by weight of the surfactant B, wherein the C12 alkyl sulfate anionic surfactant comprises a 2-alkyl branched C12 alkyl sulfate; from about 50% to about 63%, preferably from about 51% to about 62%, more preferably from about 51% to about 60% of the C13 alkyl sulfate anionic surfactant by weight of the surfactant B, wherein the C13 alkyl sulfate anionic surfactant comprises a 2-alkyl branched C13 alkyl sulfate; and less or equal than about 5% of other alkyl sulfate anionic surfactants by weight of the surfactant B, preferably less or equal than about 4% of other alkyl sulfate anionic surfactants by weight of the surfactant B, wherein other alkyl sulfate anionic surfactants are not C12 or C13 alkyl sulfate anionic surfactant.

A method of enhancing deposition of a benefit agent of a personal care composition is provided and comprises, in that order, the step of forming a personal care composition as set out hereinbefore with a surfactant A, wherein the surfactant A comprises a C13 alkyl sulfate anionic surfactant, wherein the C13 alkyl sulfate anionic surfactant consists of: a) less than about 40% by weight of the C13 alkyl sulfate anionic surfactant of a linear C13 alkyl sulfate, and b) more than about 60% by weight of the C13 alkyl sulfate anionic surfactant of a 2-branched C13 alkyl sulfate anionic surfactant, wherein the 2-branched C13 alkyl sulfate anionic surfactant comprises: about 25% or less by weight of the 2-branched C13 alkyl sulfate anionic surfactant of 2-pentyl octyl sulfate anionic surfactant, and more than about 25% by weight the 2-branched C13 alkyl sulfate anionic surfactant of 2-methyl dodecyl sulfate anionic surfactant; and c) less than about 5% by weight of the C13 alkyl sulfate anionic surfactant of other branched C13 alkyl sulfate anionic surfactant, wherein a, b and c add up to about 100% by weight of the C13 alkyl sulfate anionic surfactant; alternatively with a surfactant B, wherein the surfactant B comprises a mixture of C12 and C13 alkyl sulfate anionic surfactants, optionally wherein the surfactant B consists essentially of a mixture of C12 and C13 alkyl sulfate anionic surfactants; wherein the mixture of the C12 and C13 alkyl sulfate anionic surfactants comprises a mixture of surfactant isomers of Formula I and surfactant isomers of Formula II:

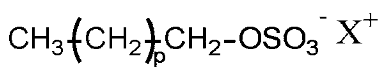

(I)

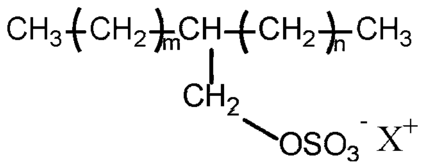

(II)

wherein $10 \leq p \leq 11$;

wherein $8 \leq m + n \leq 9$ and $0 \leq m, n \leq 9$; and

wherein X is a counter cation selected from the group consisting of lithium, sodium, potassium and ammonium, preferably sodium; wherein the surfactant B comprises: from about 37% to about 50%, preferably from about 38% to about 49%, more preferably from about 40% to about 49% of the C12 alkyl sulfate anionic surfactant by weight of the surfactant B, wherein the C12 alkyl sulfate anionic surfactant comprises a 2-alkyl branched C12 alkyl sulfate; from about 50% to about 63%, preferably from about 51% to about 62%, more preferably from about 51% to about 60% of the C13 alkyl sulfate anionic surfactant by weight of the surfactant B, wherein the C13 alkyl sulfate anionic surfactant comprises a 2-alkyl branched C13 alkyl sulfate; and less or equal than about 5% of other alkyl sulfate anionic surfactants by weight of the surfactant B, preferably less or equal than about 4% of other alkyl sulfate anionic

surfactants by weight of the surfactant B, wherein other alkyl sulfate anionic surfactants are not C12 or C13 alkyl sulfate anionic surfactant; and the step of placing the personal care composition onto the skin and/or hair of a consumer.

A method of enhancing hydration or perfume scent of the skin and/or hair of a consumer is provided and comprises, in that order, the step of forming a personal care composition as set out hereinbefore with a surfactant A, wherein the surfactant A comprises a C13 alkyl sulfate anionic surfactant, wherein the C13 alkyl sulfate anionic surfactant consists of: a) less than about 40% by weight of the C13 alkyl sulfate anionic surfactant of a linear C13 alkyl sulfate, and b) more than about 60% by weight of the C13 alkyl sulfate anionic surfactant of a 2-branched C13 alkyl sulfate anionic surfactant, wherein the 2-branched C13 alkyl sulfate anionic surfactant comprises: about 25% or less by weight of the 2-branched C13 alkyl sulfate anionic surfactant of 2-pentyl octyl sulfate anionic surfactant, and more than about 25% by weight the 2-branched C13 alkyl sulfate anionic surfactant of 2-methyl dodecyl sulfate anionic surfactant; and c) less than about 5% by weight of the C13 alkyl sulfate anionic surfactant of other branched C13 alkyl sulfate anionic surfactant, wherein a, b and c add up to about 100% by weight of the C13 alkyl sulfate anionic surfactant; alternatively with a surfactant B, wherein the surfactant B comprises a mixture of C12 and C13 alkyl sulfate anionic surfactants, optionally wherein the surfactant B consists essentially of a mixture of C12 and C13 alkyl sulfate anionic surfactants; wherein the mixture of the C12 and C13 alkyl sulfate anionic surfactants comprises a mixture of surfactant isomers of Formula I and surfactant isomers of Formula II:

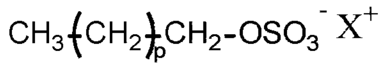

(I)

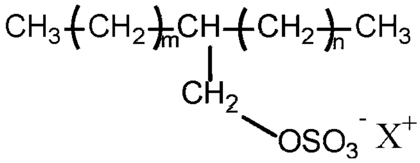

(II)

wherein $10 \leq p \leq 11$;

wherein $8 \leq m + n \leq 9$ and $0 \leq m, n \leq 9$; and

wherein X is a counter cation selected from the group consisting of lithium, sodium, potassium and ammonium, preferably sodium; and wherein the surfactant B comprises: from about 37% to about 50%, preferably from about 38% to about 49%, more preferably from about 40% to about 49% of the C12 alkyl sulfate anionic surfactant by weight of the surfactant B, wherein the C12 alkyl sulfate anionic surfactant comprises a 2-alkyl branched C12 alkyl sulfate; from about 50% to about 63%, preferably from about 51% to about 62%, more preferably from about 51% to about 60% of the C13 alkyl sulfate anionic surfactant by weight of the surfactant B, wherein the C13 alkyl sulfate anionic surfactant comprises a 2-alkyl branched C13 alkyl sulfate; and less or equal than about 5% of other alkyl sulfate anionic surfactants by weight of the surfactant B, preferably less or equal than about 4% of other alkyl sulfate anionic surfactants by weight of the surfactant B, wherein other alkyl sulfate anionic surfactants are not C12 or C13 alkyl sulfate anionic surfactant; and the step of placing the personal care composition onto the skin and/or hair of the consumer.

Use of a surfactant A or a surfactant B as set out herein for providing a structured and stable personal care composition.

Use of a surfactant A or a surfactant B as set out herein for providing a lather performance of a personal care composition.

Use of a surfactant A or a surfactant B and a hydroxysultaine as set out herein for providing mildness of a personal care composition.

Use of a surfactant A or a surfactant B as set out herein for providing fragrance experience of a personal care composition. Providing fragrance experience can mean leaving a pleasant and/or long-lasting scent onto the skin and/or hair.

Use of a surfactant A or a surfactant B as set out herein for depositing a benefit agent of a personal care composition for providing skin-feel attributes such as moisturization, or skin feeling less oily and/or sticky.

Method of Manufacturing

The personal care compositions may be prepared by any known or otherwise effective technique, suitable for

making and formulating the desired product form. It is also effective to combine toothpaste-tube filling technology with a spinning stage design. Additionally, the personal care composition may be prepared by the method and apparatus as disclosed in U.S. Pat. No. 6,213,166 issued to Thibiant, et al. The method and apparatus allows two or more compositions to be filled in a spiral configuration into a single container using at least two nozzles which fill the container, which is placed on a static mixer and spun as the composition is introduced into the container.

Alternatively, the personal care composition may be prepared by a method disclosed in commonly owned patent application published on November 18, 2004 under U.S. Publication No. 2004/0219119 A1 entitled "Visually distinctive multiple liquid phase compositions" filed by Wei, et al. on April 30, 2004. The method and apparatus allows two separate compositions to be combined in predetermined amounts, blended into a single resultant composition with visually distinct phases, and filled by one nozzle into a single container that is lowered and rotated during filling.

If the personal care compositions are patterned, it can be desirable to be packaged as a personal care article. The personal care article would comprise these compositions in a transparent or translucent package such that the consumer can view the pattern through the package. Because of the viscosity of the subject compositions it may also be desirable to include instructions to the consumer to store the package upside down, on its cap to facilitate dispensing.

Packaging

Personal care compositions can be dispensed from a squeezable package with an orifice, such as a conventional body wash or shampoo package. The package can be a compact package, i.e., contain less than about 250 ml, or 200 ml, or 150 ml of volume to signal the contents are concentrated. The shear thinning compositions can be dispensed from a package with a slit valve orifice or other flexible orifice, which is generally cut from a silicone elastomeric material and inserted into an orifice housing.

When the composition has a relatively low viscosity, preferably less than about 0.25 Pa.s, at 10 1/sec, it can be dispensed from a foaming package such as a pump foamer. Compositions can also be dispensed from liquid pump packages.

## TEST METHODS

It is understood that the test methods that are disclosed in the Test Methods Section of the present application should be used to determine the respective values of the parameters described and claimed herein.

T-Bar Viscosity Method

The viscosity of a personal care composition can be assessed by the T-Bar Viscosity Method. The apparatus for T-Bar measurements includes a Brookfield DV-II+ Pro Viscometer with Helipath Accessory; a chuck, weight and closer assembly for T-bar attachment; a T-bar Spindle D, a personal computer with Rheocalc software from Brookfield, and a cable connecting a Brookfield Viscometer to a computer. First, weigh 80 grams of a personal care composition in a 4-oz. glass jar. Measure a T-bar viscosity by carefully dropping the T-Bar Spindle to an interior bottom of the glass jar and set the Helipath stand to travel in an upward direction. Open the Rheocalc software and set the following data acquisition parameters: Speed to 5 rpm, Time Wait for Torque to 00:01 (1 second), and Loop Start Count to 100. Start data acquisition and turn on the Helipath stand to travel upward at a speed of 22 mm/minute. The T-Bar viscosity is an average T-Bar viscosity reading between the $10^{th}$ reading and the $90^{th}$ reading (the first ten readings and the last ten readings are not used for the average T-Bar viscosity calculation). The T-Bar viscosity reading is provided in cP (1 cP is equal to 1 mPa.s). After obtaining the initial viscosity reading, place the personal care composition at 50°C for 10 days for rapid aging. After finishing the stability testing at 50°C, the sample is equilibrated at 25°C for 24 hours. Then repeat viscosity measurement to obtain final viscosity. Measure percent change of the initial viscosity from the final viscosity measurement to obtain the percent change in viscosity.

Zero Shear Viscosity and Young's Modulus Methods

The Zero Shear Viscosity of a material which is a phase or a composition of the personal care composition, can be measured either prior to combining in the composition, after preparing a composition, or first separating a phase or component from a composition by suitable physical separation means, such as centrifugation, pipetting, cutting away mechanically, rinsing, filtering, or other separation means.

A controlled stress rheometer such as a TA Instruments Discovery HR2 Rheometer is used to determine the Zero Shear Viscosity. The determination is performed at 25°C with the 4 cm diameter parallel plate measuring system and a 1 mm gap. The geometry has a shear stress factor of 79580 $m^{-3}$ to convert torque obtained to stress. Serrated plates can be used to obtain consistent results when slip occurs.

First the material (i.e. the sample to be tested) is positioned on the rheometer base plate, the measurement geometry (upper plate) is moved into position 1.1 mm above the base plate. Excess material at the geometry edge is removed by scraping after locking the geometry. The geometry is then moved to the target 1 mm position above the base plate and a pause of about 1 minute is allowed to allow loading stresses to relax. This loading procedure ensures no tangential stresses are loaded at the measurement onset, which can influence results obtained. If the material comprises particles discernible to the eye or by feel (beads, e.g.) which are larger than about 150 microns in number average diameter, the gap setting between the base plate and upper plate is increased to the smaller of 4 mm or 8-fold the diameter of the 95th volume percentile particle diameter. If a phase has any particle larger than 5 mm in any dimension, the particles are removed prior to the measurement.

The measurement is performed by applying a continuous shear stress ramp from 0.1 Pa to 1,000 Pa over a time interval of 4 minutes using a logarithmic progression, i.e., measurement points evenly spaced on a logarithmic scale. Thirty (30) measurement points per decade of stress increase are obtained. If the measurement result is incomplete, for example if material is observed to flow from the gap, results obtained are evaluated with incomplete data points excluded. If there are insufficient points to obtain an accurate measurement, the measurement is repeated with increased number of sample points.

The Young's Modulus (Pa) is obtained by graphing the Stress (Pa) vs. Strain (unitless) and obtaining the slope of the regression line of the initial linear region between Stress vs. Strain, typically occurring in the region below about 4% strain. If the relationship is not linear, the linear regression line slope below 2% strain is taken as the Young's Modulus (Pa), using unitless strain.

The Zero Shear Viscosity is obtained by taking a first median value of viscosity in Pascal-seconds (Pa.sec) for viscosity data obtained between and including 0.1 Pa and the point where viscosity begins to steeply decline. After taking the first median viscosity, all viscosity values greater than 5-fold the first median value and less than 0.2x the median value are excluded, and a second median viscosity value is obtained of the same viscosity data, excluding the indicated data points. The second median viscosity so obtained is the Zero Shear Viscosity.

The personal care composition may have a Zero Shear Viscosity of at least about 1,000 Pa.s, alternatively at least about 1,500 Pa.s, alternatively at least about 2,500 Pa.s, alternatively at least about 5,000 Pa.s.

The personal care composition may have a Young's Modulus of at least about 40 Pa, alternatively at least about 50 Pa, alternatively at least about 75 Pa, alternatively at least about 100 Pa, alternatively at least about 150 Pa.

Ultracentrifugation Method

The Ultracentrifugation Method is used to determine the percent of a structured domain or an opaque structured domain (e.g., a lamellar phase) that is present in a multiphase personal care composition. The method involves the separation of the composition by ultracentrifugation into separate but distinguishable layers. The multiphase personal care composition described herein can have multiple distinguishable layers (e.g. a structured surfactant layer, and a benefit layer).

A composition is separated by ultracentrifuge into separate but distinguishable layers. The multiphase personal care composition described herein can have multiple distinguishable layers (e.g., a structured surfactant layer, and a benefit layer).

First, dispense about 4 grams of composition into a Beckman Centrifuge Tube (11x60 mm) to fill the tube. Place the centrifuge tubes in an ultracentrifuge (Beckman Model L8-M or equivalent) using a sling rotor and ultracentrifuge using the following conditions: 50,000 rpm, 2 hours, and 40°C.

Measure the relative phase volumes of the phases the composition by measuring the height of each layer using an Electronic Digital Caliper (within 0.01mm). Layers are identified by those skilled in the art by physical observation techniques paired with chemical identification if needed. For example, the structured surfactant layer is identified by transmission electron microscopically (TEM), polarized light microscopy, and/or X-ray diffraction described herein as a structured lamellar phase comprising multilamellar vesicles, and the hydrophobic benefit layer is identified by its low moisture content (less than 10% water as measured by Karl Fischer Titration). The total height $H_a$ is measured which includes all materials in the ultracentrifuge tube. Next, the height of each layer is measured from the bottom of the centrifuge tube to the top of the layer, and the span of each layer algebraically determined by subtraction. The benefit layer may comprise several layers if the benefit phase has more than one component which may phase splits into liquid and waxy layers, or if there is more than one benefit component. If the benefit phase splits, the sum of the benefit layers measured is the benefit layer height, $H_b$. Generally, a hydrophobic benefit layer when present, is at the top of the centrifuge tube.

The cleansing phase may comprise several layers or a single layer, $H_c$. There may also be a micellar, unstructured, clear isotropic layer at the bottom or next to the bottom of the ultracentrifuge tube. The layers immediately above the isotropic phase generally comprise higher surfactant concentration with higher ordered structures (such as liquid crystals). These structured layers are sometimes opaque to naked eyes, or translucent, or clear. There may be

several structured layers present, in which case $H_c$ is the sum of the individual structured layers. If any type of polymer-surfactant phase is present, it is considered a structured phase and included in the measurement of $H_c$. The sum of the aqueous phases is $H_s$.

Finally, the structured domain volume ratio is calculated as follows:

$$\text{Structured Domain Volume Ratio} = H_c / H_s * 100\%$$

If there is no benefit phase present, use the total height as the surfactant layer height, $H_s$=$H_a$. The Structured Domain Volume Ratio is the Lamellar Phase %.

The personal care composition may have a Structured Domain Volume Ratio of at least about 40%, alternatively at least about 50%, alternatively at least about 55%, alternatively at least about 60%, alternatively at least about 65%, alternatively at least about 70%, alternatively at least about 75%, alternatively at least about 80%, alternatively at least about 85%, alternatively at least about 90%, by volume of the aqueous structured surfactant phase.

<u>Third-Phase Method for Determining Structured Surfactant Stability</u>

The "Third-Phase" Method is used to determine structured surfactant phase stability in a personal care composition. The method involves placing the personal care compositions at 50°C for 10 days for rapid aging. After rapid aging, transfer about 4 grams of the composition into a Beckman Centrifuge Tube (11×60 mm). Place the centrifuge tube in a Beckman LE-80 Ultracentrifuge and operate the Ultracentrifuge under the following conditions: 50,000 rpm, 2 hours, and at 40°C.

After Ultracentrifugation, determine the third-phase volume by measuring the height of various surfactant phases using an Electronic Digital Caliper (within 0.01 mm) as illustrated in FIG. 1. An example is shown in FIG. 1 for a personal care composition comprising Expancel microsphere.

The very top layer is an hydrophobic benefit phase layer (hydrocarbons or soybean oil etc.). The layers below the hydrophobic benefit phase layers contain surfactant/water are determined in the following: $H_a$ is the height of all layers containing surfactant/water and $H_b$ is the height of the clear "third-phase" layer just below the hydrophobic benefit phase layer. It is important to record the readings within 30 mins after the Ultracentrifugation is finished to minimize material migration across different layers. The third phase volume is calculated as:

$$\text{Third-phase Volume \%} = H_b/H_a * 100\%$$

Preferably, the aqueous structured surfactant phase or composition may comprise less than 10% "third-phase" volume after rapid aging stability protocol. More preferably, the aqueous structured surfactant phase or composition may comprise less than 5% "third-phase" volume after rapid aging stability protocol. More preferably, the aqueous structured surfactant phase or composition may comprise less than 2% "third-phase" volume after rapid aging stability protocol. Even more preferably, the aqueous structured surfactant phase or composition may comprise less than 1% "third-phase" volume after rapid aging protocol. Most preferably, the aqueous structured surfactant phase or composition may comprise about 0% "third-phase" volume after rapid aging protocol.

**EXAMPLES**

The following examples further describe the compositions described herein. The examples are given solely for the purpose of illustration and are not to be construed as limitations of the present disclosure, as many variations thereof are possible without departing from the scope of the disclosure. Where applicable, ingredients are identified by chemical or CTFA name, or otherwise defined below.

<u>Examples of suitable alkyl sulfate anionic surfactants and their synthesis:</u>

<u>SURFACTANT A</u>

The following are representative and non-limiting examples of suitable C13 alkyl sulfate anionic surfactants as Surfactant A, including a non-limiting method of synthesis.

Using the above-described processes, the alcohol compositions described below in Alcohol Example 1 are obtained and analyzed by gas chromatography with flame ionization detection (GC/FID). The samples are prepared as a 1% (w/v) dichloromethane solution and injected into a capillary GC Column: DB-1 HT 15 m x 0.25 mm ID, 0.1 μ m film

thickness, using an oven temperature program [initial temperature 80°C (1 min), ramp 10°C/min to 220°C, ramp 30°C/min to 350°C (1 min)] for a total run time of 19 minutes. Additional GC parameters include Column Flow: 1.4 ml/min ($H_2$), Injection Temperature: 300°C, Sample Amount: 1 μL, Split Ratio: 1/400, FID Temperature: 350°C, $H_2$ Flow: 40 mL/min, Air Flow: 400 mL/min, and Makeup Gas Flow: 25 mL/min.

Alcohol Example 1. Synthesis of Narrow Branched Tridecanol (Alcohol 1)

A C12 linear alpha olefin feedstock (1-Dodecene) was obtained from the Chevron Phillips Chemical Company LP, as identified by product name AlphaPlus® 1-Dodecene (Chevron Phillips Chemical Company LP, P.O. Box 4910, The Woodlands, TX 77387-4910, US, phone (800) 231-3260). The homogeneous rhodium organophosphorus catalyst used in this example is prepared in a high pressure, stainless steel stirred autoclave. To the autoclave was added 0.027 wt.% Rh(CO)2ACAC ((Acetylacetonato)dicarbonylrhodium(I)), 1.36 wt.% tris (2,4,-di-t-butylphenyl) phosphite ligand and 98.62 wt.% Synfluid® PAO 4 cSt (Chevron Phillips Chemical Company LP, P.O. Box 4910, The Woodlands, TX 77387-4910) inert solvent. The mixture was heated at 80°C in the presence of a CO/$H_2$ atmosphere and 2 bar (0.2MPa above atmospheric) gauge pressure for four hours to produce the active rhodium catalyst solution (109 ppm rhodium, P:Rh molar ratio = 20). The 1-Dodecene linear alpha olefin was added to the rhodium catalyst solution in the autoclave producing a starting reaction mixture with a rhodium concentration of 35 ppm. The alpha olefin feed was then isomerized at 80°C in the presence of a CO/$H_2$ atmosphere and 1 bar (0.1MPa above atmospheric) gauge pressure for 10 hours. The isomerized olefin was then hydroformylated at 70°C in the presence of a CO/$H_2$ atmosphere and 20 bar (2MPa above atmospheric) gauge pressure for 8 hours. The molar ratio of CO to $H_2$ in both the isomerization step and the hydroformylation step was equal to 1:1.15. The resulting hydroformylation reaction product was flash distilled at 140-150°C and 25 millibar to recover the rhodium catalyst solution as a bottoms product and recover a branched C13 Aldehyde overheads product with a composition comprising:

| | |
|---|---|
| 1-Tridecanal | 13.9 wt.% |
| 2-Methyl-dodecanal | 28.3 wt.% |
| 2-Ethyl-undecanal | 15.2 wt.% |
| 2-Propyl-decanal | 14.5 wt.% |
| 2-Butyl-nonanal | 13.6 wt.% |
| 2-Pentyl-octanal | 12.6 wt.% |
| Other | 1.9 wt.% |

The weight % branching in the branched C13 aldehyde product was 86.2%.

The branched C13 aldehyde product was hydrogenated in a high pressure, Inconel 625 stirred autoclave at 150°C and 20 bar (2MPa above atmospheric) hydrogen gauge pressure. The hydrogenation catalyst used was a Raney® Nickel 3111 (W. R. Grace & Co., 7500 Grace Drive, Columbia, MD 21044, US, phone 1-410-531-4000) catalyst used at a 0.25 wt.% loading. The aldehyde was hydrogenated for 10 hours and the resultant reaction mixture was filtered to produce a branched C13 alcohol product (Alcohol 1 in Table 1) comprising:

| | |
|---|---|
| 1-Tridecanol | 13.36 wt.% |
| 2-Methyl-dodecanol | 28.95 wt.% |
| 2-Ethyl-undecanol | 16.25 wt.% |
| 2-Propyl-decanol | 13.92 wt.% |
| 2-Butyl-nonanol | 13.46 wt.% |
| 2-Pentyl-octanol | 13.02 wt.% |
| Other | 1.04 wt.% |

The weight % 2-alkyl branching in the branched C13 alcohol product was 85.6%.

Alkyl Sulfate Example 1. Synthesis of Narrow Branched Tridecanol Sulfate using a Falling Film Sulfation Reactor

The alcohol from Alcohol Example 1 is sulfated in a falling film using a Chemithon single 15 mm x 2 m tube reactor using $SO_3$ generated from a sulfur burning gas plant operating at 2.5 kg/h (5.5 lb/hr) sulfur to produce 3.76% $SO_3$ on a volume basis. Alcohol feed rate is 15.2 kg/hour and feed temperature was 27.2°C (81°F). Conversion of the alcohol to alcohol sulfate acid mix was achieved with 96.5% completeness. Neutralization with 50% sodium hydroxide is completed

at ambient process temperature to 0.65% excess sodium hydroxide. 33 gallons of sodium neutralized C13 narrow branched Alcohol Sulfate paste. Analyses by standard Cationic $SO_3$ titration method determines final average product activity to be 73.4%. The average unsulfated level is 2.10% w/w.

Surfactant A Example 2. Synthesis of Narrow Branched Tridecanol Sulfate using a Falling Film Sulfation Reactor with Amine Oxide Addition

The alcohol from Alcohol Example 1 is sulfated in a falling film using a Chemithon single 15 mm x 2 m tube reactor using $SO_3$ generated from a sulfur burning gas plant operating at 2.3 kg/h (5.0 lb/hr) sulfur to produce 3.76% $SO_3$ on a volume basis. Alcohol feed rate is 13.8 kg/hour and feed temperature was 23.9°C (75°F). Conversion of the alcohol to alcohol sulfate acid mix was achieved with 97% completeness. Neutralization is co-neutralized with 50% sodium hydroxide and with C12/14 dimethyl amine oxide at ambient process temperature to a pH of 8.0. 68 kilograms of the C13 narrow branched Alcohol Sulfate / Amine oxide paste was made to a target activity of 51.7% Alcohol sulfate and 11.76% C12/14 dimethyl amine oxide.

The effect of type of branching within the alkyl chain of the C13 alkyl sulfate anionic surfactants was evaluated for performance within personal care formulations in terms of gel stability, structured features, lather stability, miscibility and perfume benefits, following the test methods described hereinabove.

*Test materials:*

The relative performance was determined for C13 alkyl sulfate anionic surfactants based on the starting alcohol summarized in Table 1. The starting alcohol ex table 1 consisted essentially of C13 alkyl chains. Alcohol 1 used to make the C13 alkyl sulfate anionic surfactants in the inventive composition has a type of branching as described in the claims and were produced following the making process described herein.

**Table 1**: Alkyl chain distribution of starting C13 alcohols

| | Alcohol 1 from Alcohol Example 1 |
|---|---|
| Alkyl chain length | C13 |
| Linear content$^{+}$ | 13.4% |
| 2-Alkyl Branched C13 Alcohol$^{+}$ | 85.6% |
| Other $^{+++}$ | 1.0% |
| | |
| 2-methyl-1-dodecanol$^{+}$ | 29.0 |
| 2-ethyl-1-undecanol$^{+}$ | 16.2 |
| 2-propyl-1-decanol$^{+}$ | 13.9 |
| 2-butyl-1-nonanol$^{+}$ | 13.5 |
| 2-pentyl-1-octanol$^{+}$ | 13.0 |
| | |
| 2-Alkyl Branch distribution: | |
| 2-methyl-1-dodecanol$^{++}$ | 33.9% |
| 2-ethyl-1-undecanol$^{++}$ | 18.9% |
| 2-propyl-1-decanol$^{++}$ | 16.2% |
| 2-butyl-1-nonanol$^{++}$ | 15.8% |
| 2-pentyl-1-octanol$^{++}$ | 15.2% |
| $^{+}$ by weight of starting C13 alcohol<br>$^{++}$ by weight of branched C13 alcohol<br>$^{+++}$ such as isomers with branches in non-C2 positions, paraffins, alcohols with chain-lengths other than 13 carbons | |

The starting C13 alcohol of Table 1 was individually sulfated in the pilot plant according to one of the processes as set out above. The resulting alkyl sulfate distribution is retained and corresponds to the distribution of the alkyl chains as set out for the Alcohol 1 in Table 1.

SURFACTANT B

The following examples are representative and non-limiting examples of suitable surfactant B comprising a mixture of C12 and C13 alkyl sulfate anionic surfactants, including a non-limiting method of synthesis from a corresponding starting alcohol. The corresponding starting alcohol comprises the corresponding mixture of C12 and C13 alcohols.

Using the above-described process using sulfur trioxide, the starting alcohols described below as Alcohol Examples 2 and 3 are commercially available and analyzed by gas chromatography with flame ionization detection (GC/FID) as indicated below:

The samples are typically assessed by diluting the starting Alcohol Example to about 0.1% (v/v) in a volatile alcohol such as methanol or ethanol. The prepared samples were injected (1 μL Injection Volume) into a Splitless Inlet at 280°C and onto an Agilent DB-1, 30 m x 0.250 mm ID, 0.25 μm film thickness capillary GC column. The $H_2$ carrier gas is in constant pressure mode of 0.68 Bar (10.00 psi). The oven temperature program [50°C (2 min), ramped (10°C/min) to 300°C ( 3 min)] has a total run time of 30 minutes. The flame ionization detection (FID) temperature is 320°C with 30 mL/min $H_2$ flow, 300 mL/min Air flow, and 30 mL/min Makeup ($N_2$) flow.

The non-limiting starting alcohols were:

Alcohol Example 2. Tradename LIAL®123A alcohol commercially available from SASOL.
Alcohol Example 3. Tradename ISALCHEM®123A alcohol commercially available from SASOL.

Surfactant B Example 1. Synthesis of Surfactant B Alkyl Sulfate using a Falling Film Sulfation Reactor

The alcohol from Alcohol Example 2 was sulfated in a falling film using a Chemithon single 15 mm x 2 m tube reactor using $SO_3$ generated from a sulfur burning gas plant operating at 2.4 kg/h (5.3 lb/hr) sulfur to produce 3.56% $SO_3$ on a volume basis. Alcohol feed rate was 14.2 kg/hour (31.33 lb/hr) and feed temperature was 37.8°C (100°F). Conversion of the alcohol to alcohol sulfate acid mix was achieved with 97.75% completeness. Neutralization with 50% sodium hydroxide was completed at ambient process temperature to 0.55% excess sodium hydroxide. 124.9 L (33 gallons) of the sodium neutralized surfactant B sulfate paste. Analyses by standard Cationic $SO_3$ titration method determined final average product activity to be 69.8%.

Surfactant B Example 2. Synthesis of another Surfactant B Alkyl Sulfate using a Falling Film Sulfation Reactor

The same protocol as for Surfactant B Example 1 was used to prepare Surfactant B Example 2 from the alcohol from Alcohol Example 3. Analyses by standard Cationic $SO_3$ titration method determined final average product activity to be 28.3%.

The effect of type of branching within the alkyl chain of the surfactant B comprising a mixture of C12 and C13 alkyl sulfate anionic surfactants was evaluated for performance within personal care formulations in terms of Lamellar Phase Volume, Young's Modulus, and Zero Shear Viscosity, following the test methods described hereinabove.

*Test materials:*

The relative performance was determined for the surfactant B comprising a mixture of C12 and C13 alkyl sulfate anionic surfactants based on the starting alcohol summarized in Table 2. The starting Alcohol Example 2 in Table 2 comprised, or consisted essentially of C12 and C13 alkyl chains. Alcohol Example 2 used to make the surfactant B in the inventive composition has a type of branching as described in the claims and were produced following the making process described herein.

The Alcohol Example 2 was commercially available as tradename LIAL®123A alcohol supplied from SASOL and was analyzed by gas chromatography with flame ionization detection (GC/FID) as set out above. The alkyl chain distribution is provided in Table 2.

**Table 2:** Alkyl chain distribution of starting Alcohol Example 2. LIAL®123A alcohol commercially available from SASOL

| | Alcohol Example 2 |
|---|---|
| Alkyl chain length | **C12** |
| Total C12 Alcohol[1] | **45.6%** |
| Linear content C12 Alcohol[1] | 21.9% |
| 2-Alkyl Branched C12 Alcohol[1] | 23.7% |
| 2-Alkyl Branch C12 distribution: | |
| 2-methyl-1-undecanol[1] | 7.7% |
| 2-ethyl-1-decanol[1] | 4.1% |
| 2-propyl-1-nonanol[1] | 4.3% |
| 2-butyl-1-octanol, 2-pentyl-1-heptanol, other 2-Alkyl Branched C12 Alcohol[1] | 7.6% |
| | |
| Alkyl chain length | **C13** |
| Total C13 Alcohol[1] | **53.9%** |
| Linear content[1] | 27.9% |
| 2-Alkyl Branched C13 Alcohol[1] | 26.0% |
| 2-Alkyl Branch C13 distribution: | |
| 2-methyl-1-dodecanol[1] | 9.2% |
| 2-ethyl-1-undecanol[1] | 3.9% |
| 2-propyl-1-decanol[1] | 3.8% |
| 2-butyl-1-nonanol, 2-pentyl-1-octanol, other 2-Alkyl Branched C13 Alcohol[1] | 9.1% |
| Other[1, 6] | **0.5%** |
| TOTAL[1] | **100%** |
| Degree of branching | **49.7%** |
| | |
| Alkyl chain length | **C12** |
| Linear content C12 alcohol[2] | 48.0% |
| 2-Alkyl Branched C12 alcohol[2] | 52.0% |
| Total C12 alcohol[2] | 100% |
| 2-Alkyl Branch C12 distribution: | |
| 2-methyl-1-undecanol[3] | 32.5% |
| 2-ethyl-1-decanol[3] | 17.3% |
| 2-propyl-1-nonanol[3] | 18.2% |
| 2-butyl-1-octanol, 2-pentyl-1-heptanol, other 2-Alkyl Branched C12 alcohol [3] | 32.0% |
| | |
| **Alkyl chain length** | **C13** |
| Linear content C13 alcohol[4] | 51.8% |
| 2-Alkyl Branched C13 alcohol[4] | 48.2% |
| Total C13 alcohol[4] | 100% |
| 2-Alkyl Branched C13 distribution: | |

(continued)

| Alkyl chain length | C13 |
|---|---|
| 2-methyl-1-dodecanol[5] | 35.4% |
| 2-ethyl-1-undecanol[5] | 15.0% |
| 2-propyl-1-decanol[5] | 14.6% |
| 2-butyl-1-nonanol, 2-pentyl-1-octanol, other 2-Alkyl Branched C13 alcohol[5] | 35.0% |

[1] by weight of starting alcohol
[2] by weight of C12 alcohol
[3] by weight of 2-alkyl branched C12 alcohol
[4] by weight of C13 alcohol
[5] by weight of 2-alkyl branched C13 alcohol
[6] such as alcohol isomers with alkyl chain-lengths other than 12 or 13 carbons.

The starting Alcohol Example 2 of Table 2 was individually sulfated in the pilot plant according to the process as set out above to provide the Surfactant B Example 1. The resulting alkyl sulfate distribution (Table 3) is retained and corresponds to the distribution of the alkyl chains as set out for the Alcohol Example 2 in Table 2. The data as shown in Table 3 are only indicative as measured by a not yet standardized UHPLC-CAD method.

**Table 3:** Indicative alkyl chain distribution of the surfactant B including a mixture of C12 and C13 alkyl sulfate anionic surfactants obtained from LIAL®123A alcohol supplied by SASOL

| | Surfactant B Example 1 |
|---|---|
| Alkyl chain length | **C12** |
| Total C12 Alkyl sulfate[1] | **45.2%** |
| Linear content C12 Alkyl sulfate[1] | 21.6% |
| 2-Alkyl Branched C12 Alkyl sulfate[1] | 23.6% |
| 2-Alkyl Branch C12 distribution: | |
| 2-methyl-1-undecyl sulfate[1] | 7.7% |
| 2-ethyl-1-decyl sulfate[1] | 4.1% |
| 2-propyl-1-nonyl sulfate[1] | 4.3% |
| 2-butyl-1-octyl sulfate, 2-pentyl-1-heptyl sulfate, other 2-Alkyl Branched C12 Alkyl sulfate[1] | 7.5% |
| | |
| Alkyl chain length | **C13** |
| Total C13 Alkyl sulfate[1] | **51.9%** |
| Linear content C13 Alkyl sulfate[1] | 26.2% |
| 2-Alkyl Branched C13 Alkyl sulfate[1] | 25.7% |
| 2-Alkyl Branch C13 distribution: | |
| 2-methyl-1-dodecyl sulfate[1] | 9.1% |
| 2-ethyl-1-undecyl sulfate[1] | 3.9% |
| 2-propyl-1-decyl sulfate[1] | 3.9% |
| 2-butyl-1-nonyl sulfate, 2-pentyl-1-octyl sulfate, other 2-Alkyl Branched C13 Alkyl sulfate[1] | 8.8% |
| Other[1, 6] | **2.9%** |
| TOTAL[1] | **100%** |
| Degree of branching | **49.3%** |
| | |

(continued)

| Alkyl chain length | C12 |
|---|---|
| Linear content C12 Alkyl sulfate[2] | 47.8% |
| 2-Alkyl Branched C12 Alkyl sulfate[2] | 52.2% |
| Total C12 Alkyl sulfate[2] | 100% |
| 2-Alkyl Branched C12 distribution: | |
| 2-methyl-1-undecyl sulfate[3] | 32.6% |
| 2-ethyl-1-decyl sulfate[3] | 17.4% |
| 2-propyl-1-nonyl sulfate[3] | 18.2% |
| 2-butyl-1-octyl sulfate, 2-pentyl-1-heptyl sulfate, other 2-Alkyl Branched C12 Alkyl sulfate [3] | 31.8% |
| | |
| **Alkyl chain length** | **C13** |
| Linear content C13 Alkyl sulfate[4] | 50.5% |
| 2-Alkyl Branched C13 Alkyl sulfate[4] | 49.5% |
| Total C13 Alkyl sulfate[4] | 100% |
| 2-Alkyl Branched C13 distribution: | |
| 2-methyl-1-dodecyl sulfate[5] | 35.4% |
| 2-ethyl-1-undecyl sulfate[5] | 15.2% |
| 2-propyl-1-decyl sulfate[5] | 15.2% |
| 2-butyl-1-nonyl sulfate, 2-pentyl-1-octyl sulfate, other 2-Alkyl Branched C13 Alkyl sulfate[5] | 34.2% |

[1] by weight of the surfactant B
[2] by weight of C12 alkyl sulfate
[3] by weight of 2-alkyl branched C12 alkyl sulfate
[4] by weight of C13 alkyl sulfate
[5] by weight of 2-alkyl branched C13 alkyl sulfate
[6] such as isomers with alkyl chain-lengths other than 12 or 13 carbons.

The Alcohol Example 3 was commercially available as tradename ISACHELM®123A alcohol supplied from SASOL and was analyzed by gas chromatography with flame ionization detection (GC/FID) as set out above. The alkyl chain distribution is provided in Table 4.

The starting Alcohol Example 3 of Table 4 was also individually sulfated in the pilot plant according to the process as set out above to provide the Surfactant B Example 2. The resulting alkyl sulfate distribution is retained and corresponds to the distribution of the alkyl chains as set out for the Alcohol Example 3.

**Table 4:** Alkyl chain distribution of starting Alcohol Example 3. ISALCHEM®123A alcohol commercially available from SASOL

| | Alcohol Example 3 |
|---|---|
| Alkyl chain length | **C12** |
| Total C12 Alcohol[1] | 42.8% |
| | |
| Alkyl chain length | **C13** |
| Total C13 Alcohol[1] | 55.1% |
| Other[1, 6] | 2.1% |
| TOTAL[1] | **100%** |
| Degree of branching | 92.6% |

Comparative Surfactant

A comparative branched alkyl C13 alkyl sulfate as a comparative surfactant has been prepared from tridecyl alcohol. Tridecyl alcohol was commercially available as Exxal®13 from Exxon Mobil.

The comparative branched alkyl C13 alkyl sulfate was made according the following process: Tridecyl alcohol commercially available as Tradename Exxal®13 from Exxon Mobil was sulfated in a falling film using a Chemithon single 15 mm x 2 m tube reactor using $SO_3$ generated from a sulfur burning gas plant operating at 2.4 kg/h (5.3 lb/hr) sulfur to produce 3.56% $SO_3$ on a volume basis. Alcohol feed rate was 14.2 kg/hour (31.33 lb/hr) and feed temperature was 37.8°C (100°F). Conversion of the alcohol to alcohol sulfate acid mix was achieved with 97.75% completeness. Neutralization with 50% sodium hydroxide was completed at ambient process temperature to 0.55% excess sodium hydroxide. 124.9 L (33 gallons) of the sodium neutralized surfactant sulfate paste. Analyses by standard Cationic $SO_3$ titration method determined final average product activity to be about 29%.

The resulting comparative branched alkyl C13 alkyl sulfate was further concentrated to 69% - 71% activity for trying to make a concentrated personal care composition.

The resulting comparative branched alkyl C13 alkyl sulfate had two phases as evidenced by microscopy: A smeared birefringent texture had been observed with a relatively elevated level of a dispersed crystalline phase (spherical with birefringent cross pattern). It is not uncommon for a material to have a crystalline phase at ambient temperature. This requires heated transportation and storage. However, even at 65°C the comparative branched alkyl C13 alkyl sulfate still remains in two phases.

The rheology of the comparative branched alkyl C13 alkyl sulfate appears to make it unsuitable for manufacture and commercial use. It is a gel with a somewhat hard texture at all temperatures evaluated, which means it would be difficult to manufacture into a personal care composition as claimed.

Also, notably, at a large scale, since the comparative branched alkyl C13 alkyl sulfate does not have a fluid rheology, it would be nearly impossible to manufacture the comparative branched alkyl C13 alkyl sulfate because of phase separation at the end of the sulfation process. Hence, there is a need to select a better surfactant than can lead to a personal care composition being stable and having satisfying rheological properties.

**SURFACTANT COMPOSITION COMPARISONS**

Surfactant A

The surfactant compositions of Table 5 (below) were prepared by adding water in a mixing vessel. Then add the following ingredients with continuously mixing: sodium lauroamphoacetate or cocamidopropyl hydroxysultaine, the anionic surfactant, trideceth-3, EDTA, sodium benzoate, and sodium chloride. Adjust pH by adding citric acid solution (50% active) to pH = 5.7± 0.2. Then, add Methylchloroisothiazolinone and methyl isothiazolinone. Keep mixing until homogeneous.

After preparing these compositions, their Lamellar Phase Volume, Young's Modulus, T-bar viscosity and Zero Shear Viscosity were determined utilizing the methods disclosed herein. The results are captured below in Table 5.

Stability is typically assessed by measuring the Lamellar Phase Volume through the Ultracentrifugation Method as set out hereinbefore, after aging the products at 50°C for 10 days.

Comparative Example 1 comprises as the anionic surfactant, sodium isotridecyl sulfate which comprises a mixture of branched chain 13 carbon aliphatic alkyl sulfates. Comparative Example 1 was not structured and not stable at all with no lamellar phase volume and a relatively low Young's modulus and Zero Shear Viscosity.

However, when sodium isotridecyl sulfate has been replaced by a surfactant A comprising the C13 alkyl sulfate anionic surfactant as described herein like sodium C13 Alkyl sulfate anionic surfactant obtained from Alcohol 1 (Comparative Example 2), a significant improvement of the viscosity profile of the surfactant phase composition has been observed with a significant increase of both Young's modulus and Zero Shear Viscosity.

When the amphoteric surfactant sodium lauroamphoacetate has been substituted by cocamidopropyl hydroxysultaine as the zwitterionic surfactant, without any addition of an electrolyte such as sodium chloride, no lamellar phase could be obtained and phases separated. When the amphoteric surfactant sodium lauroamphoacetate has been substituted by cocamidopropyl hydroxysultaine as the zwitterionic surfactant, addition of an electrolyte such as sodium chloride can provide a further significant increase in the of both Young's modulus and Zero Shear Viscosity has been obtained. For this, the amount of sodium chloride should be at least 3% by weight of the composition. Example 3 with a maintained 100% Lamellar Phase Volume was structured and stable; and has an improved viscosity profile, showing the effectiveness of combining a C13 alkyl sulfate anionic surfactant as recited herein with a zwitterionic surfactant comprising a hydroxysultaine and sodium chloride at a higher level to drive the lamellar phase formation.

**Table 5**

| **Surfactant Phase Composition** | Comparative Example 1 (w/w %) | Comparative Example 2 (w/w %) | Comparative Example 3 (w/w %) | Comparative Example 4 (w/w %) |
|---|---|---|---|---|
| Sodium isotridecyl Sulfate [1] | 16.56 | - | - | - |
| C13 Alkyl sulfate from Alcohol 1 | - | 16.56 | 16.56 | 16.56 |
| Sodium Lauroamphoacetate [2] | 4.94 | 4.94 | - | - |
| Cocamidopropyl hydroxysultaine[3] | - | - | 4.94 | 4.94 |
| Trideceth-3 (HLB=8) [4] | 2.0 | 2.0 | 2.0 | 2.0 |
| Sodium Chloride (added) | 4.75 | 4.75 | 0 | 1 |
| Total level of electrolyte (sodium chloride) | 5.75 | 5.75 | 0.56 | 1.56 |
| Methylchloroisothiazolinone and methylisothiazolinone [5] | 0.033 | 0.033 | 0.033 | 0.033 |
| EDTA [6] | 0.15 | 0.15 | 0.15 | 0.15 |
| Sodium Benzoate | 0.2 | 0.2 | 0.2 | 0.2 |
| Citric Acid, titrate (pH = ± 0.2) | 5.7 | 5.7 | 5.7 | 5.7 |
| **Water** | **Q.S.** | **Q.S.** | **Q.S.** | **Q.S.** |
| | | | | |
| *Total Lathering Surfactant in Cleansing Phase (%)* | 21.5% | 21.5% | 21.5% | 21.5% |
| Lamellar Phase Volume (%) | 0% | 0% | - | - |
| Young's Modulus (Pa) | 0.26 | 13 | Separated | Separated |
| Zero Shear Viscosity (Pa.s) | 10.7 | 286.9 | Separated | Separated |
| T-bar Viscosity (cP) | | | 2000 | 3333 |

[1.] prepared by sulfation of Exxal™ 13 available from ExxonMobil; [2] available from Cognis Chemical Corp.; [3.] available as StarSurf™ CAPHS 50 from StarChem, LLC.; [4.] Iconal TDA-3 available from BASF Corp.; [5.] Kathon CG, available from Rohm & Haas Company, Philadephia, PA; [6.] Dissolvine NA 2x.

| **Surfactant Phase Composition** | Comparative Example 5 (w/w %) | Example 1 (w/w %) | Example 2 (w/w %) | Example 3 (w/w %) |
|---|---|---|---|---|
| Sodium isotridecyl Sulfate [1] | - | - | - | - |
| C13 Alkyl sulfate from Alcohol 1 | 16.56 | 16.56 | 16.56 | 16.56 |
| Sodium Lauroamphoacetate [2] | - | - | - | - |
| Cocamidopropyl hydroxysultaine[3] | 4.94 | 4.94 | 4.94 | 4.94 |
| Trideceth-3 (HLB=8) [4] | 2.0 | 2.0 | 2.0 | 2.0 |
| Sodium Chloride (added) | 2 | 3 | 4 | 5 |
| Total level of electrolyte (sodium chloride) | 2.56 | 3.56 | 4.56 | 5.56 |
| Methylchloroisothiazolinone and methylisothiazolinone [5] | 0.033 | 0.033 | 0.033 | 0.033 |
| EDTA [6] | 0.15 | 0.15 | 0.15 | 0.15 |
| Sodium Benzoate | 0.2 | 0.2 | 0.2 | 0.2 |
| Citric Acid, titrate (pH = ± 0.2) | 5.7 | 5.7 | 5.7 | 5.7 |
| **Water** | **Q.S.** | **Q.S.** | **Q.S.** | **Q.S.** |

(continued)

| **Surfactant Phase Composition** | Comparative Example 5 (w/w %) | Example 1 (w/w %) | Example 2 (w/w %) | Example 3 (w/w %) |
|---|---|---|---|---|
| | | | | |
| *Total Lathering Surfactant in Cleansing Phase (%)* | 21.5% | 21.5% | 21.5% | 21.5% |
| Lamellar Phase Volume (%) | - | 65% | 94% | 100% |
| Young's Modulus (Pa) | Separated | 81 | 60 | 50 |
| Zero Shear Viscosity (Pa.s) | Separated | 2304 | 1735 | 1584 |
| T-bar viscosity (cP) | 9333 | 10267 | 8533 | 8533 |

The surfactant compositions of Table 6 (below) were prepared by adding water in a mixing vessel. Then add the following ingredients with continuously mixing: cocamidopropyl hydroxysultaine, the respective sodium C13 alkyl sulfate anionic surfactant, sodium lauryl sulfate, trideceth-3, sodium benzoate, and sodium chloride. Adjust pH by adding citric acid solution (50% active) to pH = 5.7 ± 0.2. Then, add Methylchloroisothiazolinone and methyl isothiazolinone. Keep mixing until homogeneous.

After preparing these compositions, their Lamellar Phase Volume, Young's Modulus, and Zero Shear Viscosity were determined utilizing the methods disclosed herein. The results are captured below in Table 6.

The aqueous structured surfactant phase composition may also include an additional anionic surfactant such as sodium lauryl sulfate in order to reduce the level of the C13 alkyl sulfate anionic surfactant while increasing the total level of lathering surfactants without impacting the stability and the structure of the surfactant phase composition as shown in Examples 4, 5 and 6. Increasing the total level of lathering surfactants can help to enhance lather performance.

The aqueous structured surfactant phase composition including sodium C13 Alkyl sulfate anionic surfactant obtained from Alcohol 1 and cocamidopropyl hydroxysultaine could lead to an increase of the viscosity profile in terms of both Young's modulus and Zero Shear Viscosity and the formation of a lamellar structure upon a specific level of the electrolyte at least at 3% by weight of the composition. Again, the data shows that the effectiveness of using in the cleansing phase a C13 Alkyl sulfate anionic surfactant and an hydroxysultaine.

For the personal care composition, still the level of the electrolyte can be at least 3 wt.%. As shown in Example 4 which is only an aqueous surfactant phase composition, an acceptable lamellar phase could be obtained with at least at 3.0 wt.% level of electrolyte, namely 3.76 wt.%.

**Table 6**

| **Composition** | Comparative Example 6 (w/w %) | Comparative Example 7 (w/w %) | Comparative Example 8 (w/w %) | Example 4 (w/w %) |
|---|---|---|---|---|
| C13 Alkyl sulfate from Alcohol 1 | 6.66 | 6.66 | 6.66 | 6.66 |
| Sodium Lauryl Sulfate [2] | 6.66 | 6.66 | 6.66 | 6.66 |
| Cocamidopropyl hydroxysultaine[3] | 6.67 | 6.67 | 6.67 | 6.67 |
| Trideceth-3 (HLB=8) [4] | 2.00 | 2.00 | 2.00 | 2.00 |
| Sodium chloride (added) | 0.00 | 1 | 2 | 3 |
| Total level electrolyte (sodium chloride) | 0.76 | 1.76 | 2.76 | 3.76 |
| Methyl chloroisothiazolinone and methyl isothiazolinone [5] | 0.03 | 0.03 | 0.03 | 0.03 |
| EDTA 6 | 0.17 | 0.17 | 0.17 | 0.17 |
| Sodium Benzoate | 0.34 | 0.34 | 0.34 | 0.34 |
| Citric Acid, titrate (pH = ± 0.4) | 5.70 | 5.70 | 5.70 | 5.70 |
| **Water** | **Q.S.** | **Q.S.** | **Q.S.** | **Q.S.** |
| | | | | |

(continued)

| **Composition** | Comparative Example 6 (w/w %) | Comparative Example 7 (w/w %) | Comparative Example 8 (w/w %) | Example 4 (w/w %) |
|---|---|---|---|---|
| *Total Lathering Surfactant Component in Composition* | 20% | 20% | 20% | 20% |
| Lamellar Phase Volume (%) | 0% | 0% | 38% | 55% |
| Young's Modulus (Pa) | separated | separated | separated | 22 |
| Zero Shear Viscosity (Pa.s) | separated | separated | separated | 821 |
| T-bar Viscosity (cP) | separated | separated | separated | 5467 |
| 2. available from Procter & Gamble Co.; 3 available as StarSurf™ CAPHS 50 from StarChem, LLC.; 4. Iconal TDA-3 available from BASF Corp.; 5. Kathon CG, available from Rohm & Haas Company, Philadephia, PA; 6. Dissolvine NA 2x. | | | | |

| **Composition** | Example 5 (w/w %) | Example 6 (w/w %) |
|---|---|---|
| C13 Alkyl sulfate from Alcohol 1 | 6.66 | 6.66 |
| Sodium Lauryl Sulfate [2] | 6.66 | 6.66 |
| Cocamidopropyl hydroxysultaine[3] | 6.67 | 6.67 |
| Trideceth-3 (HLB=8) [4] | 2.00 | 2.00 |
| Sodium chloride (added) | 4 | 5 |
| Total level electrolyte (sodium chloride) | 4.76 | 5.76 |
| Methyl chloroisothiazolinone and methyl isothiazolinone [5] | 0.03 | 0.03 |
| EDTA 6 | 0.17 | 0.17 |
| Sodium Benzoate | 0.34 | 0.34 |
| Citric Acid, titrate (pH = ± 0.4) | 5.70 | 5.70 |
| **Water** | **Q.S.** | **Q.S.** |
| | | |
| *Total Lathering Surfactant Component in Composition* | 20% | 20% |
| Lamellar Phase Volume (%) | 89% | 100% |
| Young's Modulus (Pa) | 38 | 31 |
| Zero Shear Viscosity (Pa.s) | 1111 | 946 |
| T-bar Viscosity (cP) | 4933 | 4800 |

The surfactant compositions forming a cleansing phase may also comprise a rheology modifier to build the cleansing phase structure. The surfactant compositions of Table 7 (below) were prepared by adding water in a mixing vessel. Then add the following ingredients with continuously mixing: sodium chloride, guar hydroxypropyltrimonium chloride, rheology modifier powers (Aqupec or Structure XL or C*HiForm™ A12747), trideceth-3, xanthan gum, cocamidopropyl hydroxysultaine, sodium C13 alkyl sulfate anionic surfactant with an adequate agitation. Then add EDTA and sodium benzoate. Adjust pH by adding citric acid solution (50% active) to pH = 5.7 ± 0.2. Then, add methylchloroisothiazolinone and methyl isothiazolinone. Keep mixing until homogeneous.

After preparing these compositions, their Lamellar Phase Volume, Young's Modulus, and Zero Shear Viscosity were determined utilizing the methods disclosed herein. The results are captured below in Table 7.

Stability was assessed by measuring through Ultracentrifugation Method after aging the products at 50°C for 10 days.

**Table 7**

| **Composition** | Example 7 (w/w %) | Example 8 (w/w %) | Example 9 (w/w %) |
|---|---|---|---|
| C13 Alkyl sulfate from Alcohol 1 | 6.66 | 6.66 | 6.66 |
| Sodium Lauryl Sulfate [2] | 6.66 | 6.66 | 6.66 |
| Cocamidopropyl hydroxysultaine[3] | 6.66 | 6.67 | 6.67 |
| Trideceth-3 (HLB=8) [4] | 1.64 | 1.64 | 1.64 |
| Sodium chloride (added) | 5 | 5 | 5 |
| Total level electrolyte (sodium chloride) | 5.76 | 5.76 | 5.76 |
| Acrylates/C10-C30 alkyl acrylates crosspolymer (Aqupec SER-300) | - | - | 0.05 |
| Hydroxylpropyl starch phosphate (Structure XL) | 1.00 | - | - |
| Hydroxylpropyl starch phosphate (C*HiForm™ A12747) | - | 1.00 | - |
| Guar Hydroxypropyltrimonium Chloride | 0.43 | 0.43 | 0.43 |
| Xanthan gum | 0.33 | 0.33 | 0.33 |
| Methyl chloroisothiazolinone and methyl isothiazolinone [5] | 0.04 | 0.04 | 0.04 |
| EDTA [6] | 0.17 | 0.17 | 0.17 |
| Sodium Benzoate | 0.34 | 0.34 | 0.34 |
| Citric Acid, titrate (pH = ± 0.4) | 5.70 | 5.70 | 5.70 |
| **Water** | **Q.S.** | **Q.S.** | **Q.S.** |
| | | | |
| *Total Lathering Surfactant Component in Composition* | 20% | 20% | 20% |
| Lamellar Phase Volume (%) | 66% | 62% | 72% |
| Young's Modulus (Pa) | 117 | 105 | 52 |
| Zero Shear Viscosity (Pa.s) | 2813 | 2434 | 1218 |
| T-bar viscosity (cP) | 32000 | 38930 | 25470 |
| **Stability** | **10 days at 50°C** | | |
| Lamellar Phase Volume (%) | 64% | 63% | 72% |
| Young's Modulus (Pa) | 71 | 74 | 38 |
| Zero Shear Viscosity (Pa.s) | 1765 | 1909 | 902 |
| T-bar viscosity (cP) | 28930 | 31470 | 17600 |
| [2.] available from Procter & Gamble Co.; [3] available as StarSurf™ CAPHS 50 from StarChem, LLC.; [4.] Iconal TDA-3 available from BASF Corp.; [5.] Kathon CG, available from Rohm & Haas Company, Philadephia, PA; [6.] Dissolvine NA 2x. | | | |

Examples 7-9 appeared also very stable after being maintained during 10 days at 50°C. The Lamellar Phase Volume, Young's Modulus, Zero Shear Viscosity, and T-bar viscosity did not drop significantly.

The surfactant compositions forming a cleansing phase may comprise a rheology modifier to build the cleansing phase structure. The surfactant compositions are combined with a benefit phase to form a personal care composition. The cleansing and benefit phases were combined through SpeedMixer™ (Model DAC, 400FV available from FleckTeck, Inc USA) at 2 000 rpm for 60 seconds.

The compositions of Table 8 (below) were prepared by first adding water in a mixing vessel. Then add the following ingredients with continuously mixing: sodium chloride, guar hydroxypropyltrimonium chloride, rheology modifier powers (Aqupec or Structure XL), trideceth-3, xanthan gum, cocamidopropyl hydroxysultaine, sodium C13 alkyl sulfate anionic surfactant as surfactant A with an adequate agitation. Then add EDTA and sodium benzoate. Adjust pH by adding citric acid solution (50% active) to pH = 5.7 ± 0.2. Then, add methylchloroisothiazolinone and methyl isothiazolinone. Keep

mixing until homogeneous.

The personal care compositions are prepared by adding soybean oil or petrolatum, glyceryl monooleate and other ingredients such as fragrance and dye into the cleansing phase composition through a SpeedMixer™ at a speed of 2,000rpm for 60 seconds.

After forming the personal care compositions, their Lamellar Phase Volume, Young's Modulus, and Zero Shear Viscosity were determined utilizing the methods disclosed. The results are captured below in Table 8.

Stability was assessed by measuring through Ultracentrifugation Method after aging the products at 50°C for 10 days.

**Table 8**

| **Composition** | Example 10 (w/w %) |
|---|---|
| C13 Alkyl sulfate from Alcohol 1 | 6.24 |
| Sodium Lauryl Sulfate [2] | 6.24 |
| Cocamidopropyl hydroxysultaine[3] | 6.25 |
| Trideceth-3 (HLB=8) [4] | 1.54 |
| Sodium chloride (added) | 4.68 |
| Total level electrolyte (sodium chloride) | 5.39 |
| Hydroxylpropyl starch phosphate (Structure XL) | 0.94 |
| Guar Hydroxypropyltrimonium Chloride | 0.40 |
| Xanthan gum | 0.31 |
| Methyl chloroisothiazolinone and methyl isothiazolinone [5] | 0.04 |
| EDTA [6] | 0.16 |
| Sodium Benzoate | 0.32 |
| Citric Acid, titrate (pH = ± 0.4) | 5.70 |
| Soybean oil | 4.85 |
| Glyceryl Monooleate | 0.05 |
| BHT | 0.10 |
| Fragrance | 1.35 |
| **Water** | **Q.S.** |
| | |
| *Total Lathering Surfactant Component in Composition* | 18.73% |
| Lamellar Phase Volume (%) | 64% |
| Young's Modulus (Pa) | 161 |
| Zero Shear Viscosity (Pa.s) | 4344 |
| T-bar viscosity (cP) | 41200 |
| **Stability** | **10 days at 50°C** |
| Lamellar Phase Volume (%) | 63% |
| Young's Modulus (Pa) | 124 |
| Zero Shear Viscosity (Pa.s) | 3254 |
| T-bar viscosity (cP) | 37500 |

Example 10 illustrate the effectiveness of an hydrophobically modified polyacrylate like an hydrophobically modified polysaccharide, particularly a modified starch for providing the higher structure and higher stability to the overall personal care composition. Another noticeable advantage of using an hydrophobically modified polysaccharide, particularly a modified starch is that the modified starch is a naturally-derived and biodegradable rheology modifier which can

form sustainable personal care compositions.

Example 10 appeared also very stable after being maintained during 10 days at 50°C. The Lamellar Phase Volume, Young's Modulus, Zero Shear Viscosity, and T-bar viscosity did not drop significantly.

Surfactant B

The following compositions were prepared and assessed. Surfactant phase compositions were prepared using the Surfactant B Example 1. The Surfactant B Example 1 is the sulfated product prepared from the Alcohol Example 2 that was commercially available as tradename LIAL®123A alcohol supplied from SASOL.

The surfactant compositions of Table 9 (below) were prepared by adding water in a mixing vessel. Then add the following ingredients with continuously mixing: sodium lauroamphoacetate or cocamidopropyl hydroxysultaine, the anionic surfactant (Surfactant B Example 1 or 2, or sodium isotridecyl sulfate), trideceth-3, EDTA, sodium benzoate, and sodium chloride. Adjust pH by adding citric acid solution (50% active) to pH = 5.7± 0.2. Then, add methylchloroisothiazolinone and methyl isothiazolinone. Keep mixing until homogeneous.

After preparing these compositions, their Lamellar Phase Volume, Young's Modulus, and Zero Shear Viscosity were determined utilizing the methods disclosed herein. The results are captured below in Table 9.

Stability is typically assessed by measuring the Lamellar Phase Volume through the Ultracentrifugation Method as set out hereinbefore, after aging the products at 50°C for 10 days.

Comparative Example 1 comprises as the anionic surfactant, sodium isotridecyl sulfate which comprises a mixture of branched chain 13 carbon aliphatic alkyl sulfates. Comparative Example 1 was not structured and not stable at all with no lamellar phase volume and a relatively low Young's modulus and Zero Shear Viscosity.

However, when sodium isotridecyl sulfate has been replaced by a Surfactant B Example 1 being the sulfated product prepared from the Alcohol Example 2 that was commercially available as tradename LIAL®123A alcohol supplied from SASOL (Comparative Example 2a), a significant improvement of the viscosity profile of the surfactant phase composition has been observed with a significant increase of both Young's modulus and Zero Shear Viscosity.

When the amphoteric surfactant sodium lauroamphoacetate has been substituted by cocamidopropyl hydroxysultaine as the zwitterionic surfactant, without any addition of an electrolyte such as sodium chloride, no lamellar phase could be obtained and phases separated. When the amphoteric surfactant sodium lauroamphoacetate has been substituted by cocamidopropyl hydroxysultaine as the zwitterionic surfactant, addition of an electrolyte such as sodium chloride can provide a further significant increase in the of both Young's modulus and Zero Shear Viscosity has been obtained. For this, the amount of sodium chloride should be at least 3% by weight of the composition. Example 11 with a maintained 63% Lamellar Phase Volume was structured and stable; and has an improved viscosity profile, showing the effectiveness of combining a Surfactant B as recited herein with a zwitterionic surfactant comprising a hydroxysultaine to drive the lamellar phase formation.

**Table 9**

| **Surfactant Phase Composition** | Comparative Example 1 (w/w %) | Comparative Example 2a (w/w %) | Comparative Example 9 (w/w %) | Comparative Example 10 (w/w %) |
|---|---|---|---|---|
| Sodium isotridecyl Sulfate [1] | 16.56 | - | - | - |
| Surfactant B Example 1 | - | 16.56 | 16.56 | 16.56 |
| Sodium Lauroamphoacetate [2] | 4.94 | 4.94 | - | - |
| Cocamidopropyl hydroxysultaine[3] | - | - | 4.94 | 4.94 |
| Trideceth-3 (HLB=8) [4] | 2.0 | 2.0 | 2.0 | 2.0 |
| Sodium Chloride (added) | 4.75 | 4.75 | 0.00 | 1.00 |
| Total level electrolyte (sodium chloride) | 5.75 | 5.75 | 0.56 | 1.56 |
| Methylchloroisothiazolinone and methylisothiazolinone [5] | 0.033 | 0.033 | 0.033 | 0.033 |
| EDTA [6] | 0.15 | 0.15 | 0.15 | 0.15 |
| Sodium Benzoate | 0.2 | 0.2 | 0.2 | 0.2 |
| Citric Acid, titrate (pH = ± 0.2) | 5.7 | 5.7 | 5.7 | 5.7 |

(continued)

| Surfactant Phase Composition | Comparative Example 1 (w/w %) | Comparative Example 2a (w/w %) | Comparative Example 9 (w/w %) | Comparative Example 10 (w/w %) |
|---|---|---|---|---|
| **Water** | **Q.S.** | **Q.S.** | **Q.S.** | **Q.S.** |
| | | | | |
| *Total Lathering Surfactant in Cleansing Phase (%)* | 21.5% | 21.5% | 21.5% | 21.5% |
| Lamellar Phase Volume (%) | 0% | 0% | 0% | 0% |
| Young's Modulus (Pa) | 0.26 | 2 | separated | separated |
| Zero Shear Viscosity (Pa.s) | 10.7 | 52 | separated | separated |
| **Surfactant Phase Composition** | Comparative Example 11 (w/w %) | Example 11 (w/w %) | Example 12 (w/w %) | Example 13 (w/w %) |
| Sodium isotridecyl Sulfate [1] | - | - | - | - |
| Surfactant B Example 1 | 16.56 | 16.56 | 16.56 | 16.56 |
| Sodium Lauroamphoacetate [2] | - | - | - | - |
| Cocamidopropyl hydroxysultaine[3] | 4.94 | 4.94 | 4.94 | 4.94 |
| Trideceth-3 (HLB=8) [4] | 2.0 | 2.0 | 2.0 | 2.0 |
| Sodium Chloride (added) | 2.00 | 3.00 | 4.00 | 5.00 |
| Total level electrolyte (sodium chloride) | 2.56 | 3.56 | 4.56 | 5.56 |
| Methylchloroisothiazolinone and methylisothiazolinone [5] | 0.033 | 0.033 | 0.033 | 0.033 |
| EDTA [6] | 0.15 | 0.15 | 0.15 | 0.15 |
| Sodium Benzoate | 0.2 | 0.2 | 0.2 | 0.2 |
| Citric Acid, titrate (pH = ± 0.2) | 5.7 | 5.7 | 5.7 | 5.7 |
| **Water** | **Q.S.** | **Q.S.** | **Q.S.** | **Q.S.** |
| | | | | |
| *Total Lathering Surfactant in Cleansing Phase (%)* | 21.5% | 21.5% | 21.5% | 21.5% |
| Lamellar Phase Volume (%) | 13% | 28.5% | 41% | 63% |
| Young's Modulus (Pa) | separated | | 48 | 71 |
| Zero Shear Viscosity (Pa.s) | separated | | 1779 | 1999 |

1. prepared by sulfation of Exxal™ 13 available from ExxonMobil; 2. available from Cognis Chemical Corp.; 3. available as StarSurf™ CAPHS 50 from StarChem, LLC.; 4. Iconal TDA-3 available from BASF Corp.; 5. Kathon CG, available from Rohm & Haas Company, Philadephia, PA; 6. Dissolvine NA 2x.

For the personal care composition, still the level of the electrolyte can be at least 3 wt.%. As shown in Example 11 which is only an aqueous surfactant phase composition, an acceptable lamellar phase at 28.5% is obtained at least at 3.56 wt.% level of electrolyte. However, with the personal care composition comprising a benefit agent, a lamellar phase could be obtained with a level of electrolyte of at least 3 wt.%.

The surfactant compositions forming a cleansing phase of Table 10 (below) were prepared by first adding water in a mixing vessel. Then add the following ingredients with continuously mixing: cocamidopropyl hydroxysultaine, the respective surfactant B, sodium lauryl sulfate, trideceth-3, sodium benzoate, and sodium chloride. Adjust pH by adding citric acid solution (50% active) to pH = 5.7 ± 0.2. Then, add Methylchloroisothiazolinone and methyl isothiazolinone. Keep

mixing until homogeneous.

After forming the personal care compositions, their Lamellar Phase Volume, Young's Modulus, and Zero Shear Viscosity were determined utilizing the methods disclosed. The results are captured below in Table 10.

The aqueous structured surfactant phase composition may also include an additional anionic surfactant such as sodium lauryl sulfate in order to reduce the level of the surfactant B while increasing the total level of lathering surfactants without impacting the stability and the structure of the surfactant phase composition. Increasing the total level of lathering surfactants can help to enhance lather performance.

The aqueous structured surfactant phase composition including Surfactant B Example 1 and cocamidopropyl hydroxysultaine could lead to an increase of the viscosity profile in terms of both Young's modulus and Zero Shear Viscosity and the formation of a lamellar structure upon a specific level of the electrolyte at least at 3% by weight of the composition. Again, the data shows that the effectiveness of using in the cleansing phase a surfactant B and an hydroxysultaine.

**Table 10**

| **Composition** | Comparative Example 12 (w/w %) | Comparative Example 13 (w/w %) | Comparative Example 14 (w/w %) | Example 14 (w/w %) |
|---|---|---|---|---|
| Surfactant B Example 1 | 6.66 | 6.66 | 6.66 | 6.66 |
| Sodium Lauryl Sulfate [2] | 6.66 | 6.66 | 6.66 | 6.66 |
| Cocamidopropyl hydroxysultaine[3] | 6.67 | 6.67 | 6.67 | 6.67 |
| Trideceth-3 (HLB=8) [4] | 2.00 | 2.00 | 2.00 | 2.00 |
| Sodium chloride (added) | 0 | 1 | 2 | 3 |
| Total level electrolyte (sodium chloride) | 0.76 | 1.76 | 2.76 | 3.76 |
| Methyl chloroisothiazolinone and methyl isothiazolinone [5] | 0.03 | 0.03 | 0.03 | 0.03 |
| EDTA [6] | 0.15 | 0.15 | 0.15 | 0.15 |
| Sodium Benzoate | 0.20 | 0.20 | 0.20 | 0.20 |
| Citric Acid, titrate (pH = ± 0.4) | 5.70 | 5.70 | 5.70 | 5.70 |
| **Water** | **Q.S.** | **Q.S.** | **Q.S.** | **Q.S.** |
| | | | | |
| *Total Lathering Surfactant Component in Composition* | 20% | 20% | 20% | 20% |
| Lamellar Phase Volume (%) | 0% | 0% | 16% | 43% |
| Young's Modulus (Pa) | separated | separated | separated | 58 |
| Zero Shear Viscosity (Pa.s) | separated | separated | separated | 2096 |

| **Composition** | Example 15 (w/w %) | Example 16 (w/w %) |
|---|---|---|
| Surfactant B Example 1 | 6.66 | 6.66 |
| Sodium Lauryl Sulfate [2] | 6.66 | 6.66 |
| Cocamidopropyl hydroxysultaine[3] | 6.67 | 6.67 |
| Trideceth-3 (HLB=8) [4] | 2.00 | 2.00 |
| Sodium chloride (added) | 4 | 5 |
| Total level electrolyte | 4.76 | 5.76 |
| Methyl chloroisothiazolinone and methyl isothiazolinone [5] | 0.03 | 0.03 |
| EDTA [6] | 0.15 | 0.15 |

(continued)

| Composition | Example 15 (w/w %) | Example 16 (w/w %) |
|---|---|---|
| Sodium Benzoate | 0.20 | 0.20 |
| Citric Acid, titrate (pH = ± 0.4) | 5.70 | 5.70 |
| **Water** | **Q.S.** | **Q.S.** |
| | | |
| *Total Lathering Surfactant Component in Composition* | 20% | 20% |
| Lamellar Phase Volume (%) | 91% | 100% |
| Young's Modulus (Pa) | 60 | 67 |
| Zero Shear Viscosity (Pa.s) | 1866 | 1987 |

The surfactant compositions forming a cleansing phase may also comprise a rheology modifier to build the cleansing phase structure. The surfactant compositions of Table 11 (below) were prepared by adding water in a mixing vessel. Then add the following ingredients with continuously mixing: sodium chloride, guar hydroxypropyltrimonium chloride, rheology modifier powers (Structure XL), trideceth-3, xanthan gum, cocamidopropyl hydroxysultaine, Surfactant B Example 1 with an adequate agitation. Then add EDTA and sodium benzoate. Adjust pH by adding citric acid solution (50% active) to pH = 5.7 ± 0.2. Then, add methylchloroisothiazolinone and methyl isothiazolinone. Keep mixing until homogeneous.

After preparing these compositions, their Lamellar Phase Volume, Young's Modulus, Zero Shear Viscosity, and T-bar viscosity were determined utilizing the methods disclosed herein. The results are captured below in Table 11.

Stability was assessed by measuring through Ultracentrifugation Method after aging the products at 50°C for 10 days.

**Table 11**

| Composition | Example 17 (w/w %) |
|---|---|
| Surfactant B Example 1 | 6.66 |
| Sodium Lauryl Sulfate [2] | 6.66 |
| Cocamidopropyl hydroxysultaine[3] | 6.67 |
| Trideceth-3 (HLB=8) [4] | 1.64 |
| Sodium chloride (added) | 5 |
| Total level electrolyte (sodium chloride) | 5.76 |
| Hydroxylpropyl starch phosphate (Structure XL) | 1.00 |
| Guar Hydroxypropyltrimonium Chloride | 0.43 |
| Xanthan gum | 0.33 |
| Methyl chloroisothiazolinone and methyl isothiazolinone [5] | 0.04 |
| EDTA [6] | 0.17 |
| Sodium Benzoate | 0.34 |
| Citric Acid, titrate (pH = ± 0.4) | 5.70 |
| **Water** | **Q.S.** |
| | |
| *Total Lathering Surfactant Component in Composition* | 20% |
| Lamellar Phase Volume (%) | 66% |
| Young's Modulus (Pa) | 98 |
| Zero Shear Viscosity (Pa.s) | 22271 |
| T-bar viscosity (cP) | 43330 |

(continued)

| Stability | 10 days at 50°C |
|---|---|
| Lamellar Phase Volume (%) | 67% |
| Young's Modulus (Pa) | 89 |
| Zero Shear Viscosity (Pa.s) | 2277 |
| T-bar viscosity (cP) | 40270 |
| [2] available from Procter & Gamble Co.; [3] available as StarSurf™ CAPHS 50 from StarChem, LLC.; [4] Iconal TDA-3 available from BASF Corp.; [5] Kathon CG, available from Rohm & Haas Company, Philadephia, PA; [6] Dissolvine NA 2x. | |

Similar results as shown in Table 7 have demonstrated that a rheology modifier can help for building the cleansing phase structure such as hydroxylpropyl starch phosphate and now with another anionic surfactant like the sulfated product prepared from the Alcohol Example 2 that was commercially available as tradename LIAL®123A alcohol supplied from SASOL (Surfactant B Example 1). Example 17 appeared stable at 50°C after 10 days.

**Odor attributes**

Example 7 was compared to Comparative Example 15 which is a reference surfactant composition. C. Ex. 15 is the same composition as in Example 7 but comprising 6.66 wt.% of sodium trideceth-2 sulfate instead of C13 Alkyl sulfate from Alcohol 1. Off-odor attributes for the compositions are provided in Table 12:

**Table 12**

| Sample Code | Average Off Odor Intensity (0-100 scale, n=10) |
|---|---|
| Example 7 | **42** |
| Comparative Example 15 | **62.5** |
| **P-value** | **0.008** |

The data in Table 12 showed a directional difference between the comparative personal care composition of C. Ex. 15 having slightly higher off odor (i.e. slightly more odorous) than the inventive personal care composition of Ex. 7 having the specific C13 Alkyl sulfate from Alcohol 1 as recited herein.

## Claims

**1.** A personal care composition comprising at least a cleansing phase and a benefit phase,

wherein the personal care composition is free of ethoxylated anionic sulfate surfactant,
wherein the cleansing phase comprises an aqueous structured surfactant phase;
wherein the cleansing phase comprises:

(i) from 1% to 20% by weight of the personal care composition, of a surfactant A, wherein the surfactant A comprises a C13 alkyl sulfate anionic surfactant, wherein the C13 alkyl sulfate anionic surfactant consists of:

(a) less than 40% by weight of the C13 alkyl sulfate anionic surfactant of a linear C13 alkyl sulfate, and
(b) more than 60% by weight of the C13 alkyl sulfate anionic surfactant of a 2-branched C13 alkyl sulfate anionic surfactant, wherein the 2-branched C13 alkyl sulfate anionic surfactant comprises:
25% or less by weight of the 2-branched C13 alkyl sulfate anionic surfactant of 2-pentyl octyl sulfate anionic surfactant, and more than 25% by weight the 2-branched C13 alkyl sulfate anionic surfactant of 2-methyl dodecyl sulfate anionic surfactant; and
(c) less than 5% by weight of the C13 alkyl sulfate anionic surfactant of other branched C13 alkyl sulfate anionic surfactant,

wherein a, b and c add up to 100% by weight of the C13 alkyl sulfate anionic surfactant; or

from 1% to 20% by weight of the personal care composition, of a surfactant B, wherein the surfactant B comprises a mixture of C12 and C13 alkyl sulfate anionic surfactants, optionally wherein the surfactant B consists essentially of a mixture of C12 and C13 alkyl sulfate anionic surfactants,
wherein the mixture of the C12 and C13 alkyl sulfate anionic surfactants comprises a mixture of surfactant isomers of Formula I and surfactant isomers of Formula II:

$$CH_3(CH_2)_pCH_2\text{-}OSO_3^- X^+ \qquad (I)$$

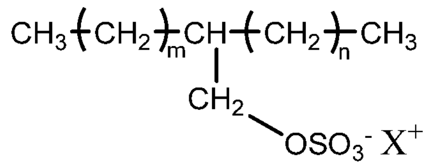

(II)

wherein 10 ≤ p ≤ 11;
wherein 8 ≤ m + n ≤ 9 and 0 ≤ m, n ≤ 9;
wherein X is a counter cation selected from the group consisting of lithium, sodium, potassium and ammonium, preferably sodium;
wherein the surfactant B comprises:
from 37% to 50%, preferably from 38% to 49%, more preferably from 40% to 49% of the C12 alkyl sulfate anionic surfactant by weight of the surfactant B, wherein the C12 alkyl sulfate anionic surfactant comprises a 2-alkyl branched C12 alkyl sulfate;
from 50% to 63%, preferably from 51% to 62%, more preferably from 51% to 60% of the C13 alkyl sulfate anionic surfactant by weight of the surfactant B, wherein the C13 alkyl sulfate anionic surfactant comprises a 2-alkyl branched C13 alkyl sulfate;
and less or equal than 5% of other alkyl sulfate anionic surfactants by weight of the surfactant B, preferably less or equal than 4% of other alkyl sulfate anionic surfactants by weight of the surfactant B, wherein other alkyl sulfate anionic surfactants are not C12 or C13 alkyl sulfate anionic surfactant

(ii) from 0.1% to 20% by weight of the personal care composition, of a zwitterionic surfactant, wherein the zwitterionic surfactant comprises an hydroxysultaine;
(iii) a structuring system comprising:

(iiia) a non-ionic emulsifier;
(iiib) from 0.01% to 5% by weight of the personal care composition, of a rheology modifier;
(iiic) from 3% to 10% by weight of the personal care composition, of an electrolyte;

wherein the benefit phase comprises: from 0.1% to 50%, by weight of said personal care composition, of a benefit agent.

**2.** The personal care composition of claim 1, wherein the C13 alkyl sulfate anionic surfactant of the surfactant A consists of:

(i) less than 30% by weight of the C13 alkyl sulfate anionic surfactant of the linear C13 alkyl sulfate, and
(ii) more than 70% by weight of the C13 alkyl sulfate anionic surfactant of the 2-branched C13 alkyl sulfate anionic surfactant, and
(iii) less than 3.0% by weight of the C13 alkyl sulfate anionic surfactant of other branched C13 alkyl sulfate anionic surfactant.

**3.** The personal care composition of any ones of claim 1 or 2, wherein the 2-branched C13 alkyl sulfate anionic surfactant of the surfactant A comprises:

(i) less than 20% by weight of the 2-branched C13 alkyl sulfate anionic surfactant of 2-pentyl octyl sulfate anionic surfactant; and

(ii) more than 30% by weight of the 2-branched C13 alkyl sulfate anionic surfactant of 2-methyl dodecyl sulfate anionic surfactant.

4. The personal care composition of claim 1, wherein the surfactant B has an average degree of branching of greater than 90% or from 40% to 60% by weight, preferably from 45% to 55% by weight, more preferably from 46% to 54% by weight, most preferably from 47% to 54%.

5. The personal care composition of any preceding claims, wherein the hydroxysultaine is selected from the group consisting of lauryl hydroxysultaine, coco-hydroxysultaine, lauramidopropyl hydroxysultaine, cocamidopropyl hydroxysultaine, cocobutyramido hydroxysultaine, capryllcapramidopropyl hydroxysultaine, cetyl/lauryl/myristyl hydroxysultaine, erucamidopropyl hydroxysultaine, methoxycinnamidopropyl hydroxysultaine, myristamidopropyl hydroxysultaine, oleamidopropyl hydroxysultaine, tallowamidopropyl hydroxysultaine and mixtures thereof.

6. The personal care composition of any preceding claims, wherein the rheology modifier is selected from the group consisting of a polyacrylate, a polysaccharide, a modified polyol, an hydrophobically modified polyacrylate, an hydrophobically modified polysaccharide, and mixtures thereof.

7. The personal care composition of any preceding claims, wherein the rheology modifier is selected from the group consisting of sodium polyacrylate, acrylates copolymer, Acrylates/Vinyl Isodecanoate Crosspolymer, Acrylates/C10-30 Alkyl Acrylate Crosspolymer, Acrylates/C10-30 alkyl acrylate crosspolymer and comprises stearyl side chains with less than 1% Hydrophobic modification, acrylates/C10-30 alkyl acrylate crosspolymer including octyl side chains with less than 5% Hydrophobic modification, Ammonium Acryloyldimethyltaurate/Beheneth-25 Methacrylate Crosspolymer, Acrylates/Beheneth-25 Methacrylate Copolymer, Acrylates/Steareth-20 Methacrylate Copolymer, and Acrylates/Steareth-20 Methacrylate Crosspolymer, PEG-150/Decyl Alcohol/SMDI Copolymer, PEG-150/stearyl alcohol/SMDI copolymer, hydroxypropyl starch phosphate, distarch phosphate, sodium carboxymethyl starch, starch, Tapioca starch, xanthan gum, gellan gum, carboxymethyl cellulose, carboxymethyl hydroxyethyl cellulose, hydroxypropyl methyl cellulose, guar gum, hydroxypropyl guar, sodium alginate, and mixtures thereof.

8. The personal care composition of any preceding claims, wherein the non-ionic emulsifier is selected from the group consisting of glyceryl monohydroxystearate, isosteareth-2, trideceth-2, trideceth-3, hydroxystearic acid, propylene glycol stearate, PEG-2 stearate, sorbitan monostearate, glyceryl laurate, laureth-2, cocamide monoethanolamine, lauramide monoethanolamine, and mixtures thereof.

9. The personal care composition of any preceding claims, wherein the electrolyte comprises an anion selected from the group consisting of phosphate, chloride, sulfate, citrate, and mixtures thereof; and a cation selected from the group consisting of sodium, ammonium, potassium, magnesium, and mixtures thereof.

10. The personal care composition of claim 9, wherein the electrolyte is selected from the group consisting of sodium chloride, ammonium chloride, sodium sulfate, ammonium sulfate, and mixtures thereof.

11. The personal care composition of any preceding claims, wherein the benefit phase is anhydrous.

12. The personal care composition of any preceding claims, wherein the benefit agent is selected from the group consisting of petrolatum; lanolin; natural waxes; synthetic waxes; volatile organosiloxanes; derivatives of volatile organosiloxanes; non-volatile organosiloxanes; derivatives of non-volatile organosiloxanes; lanolin oil; lanolin esters; natural triglycerides; synthetic triglycerides; and mixtures thereof.

13. The personal care composition of any preceding claims, wherein the personal care composition comprises a cationic deposition polymer, wherein the cationic deposition polymer is selected from the group consisting of a cationic cellulose polymer, a cationic guar gum polymer, and mixtures thereof.

14. The personal care composition of any preceding claims, wherein the personal care composition comprises at least 20% lamellar structure.

15. The personal care composition of any preceding claims, wherein the personal care composition is substantially free or free of betaine.

16. A method of providing a stable personal care composition comprises the step of forming a personal care composition

of any preceding claims with a surfactant A, wherein the surfactant A comprises a C13 alkyl sulfate anionic surfactant, wherein the C13 alkyl sulfate anionic surfactant consists of:

(a) less than 40% by weight of the C13 alkyl sulfate anionic surfactant of a linear C13 alkyl sulfate, and
(b) more than 60% by weight of the C13 alkyl sulfate anionic surfactant of a 2-branched C13 alkyl sulfate anionic surfactant, wherein the 2-branched C13 alkyl sulfate anionic surfactant comprises: 25% or less by weight of the 2-branched C13 alkyl sulfate anionic surfactant of 2-pentyl octyl sulfate anionic surfactant, and more than 25% by weight the 2-branched C13 alkyl sulfate anionic surfactant of 2-methyl dodecyl sulfate anionic surfactant; and
(c) less than 5% by weight of the C13 alkyl sulfate anionic surfactant of other branched C13 alkyl sulfate anionic surfactant, wherein a, b and c add up to 100% by weight of the C13 alkyl sulfate anionic surfactant; or
with a surfactant B, wherein the surfactant B comprises a mixture of C12 and C13 alkyl sulfate anionic surfactants, optionally wherein the surfactant B consists essentially of a mixture of C12 and C13 alkyl sulfate anionic surfactants,
wherein the mixture of the C12 and C13 alkyl sulfate anionic surfactants comprises a mixture of surfactant isomers of Formula I and surfactant isomers of Formula II:

$$CH_3 - (CH_2)_p - CH_2 - OSO_3^- \; X^+ \qquad (I)$$

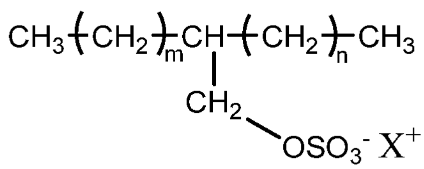

(II)

wherein $10 \le p \le 11$;
wherein $8 \le m + n \le 9$ and $0 \le m, n \le 9$;
wherein X is a counter cation selected from the group consisting of lithium, sodium, potassium and ammonium, preferably sodium;
wherein the surfactant B comprises:

from 37% to 50%, preferably from 38% to 49%, more preferably from 40% to 49% of the C12 alkyl sulfate anionic surfactant by weight of the surfactant B, wherein the C12 alkyl sulfate anionic surfactant comprises a 2-alkyl branched C12 alkyl sulfate;
from 50% to 63%, preferably from 51% to 62%, more preferably from 51% to 60% of the C13 alkyl sulfate anionic surfactant by weight of the surfactant B, wherein the C13 alkyl sulfate anionic surfactant comprises a 2-alkyl branched C13 alkyl sulfate;
and less or equal than 5% of other alkyl sulfate anionic surfactants by weight of the surfactant B, preferably less or equal than 4% of other alkyl sulfate anionic surfactants by weight of the surfactant B, wherein other alkyl sulfate anionic surfactants are not C12 or C13 alkyl sulfate anionic surfactant.

**Patentansprüche**

**1.** Körperpflegezusammensetzung, umfassend wenigstens eine Reinigungsphase und eine Nutzwirkungsphase, wobei die Körperpflegezusammensetzung frei von ethoxyliertem anionischem Sulfattensid ist, wobei die Reinigungsphase eine wässrige strukturierte Tensidphase umfasst;
wobei die Reinigungsphase umfasst:

(i) von 1 Gew.-% bis 20 Gew.-% der Körperpflegezusammensetzung ein Tensid A, wobei das Tensid A ein anionisches C13-Alkylsulfattensid umfasst, wobei das anionische C13-Alkylsulfattensid besteht aus:

(a) zu weniger als 40 Gew.-% aus dem anionischen C13-Alkylsulfattensid eines linearen C13-Alkylsulfats und
(b) zu mehr als 60 Gew.-% aus dem anionischen C13-Alkylsulfattensid eines 2-verzweigten anionischen C13-Alkylsulfattensids, wobei das 2-verzweigte anionische C13-Alkylsulfattensid umfasst:
zu 25 Gew.-% oder weniger das 2-verzweigte anionische C13-Alkylsulfattensid von anionischem 2-Pentyloctylsulfattensid und mehr als zu 25 Gew.-% das 2-verzweigte anionische C13-Alkylsulfattensid von anionischem 2-Methyldodecylsulfattensid; und

(c) zu weniger als 5 Gew.-% das anionische C13-Alkylsulfattensid von einem anderen verzweigten anionischen C13-Alkylsulfattensid,

wobei a, b und c zusammen zu 100 Gew.-% das anionische C13-Alkylsulfattensid ergeben; oder

von zu 1 Gew.-% bis 20 Gew.-% der Körperpflegezusammensetzung ein Tensid B, wobei das Tensid B eine Mischung aus anionischen C12- und C13-Alkylsulfattensiden umfasst, optional wobei das Tensid B im Wesentlichen aus einer Mischung aus anionischen C12- und C13-Alkylsulfattensiden besteht,

wobei die Mischung der anionischen C12- und C13-Alkylsulfattenside eine Mischung aus Tensidisomeren von Formel I und Tensidisomeren von Formel II umfasst:

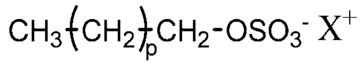 (I)

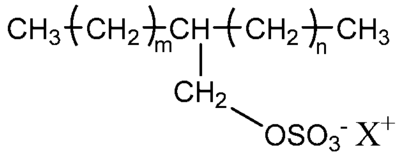 (II)

wobei $10 \leq p \leq 11$;

wobei $8 \leq m + n \leq 9$ und $0 \leq m, n \leq 9$;

wobei X ein Gegenkation ist, ausgewählt aus der Gruppe bestehend aus Lithium, Natrium, Kalium und Ammonium, vorzugsweise Natrium;

wobei das Tensid B umfasst:

von zu 37 Gew.-% bis 50 Gew.-%, vorzugsweise von zu 38 Gew.-% bis 49 Gew.-%, mehr bevorzugt von zu 40 Gew.-% bis 49 Gew.-% des Tensids B das anionische C12-Alkylsulfattensid, wobei das anionische C12-Alkylsulfattensid ein verzweigtes 2-Alkyl-C12-Alkylsulfat umfasst;

von zu 50 Gew.-% bis 63 Gew.-%, vorzugsweise von zu 51 Gew.-% bis 62 Gew.-%, mehr bevorzugt von zu 51 Gew.-% bis 60 Gew.-% des Tensids B das anionische C13-Alkylsulfattensid, wobei das anionische C13-Alkylsulfattensid ein verzweigtes 2-Alkyl-C13-Alkylsulfat umfasst;

und zu weniger als oder gleich 5 Gew.-% des Tensids B andere anionische Alkylsulfattenside, vorzugsweise zu weniger als oder gleich 4 Gew.-% des Tensids B andere anionische Alkylsulfattenside, wobei andere anionische Alkylsulfattenside nicht anionische C12- oder C13-Alkylsulfattenside sind;

(ii) von zu 0,1 Gew.-% bis 20 Gew.-% der Körperpflegezusammensetzung ein zwitterionisches Tensid, wobei das zwitterionische Tensid ein Hydroxysultain umfasst;

(iii) ein strukturierendes System, umfassend:

(iiia) einen nichtionischen Emulgator;

(iiib) von zu 0,01 Gew.-% bis 5 Gew.-% der Körperpflegezusammensetzung einen Rheologiemodifikator;

(iiic) von zu 3 Gew.-% bis 10 Gew.-% der Körperpflegezusammensetzung ein Elektrolyt;

wobei die Nutzwirkungsphase umfasst: von zu 0,1Gew.-% bis 50 Gew.-% der Körperpflegezusammensetzung einen Wirkstoff.

**2.** Körperreinigungszusammensetzung nach Anspruch 1, wobei das anionische C13-Alkylsulfattensid des Tensids A besteht aus:

(i) zu weniger als 30 Gew.-% des anionischen C13-Alkylsulfattensids aus dem linearen C13-Alkylsulfattensid und

(ii) zu mehr als 70 Gew.-% des anionischen C13-Alkylsulfattensids aus dem 2-verzweigten anionischen C13-Alkylsulfattensid und

(iii) zu weniger als 3,0 Gew.-% des anionischen C13-Alkylsulfattensids aus einem anderen verzweigten anionischen C13-Alkylsulfattensid.

**3.** Körperreinigungszusammensetzung nach einem der Ansprüche 1 oder 2, wobei das 2-verzweigte anionische C13-Alkylsulfattensid des Tensids A umfasst:

(i) zu weniger als 20 Gew.-% des 2-verzweigten anionischen C13-Alkylsulfattensids ein anionisches 2-Pentyloctylsulfattensid; und
(ii) zu mehr als 30 Gew.-% des 2-verzweigten anionischen C13-Alkylsulfattensids ein anionisches 2-Methyldodecylsulfattensid.

4. Körperpflegezusammensetzung nach Anspruch 1, wobei das Tensid B einen durchschnittlichen Verzweigungsgrad von mehr als 90 Gew.-% oder von zu 40 Gew.-% bis 60 Gew.-%, vorzugsweise von zu 45 Gew.-% bis 55 Gew.-%, mehr bevorzugt von zu 46 Gew.-% bis 54 Gew.-%, am meisten bevorzugt von zu 47 % bis 54 % aufweist.

5. Körperpflegezusammensetzung nach einem der vorstehenden Ansprüche, wobei das Hydroxysultain ausgewählt ist aus der Gruppe bestehend aus Laurylhydroxysultain, Coco-hydroxysultain, Lauramidopropylhydroxysultain, Cocamidopropylhydroxysultain, Cocobutyramidohydroxysultain, Capryl/Capramidopropylhydroxysultain, Cetyl/Lauryl/-Myristylhydroxysultain, Erucamidopropylhydroxysultain, Methoxycinnamidopropylhydroxysultain, Myristamidopropylhydroxysultain, Oleamidopropylhydroxysultain, Tallowamidopropylhydroxysultain und Mischungen davon.

6. Körperpflegezusammensetzung nach einem der vorstehenden Ansprüche, wobei der Rheologiemodifikator ausgewählt ist aus der Gruppe bestehend aus einem Polyacrylat, einem Polysaccharid, einem modifizierten Polyol, einem hydrophob modifizierten Polyacrylat, einem hydrophob modifizierten Polysaccharid und Mischungen davon.

7. Körperpflegezusammensetzung nach einem der vorstehenden Ansprüche, wobei der Rheologiemodifikator ausgewählt ist aus der Gruppe bestehend aus Natriumpolyacrylat, Acrylaten-Copolymer, Acrylaten/Vinylisodecanoatkreuzpolymer, Acrylaten/C10-30-Alkylacrylatkreuzpolymer, Acrylaten/C10-30-Alkylacrylatkreuzpolymer und Stearylseitenketten mit weniger als 1 % hydrophober Modifikation umfasst, Acrylaten/C10-30-Alkylacrylatkreuzpolymer einschließlich Octylseitenketten mit weniger als 5 % hydrophober Modifikation, Ammoniumacryloyldimethyltaurat/-Beheneth-25-Methacrylatkreuzpolymer, Acrylaten/Beheneth-25-Methacrylat-Copolymer, Acrylaten/Steareth-20-Methacrylat-Copolymer und Acrylaten/Steareth-20-Methacrylatkreuzpolymer, PEG-150/Decylalkohol/SMDI-Copolymer, PEG-150/Stearylalkohol/SMDI-Copolymer, Hydroxypropylstärkephosphat, Distärkephosphat, Natriumcarboxymethylstärke, Stärke, Tapiokastärke, Xanthangummi, Gellangummi, Carboxymethylcellulose, Carboxymethylhydroxyethylcellulose, Hydroxypropylmethylcellulose, Guargummi, Hydroxypropylguargummi, Natriumalginat und Mischungen davon.

8. Körperpflegezusammensetzung nach einem der vorstehenden Ansprüche, wobei der nichtionische Emulgator ausgewählt ist aus der Gruppe bestehend aus Glycerylmonohydroxystearat, Isosteareth-2, Trideceth-2, Trideceth-3, Hydroxystearinsäure, Propylenglycolstearat, PEG-2-stearat, Sorbitanmonostearat, Glyceryllaurat, Laureth-2, Cocamidmonoethanolamin, Lauramidmonoethanolamin und Mischungen davon.

9. Körperpflegezusammensetzung nach einem der vorstehenden Ansprüche, wobei der Elektrolyt ein Anion, ausgewählt aus der Gruppe bestehend aus Phosphat, Chlorid, Sulfat, Citrat und Mischungen davon; und ein Kation umfasst, ausgewählt aus der Gruppe bestehend aus Natrium, Ammonium, Kalium, Magnesium und Mischungen davon.

10. Körperpflegezusammensetzung nach Anspruch 9, wobei der Elektrolyt ausgewählt ist aus der Gruppe bestehend aus Natriumchlorid, Ammoniumchlorid, Natriumsulfat, Ammoniumsulfat und Mischungen davon.

11. Körperpflegezusammensetzung nach einem der vorstehenden Ansprüche, wobei die Nutzwirkungsphase wasserfrei ist.

12. Körperpflegezusammensetzung nach einem der vorstehenden Ansprüche, wobei der Wirkstoff ausgewählt ist aus der Gruppe bestehend aus Petrolatum; Lanolin; natürlichen Wachsen; synthetischen Wachsen; flüchtigen Organosiloxanen; Derivativen von flüchtigen Organosiloxanen; nichtflüchtigen Organosiloxanen; Derivativen von nichtflüchtigen Organosiloxanen; Lanolinöl; Lanolinestern; natürlichen Triglyceriden; synthetischen Triglyceriden; und Mischungen davon.

13. Körperpflegezusammensetzung nach einem der vorstehenden Ansprüche, wobei die Körperpflegezusammensetzung ein kationisch geladenes Abscheidepolymer umfasst, wobei das kationisch geladene Abscheidepolymer ausgewählt ist aus der Gruppe bestehend aus einem kationischen Cellulosepolymer, einem kationischen Guarkernmehlpolymer und Mischungen davon.

**14.** Körperpflegezusammensetzung nach einem der vorstehenden Ansprüche, wobei die Körperpflegezusammensetzung zu wenigstens 20 % eine lamellare Struktur umfasst.

**15.** Körperpflegezusammensetzung nach einem der vorstehenden Ansprüche, wobei die Körperpflegezusammensetzung im Wesentlichen frei oder frei von Betain ist.

**16.** Verfahren zum Bereitstellen einer stabilen Körperpflegezusammensetzung umfasst den Schritt eines Ausbildens einer Körperpflegezusammensetzung nach einem der vorstehenden Ansprüche mit einem Tensid A, wobei das Tensid A ein anionisches C13-Alkylsulfattensid umfasst, wobei das anionische C13-Alkylsulfattensid besteht aus:

(a) zu weniger als 40 Gew.-% aus dem anionischen C13-Alkylsulfattensid eines linearen C13-Alkylsulfats und
(b) zu mehr als 60 Gew.-% aus dem anionischen C13-Alkylsulfattensid eines 2-verzweigten anionischen C13-Alkylsulfattensids, wobei das 2-verzweigte anionische C13-Alkylsulfattensid umfasst: zu 25 Gew.-% oder weniger das 2-verzweigte anionische C13-Alkylsulfattensid von anionischem 2-Pentyloctylsulfattensid und mehr als zu 25 Gew.-% das 2-verzweigte anionische C13-Alkylsulfattensid von anionischem 2-Methyldodecylsulfattensid; und
(c) zu weniger als 5 Gew.-% aus dem anionischen C13-Alkylsulfattensid eines anderen verzweigten anionischen C13-Alkylsulfattensids, wobei a, b und c zusammen 100 Gew.-% des anionischen C13-Alkylsulfattensids ergeben; oder

oder mit einem Tensid B, wobei das Tensid B eine Mischung aus anionischen C12- und C13-Alkylsulfattensiden umfasst, optional wobei das Tensid B im Wesentlichen aus einer Mischung aus anionischen C12- und C13-Alkylsulfattensiden besteht,
wobei die Mischung der anionischen C12- und C13-Alkylsulfattenside eine Mischung aus Tensidisomeren von Formel I und Tensidisomeren von Formel II umfasst:

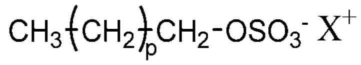
(I)

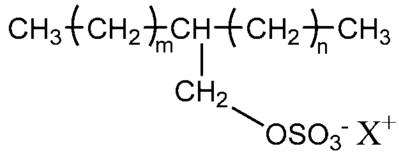
(II)

wobei $10 \leq p \leq 11$;
wobei $8 \leq m + n \leq 9$ und $0 \leq m, n \leq 9$;
wobei X ein Gegenkation ist, ausgewählt aus der Gruppe bestehend aus Lithium, Natrium, Kalium und Ammonium, vorzugsweise Natrium;
wobei das Tensid B umfasst:
von zu 37 Gew.-% bis 50 Gew.-%, vorzugsweise von zu 38 Gew.-% bis 49 Gew.-%, mehr bevorzugt von zu 40 Gew.-% bis 49 Gew.-% des Tensids B das anionische C12-Alkylsulfattensid, wobei das anionische C12-Alkylsulfattensid ein verzweigtes 2-Alkyl-C12-Alkylsulfat umfasst;
von zu 50 Gew.-% bis 63 Gew.-%, vorzugsweise von zu 51 Gew.-% bis 62 Gew.-%, mehr bevorzugt von zu 51 Gew.-% bis 60 Gew.-% des Tensids B das anionische C13-Alkylsulfattensid, wobei das anionische C13-Alkylsulfattensid ein verzweigtes 2-Alkyl-C13-Alkylsulfat umfasst;
und zu weniger als oder gleich 5 Gew.-% des Tensids B andere anionische Alkylsulfattenside, vorzugsweise zu weniger als oder gleich 4 Gew.-% des Tensids B andere anionische Alkylsulfattenside, wobei andere anionische Alkylsulfattenside nicht anionische C12- oder C13-Alkylsulfattenside sind.

## Revendications

**1.** Composition de soins personnels comprenant au moins une phase nettoyante et une phase bénéfique, dans laquelle la composition de soins personnels est exempte d'agent tensioactif sulfate anionique éthoxylé, dans laquelle la phase nettoyante comprend une phase aqueuse d'agent tensioactif structuré ;

dans laquelle la phase nettoyante comprend :

(i) de 1 % à 20 %, en poids de la composition de soins personnels, d'un agent tensioactif A, dans laquelle l'agent tensioactif A comprend un agent tensioactif anionique sulfate d'alkyle en C13, dans laquelle l'agent tensioactif anionique sulfate d'alkyle en C13 est constitué de :

(a) moins de 40 %, en poids de l'agent tensioactif anionique sulfate d'alkyle en C13, d'un sulfate d'alkyle en C13 linéaire, et
(b) plus de 60 %, en poids de l'agent tensioactif anionique sulfate d'alkyle en C13, d'un agent tensioactif anionique sulfate d'alkyle en C13 ramifié en position 2, dans laquelle l'agent tensioactif anionique sulfate d'alkyle en C13 ramifié en position 2 comprend :
25 % ou moins, en poids de l'agent tensioactif anionique sulfate d'alkyle en C13 ramifié en position 2, d'agent tensioactif anionique sulfate de 2-pentyl octyle, et plus de 25 %, en poids de l'agent tensioactif anionique sulfate d'alkyle en C13 ramifié en position 2, d'agent tensioactif anionique sulfate de 2-méthyl dodécyle ; et
(c) moins de 5 %, en poids de l'agent tensioactif anionique sulfate d'alkyle en C13, d'agent tensioactif anionique sulfate d'alkyle en C13 ramifié autrement,

dans laquelle a, b et c s'additionnent jusqu'à 100 % en poids de l'agent tensioactif anionique sulfate d'alkyle en C13 ; ou
de 1 % à 20 %, en poids de la composition de soins corporels, d'un agent tensioactif B, dans laquelle l'agent tensioactif B comprend un mélange d'agents tensioactifs anioniques sulfate d'alkyle en C12 et C13, facultativement dans laquelle l'agent tensioactif B est constitué sensiblement d'un mélange d'agents tensioactifs anioniques sulfate d'alkyle en C12 et C13,
dans laquelle le mélange des agents tensioactifs anioniques sulfate d'alkyle en C12 et C13 comprend un mélange d'isomères d'agents tensioactifs de Formule I et d'isomères d'agents tensioactifs de Formule II :

$$CH_3\text{-}(CH_2)_p\text{-}CH_2\text{-}OSO_3^-\ X^+ \qquad (I)$$

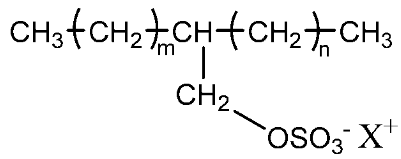

(II)

dans lesquelles $10 \leq p \leq 11$ ;
dans lesquelles $8 \leq m + n \leq 9$ et $0 \leq m, n \leq 9$ ;
dans lesquelles X est un contre-cation choisi dans le groupe constitué de lithium, sodium, potassium et ammonium, de préférence est le sodium ;
dans laquelle l'agent tensioactif B comprend :
de 37 % à 50 %, de préférence de 38 % à 49 %, plus préférablement de 40 % à 49 % de l'agent tensioactif anionique sulfate d'alkyle en C12 en poids de l'agent tensioactif B, dans laquelle l'agent tensioactif anionique sulfate d'alkyle en C12 comprend un sulfate d'alkyle en C12 ramifié par alkyle en position 2 ;
de 50 % à 63 %, de préférence de 51 % à 62 %, plus préférablement de 51 % à 60 % de l'agent tensioactif anionique sulfate d'alkyle en C13 en poids de l'agent tensioactif B, dans laquelle l'agent tensioactif anionique sulfate d'alkyle en C13 comprend un sulfate d'alkyle en C13 ramifié par alkyle en position 2 ;
et 5 % ou moins d'autres agents tensioactifs anioniques sulfate d'alkyle, en poids de l'agent tensioactif B, de préférence 4 % ou moins d'autres agents tensioactifs anioniques sulfate d'alkyle, en poids de l'agent tensioactif B, dans laquelle les autres agents tensioactifs anioniques sulfate d'alkyle ne sont pas un agent tensioactif anionique sulfate d'alkyle en C12 ou C13

(ii) de 0,1 % à 20 %, en poids de la composition de soins corporels, d'un agent tensioactif zwitterionique, dans laquelle l'agent tensioactif zwitterionique comprend une hydroxysultaïne ;

(iii) un système structurant comprenant :

(iiia) un émulsifiant non ionique ;
(iiib) de 0,01 % à 5 %, en poids de la composition de soins corporels, d'un agent modifiant la rhéologie ;
(iiic) de 3 % à 10 %, en poids de la composition de soins corporels, d'un électrolyte ;

dans laquelle la phase bénéfique comprend : de 0,1 % à 50 %, en poids de ladite composition de soins corporels, d'un agent bénéfique.

**2.** Composition de soins personnels selon la revendication 1, dans laquelle l'agent tensioactif anionique sulfate d'alkyle en C13 de l'agent tensioactif A est constitué de :

(i) moins de 30 %, en poids de l'agent tensioactif anionique sulfate d'alkyle en C13, du sulfate d'alkyle en C13 linéaire, et
(ii) plus de 70 %, en poids de l'agent tensioactif anionique sulfate d'alkyle en C13, de l'agent tensioactif anionique sulfate d'alkyle en C13 ramifié en position 2, et
(iii) moins de 3,0 %, en poids de l'agent tensioactif anionique sulfate d'alkyle en C13, d'agent tensioactif anionique sulfate d'alkyle en C13 ramifié autrement.

**3.** Composition de soins personnels selon l'une quelconque des revendications 1 ou 2, dans laquelle l'agent tensioactif anionique sulfate d'alkyle en C13 ramifié en position 2 de l'agent tensioactif A comprend :

(i) moins de 20 %, en poids de l'agent tensioactif anionique sulfate d'alkyle en C13 ramifié en position 2, d'agent tensioactif anionique sulfate de 2-pentyl octyle ; et
(ii) plus de 30 %, en poids de l'agent tensioactif anionique sulfate d'alkyle en C13 ramifié en position 2, d'agent tensioactif anionique sulfate de 2-méthyldodécyle.

**4.** Composition de soins corporels selon la revendication 1, dans laquelle l'agent tensioactif B a un degré moyen de ramification supérieur à 90 % ou allant de 40 % à 60 % en poids, de préférence de 45 % à 55 % en poids, plus préférablement de 46 % à 54 % en poids, le plus préférablement de 47 % à 54 %.

**5.** Composition de soins personnels selon l'une quelconque des revendications précédentes, dans laquelle l'hydroxysultaïne est choisie dans le groupe constitué de lauryl hydroxysultaïne, coco-hydroxysultaïne, lauramidopropyl hydroxysultaïne, cocamidopropyl hydroxysultaïne, cocobutyramido hydroxysultaïne, capryl/capramidopropyl hydroxysultaïne, cétyl/lauryl/myristyl hydroxysultaïne, érucamidopropyl hydroxysultaïne, méthoxycinnamidopropyl hydroxysultaïne, myristamidopropyl hydroxysultaïne, oléamidopropyl hydroxysultaïne, tallowamidopropyl hydroxysultaïne et mélanges de celles-ci.

**6.** Composition de soins personnels selon l'une quelconque des revendications précédentes, dans laquelle l'agent modifiant la rhéologie est choisi dans le groupe constitué d'un polyacrylate, d'un polysaccharide, d'un polyol modifié, d'un polyacrylate à modification hydrophobe, d'un polysaccharide à modification hydrophobe, et mélanges de ceux-ci.

**7.** Composition de soins corporels selon l'une quelconque des revendications précédentes, dans laquelle l'agent modifiant la rhéologie est choisi dans le groupe constitué de polyacrylate de sodium, copolymère d'acrylates, polymère réticulé d'acrylates/isodécanoate de vinyle, polymère réticulé d'acrylates/acrylate d'alkyle en C10-30, polymère réticulé d'acrylates/acrylate d'alkyle en C10-30 et comprend des chaînes latérales stéaryle avec moins de 1 % de modification hydrophobe, polymère réticulé d'acrylates/acrylate d'alkyle en C10-30 comportant des chaînes latérales octyle avec moins de 5 % de modification hydrophobe, polymère réticulé d'acryloyldiméthyltaurate d'ammonium/méthacrylate de béhéneth-25, polymère réticulé d'acrylates/méthacrylate de béhéneth-25, copolymère d'acrylates/méthacrylate de stéareth-20, et polymère réticulé d'acrylates/méthacrylate de stéareth-20, copolymère de PEG-150/alcool décylique/SMDI, copolymère de PEG-150/alcool stéarylique/SMDI, phosphate d'hydroxypropyl-amidon, phosphate de diamidon, carboxyméthylamidon de sodium, amidon, amidon de tapioca, gomme de xanthane, gomme gellane, carboxyméthylcellulose, carboxyméthyl-hydroxyéthylcellulose, hydroxypropylméthylcellulose, gomme de guar, hydroxypropyl-guar, alginate de sodium, et mélanges de ceux-ci.

**8.** Composition de soins corporels selon l'une quelconque des revendications précédentes, dans laquelle l'émulsifiant non ionique est choisi dans le groupe constitué de monohydroxystéarate de glycéryle, isostéareth-2, trideceth-2,

tridéceth-3, acide hydroxystéarique, stéarate de propylène glycol, stéarate PEG-2, monostéarate de sorbitane, laurate de glycéryle, laureth-2, cocamide monoéthanolamine, lauramide monoéthanolamine, et mélanges de ceux-ci.

**9.** Composition de soins personnels selon l'une quelconque des revendications précédentes, dans laquelle l'électrolyte comprend un anion choisi dans le groupe constitué de phosphate, chlorure, sulfate, citrate, et mélanges de ceux-ci ; et un cation choisi dans le groupe constitué de sodium, ammonium, potassium, magnésium, et mélanges de ceux-ci.

**10.** Composition de soins personnels selon la revendication 9, dans laquelle l'électrolyte est choisi dans le groupe constitué de chlorure de sodium, chlorure d'ammonium, sulfate de sodium, sulfate d'ammonium, et mélanges de ceux-ci.

**11.** Composition de soins personnels selon l'une quelconque des revendications précédentes, dans laquelle la phase bénéfique est anhydre.

**12.** Composition de soins personnels selon l'une quelconque des revendications précédentes, dans laquelle l'agent bénéfique est choisi dans le groupe constitué de pétrolatum ; lanoline ; cires naturelles ; cires synthétiques ; organosiloxanes volatils ; dérivés d'organosiloxanes volatils ; organosiloxanes non volatils ; dérivés d'organosiloxanes non volatils ; huile de lanoline ; esters de lanoline ; triglycérides naturels ; triglycérides synthétiques ; et mélanges de ceux-ci.

**13.** Composition de soins personnels selon l'une quelconque des revendications précédentes, dans laquelle la composition de soins personnels comprend un polymère de dépôt cationique, dans laquelle le polymère de dépôt cationique est choisi dans le groupe constitué d'un polymère de cellulose cationique, d'un polymère de gomme de guar cationique, et de mélanges de ceux-ci.

**14.** Composition de soins personnels selon l'une quelconque des revendications précédentes, dans laquelle la composition de soins personnels comprend au moins 20 % de structure lamellaire.

**15.** Composition de soins personnels selon l'une quelconque des revendications précédentes, dans laquelle la composition de soins personnels est sensiblement exempte, ou exempte, de bétaïne.

**16.** Procédé de fourniture d'une composition de soins personnels stable comprenant l'étape consistant à former une composition de soins personnels selon l'une quelconque des revendications précédentes avec un agent tensioactif A, dans lequel l'agent tensioactif A comprend un agent tensioactif anionique sulfate d'alkyle en C13, dans lequel l'agent tensioactif anionique sulfate d'alkyle en C13 est constitué de :

(a) moins de 40 %, en poids de l'agent tensioactif anionique sulfate d'alkyle en C13, d'un sulfate d'alkyle en C13 linéaire, et
(b) plus de 60 %, en poids de l'agent tensioactif anionique sulfate d'alkyle en C13, d'un agent tensioactif anionique sulfate d'alkyle en C13 ramifié en position 2, dans laquelle l'agent tensioactif anionique sulfate d'alkyle en C13 ramifié en position 2 comprend : 25 % ou moins, en poids de l'agent tensioactif anionique sulfate d'alkyle en C13 ramifié en position 2, d'agent tensioactif anionique sulfate de 2-pentyl octyle, et plus de 25 %, en poids de l'agent tensioactif anionique sulfate d'alkyle en C13 ramifié en position 2, d'agent tensioactif anionique sulfate de 2-méthyl dodécyle ; et
(c) moins de 5 %, en poids de l'agent tensioactif anionique sulfate d'alkyle en C13, d'agent tensioactif anionique sulfate d'alkyle en C13 ramifié autrement, dans lequel a, b et c s'additionnent jusqu'à 100 % en poids de l'agent tensioactif anionique sulfate d'alkyle en C13 ; ou

avec un agent tensioactif B, dans lequel l'agent tensioactif B comprend un mélange d'agents tensioactifs anioniques sulfate d'alkyle en C12 et C13, facultativement dans lequel l'agent tensioactif B est sensiblement constitué d'un mélange d'agents tensioactifs anioniques sulfate d'alkyle en C12 et C13,
dans lequel le mélange des agents tensioactifs anioniques sulfate d'alkyle en C12 et C13 comprend un mélange d'isomères d'agents tensioactifs de Formule I et d'isomères d'agents tensioactifs de Formule II :

$$CH_3\left(CH_2\right)_pCH_2\text{-}OSO_3^-\ X^+ \qquad (I)$$

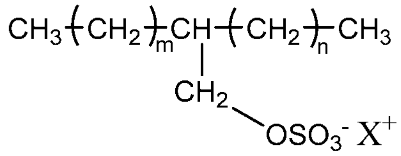

(II)

dans lesquelles 10 ≤ p ≤ 11 ;

dans lesquelles 8 ≤ m + n ≤ 9 et 0 ≤ m, n ≤ 9 ;

dans lesquelles X est un contre-cation choisi dans le groupe constitué de lithium, sodium, potassium et ammonium, de préférence est le sodium ;

dans lequel l'agent tensioactif B comprend :

de 37 % à 50 %, de préférence de 51 % à 49 %, plus préférablement de 51 % à 49 % de l'agent tensioactif anionique sulfate d'alkyle en C12 en poids de l'agent tensioactif B, dans lequel l'agent tensioactif anionique sulfate d'alkyle en C12 comprend un sulfate d'alkyle en C12 ramifié par alkyle en position 2 ;

de 50 % à 63 %, de préférence de 51 % à 62 %, plus préférablement de 51 % à 60 % de l'agent tensioactif anionique sulfate d'alkyle en C13 en poids de l'agent tensioactif B, dans lequel l'agent tensioactif anionique sulfate d'alkyle en C13 comprend un sulfate d'alkyle en C13 ramifié par alkyle en position 2 ;

et 5 % ou moins d'autres agents tensioactifs anioniques sulfate d'alkyle, en poids de l'agent tensioactif B, de préférence 4 % ou moins d'autres agents tensioactifs anioniques sulfate d'alkyle, en poids de l'agent tensioactif B, dans lequel les autres agents tensioactifs anioniques sulfate d'alkyle ne sont pas un agent tensioactif anionique sulfate d'alkyle en C12 ou C13.

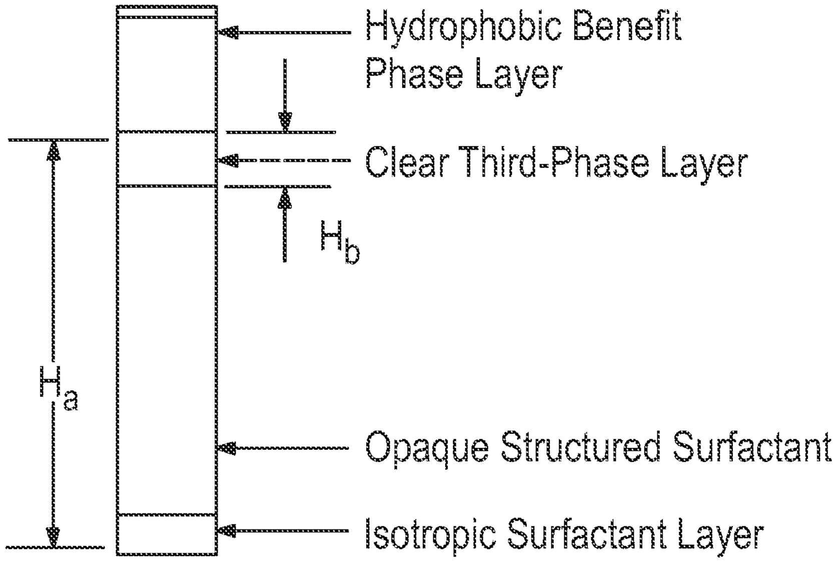
Hydrophobic Benefit
Phase Layer
Clear Third-Phase Layer
$H_b$
$H_a$
Opaque Structured Surfactant
Isotropic Surfactant Layer

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2006079418 A1
- US 5104646 A
- US 5106609 A
- US 2658072 A
- US 2438091 A
- US 2528378 A
- US 5011681 A, Ciotti
- US 5487884 A, Bisset
- US 5681852 A
- US 5652228 A, Bisset
- US 6395691 B, Liang Sheng Tsaur
- US 6645511 B, Aronson
- US 6759376 B, Zhang
- US 6780826 B, Zhang
- WO 20060182699 A, Taylor
- US 6213166 B, Thibiant
- US 20040219119 A1, Wei


### Non-patent literature cited in the description


- **C.F. PUTNIK** ; **S.E. MCGUIRE**. The Effect of Reaction By-Products on the Viscosities of Sodium Lauryl Sulfate Solutions. *Journal of the American Oil Chemists' Society*, 1978, vol. 55 (12), 909-913
- Water Soluble Polymers. ACS Symposium Series. Am. Chem. Soc. Washington, 1991, vol. 467, 82-200
- **S. BIGGS**. *J. Phys Chem*, 1992, vol. 96, 1505-11
- HLB Index. McCutcheon's Emulsifiers and Detergents. MC Publishing Co., 2004
- **GRIFFIN**. *J. Soc. Cosmetic Chem.*, 1949, vol. 1, 311
- **DAVIES**. Interfacial Phenomena. Academic Press, 1963
- **LIN**. *J. Phys. Chem.*, 1972, vol. 76, 2019-2013
- **VAUGHAN**. *Cosmetics and Toiletries*, October 1988, 47-69
- CTFA Cosmetic Ingredient Handbook. The Cosmetic, Toiletries, and Fragrance Association, Inc., 1988